(19) Europäisches Patentamt
European Patent Office
Office européen des brevets

(11) EP 1 262 190 A1

(12) **EUROPEAN PATENT APPLICATION**
published in accordance with Art. 158(3) EPC

(43) Date of publication:
**04.12.2002 Bulletin 2002/49**

(21) Application number: **01908337.7**

(22) Date of filing: **06.03.2001**

(51) Int Cl.[7]: **A61K 38/22**, A61K 45/00, A61P 5/10, A61P 15/00, A61P 15/08, A61P 15/10, C07K 14/72, C07K 16/28, C12N 15/12, C12Q 1/02, G01N 33/12, G01N 33/50, G01N 33/566, G01N 33/53

(86) International application number:
**PCT/JP01/01716**

(87) International publication number:
**WO 01/066134 (13.09.2001 Gazette 2001/37)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **06.03.2000 JP 2000065752**
**07.12.2000 JP 2000378001**

(71) Applicant: **Takeda Chemical Industries, Ltd.**
**Osaka-shi, Osaka 541-0045 (JP)**

(72) Inventors:
- **MATSUMOTO, Yoshio,**
**Takedayakuhin Satsukigaokaryou**
**Ikeda-shi, Osaka 563-0029 (JP)**
- **WATANABE, Takuya**
**Osaka-shi, Osaka 532-0033 (JP)**
- **HINUMA, Shuji**
**Tsukuba-shi, Ibaraki 305-0821 (JP)**
- **HABATA, Yugo**
**Tsukuba-shi, Ibaraki 305-0044 (JP)**
- **YOSHIDA, Hiromi**
**Yuki-gun, Ibaraki 300-2741 (JP)**

(74) Representative: **Lewin, John Harvey et al**
**Takeda Patent Office,**
**11-12 Charles II Street**
**London SW1Y 4QU (GB)**

(54) **RFRP-CONTAINING PROLACTIN SECRETION REGULATORY AGENT**

(57) The polypeptides in the present invention possess the effects of promoting and inhibiting the secretion of prolactin, and are thus useful as drugs for the prevention and treatment of various diseases, in terms of prolactin secretion stimulants, which are associated with the secretion of prolactin, such as hypoovarianism, spermatic underdevelopment, menopausal symptoms, hypothyroidism, etc. The polypeptides are useful as drugs for the prevention and treatment of various diseases, in terms of prolactin secretion inhibitors, which are associated with the secretion of prolactin, such as pituitary tumor, diencephalon tumor, menstrual disorder, autoimmune diseases, prolactinoma, sterility, impotence, amenorrhea, lactorrhea, acromegaly, Chiari-Frommel syndrome, Argonz-del Castilo syndrome, Forbes-Albright syndrome, lymphoma, Sheehan's syndrome, spermatogenesis disorder, etc.



Printed by Jouve, 75001 PARIS (FR)

## Description

FIELD OF THE INVENTION

The present invention relates to a prolactin secretion regulatory agent comprising a novel polypeptide (hereinafter sometimes referred to as a novel physiologically active polypeptide in the specification), its partial peptide, etc., and the like.

BACKGROUND ART

A variety of hormones and neurotransmitters regulate biological functions in vivo through specific receptor proteins located on the cell membrane. Many of these receptor proteins are coupled with guanine nucleotide-binding protein (hereinafter sometimes referred to merely as G protein) and engage themselves in the intracellular signal transduction through activation of the G protein. These receptor proteins possess the common structure, i.e. seven transmembrane regions and are thus collectively referred to as G protein-coupled receptors or seven-transmembrane receptors (7TMR).

One of the pathways to modulate biological functions mediated by the interactions of hormones or neurotransmitters with G protein-coupled receptors is the hypothalamus-pituitary system. In this system, the secretion of pituitary hormones from the pituitary gland is regulated by hypothalamic hormones (pituitatropic hormones), and the functions of the target cells/organs are regulated by the pituitary hormones released into the blood. Functions, which are important for the living body, including the maintenance of homeostasis and the control of development, metabolism and growth of a genital system and an individual organism, are regulated through this pathway.

The secretion of pituitary hormones is controlled by a positive feedback or a negative feedback mechanism by the hypothalamic hormone and the peripheral hormone secreted from the target endocrine glands.

It is also widely known that these hormones and factors as well as their receptors are not localized in the hypothalamus-pituitary system but are broadly distributed in the brain. This fact suggests that the substances called hypothalamic hormones are functioning as neurotransmitters or neuromodulators in the central nervous system.

Moreover, these substances are distributed even in the peripheral tissues as well and thought to be playing the role of important functions in the respective tissues.

In view of the foregoing, it has been desired to develop a drug for regulating the biological functions by G protein-coupled receptors and their ligands, in particular, for regulating the secretion of pituitary hormones from the pituitary gland by regulating the secretion of hypothalamic hormones.

DISCLOSURE OF THE INVENTION

In order to solve the foregoing problems, the present inventors have made extensive studies and as a result, have found that physiologically active peptides characterized by containing an RF amide-like or RS amide-like structure, in particular, polypeptides characterized by containing the same or substantially the same amino acid sequence as the amino acid sequence represented by SEQ ID NO:1 or partial peptides thereof possess the function of regulating prolactin release. As a result of further investigations, the present invention has come to be accomplished.

Thus, the present invention provides the following.

(1) A prolactin secretion regulatory agent comprising a polypeptide containing the same or substantially the same amino acid sequence as the amino acid sequence represented by SEQ ID NO:1, its amide or ester, or a salt thereof;
(2) The agent according to (1), wherein substantially the same amino acid sequence is represented by SEQ ID NO:8, SEQ ID NO:14, SEQ ID NO:18, SEQ ID NO:33 or SEQ ID NO:50;
(3) A prolactin secretion regulatory agent comprising a partial peptide of a polypeptide containing the same or substantially the same amino acid sequence as the amino acid sequence represented by SEQ ID NO:1, its amide or ester, or a salt thereof;
(4) The prolactin secretion regulatory agent according to (3), comprising a partial peptide composed of acid residues 81 (Met) to 92 (Phe) of SEQ ID NO:1, its amide or ester, or a salt thereof;
(5) The prolactin secretion regulatory agent according to (3), comprising a partial peptide composed of amino acid residues 101 (Ser) to 112 (Ser) of SEQ ID NO:1, its amide or ester, or a salt thereof;
(6) The prolactin secretion regulatory agent according to (3), comprising a partial peptide composed of amino acid residues 124 (Val) to 131 (Phe) of SEQ ID NO:1, its amide or ester, or a salt thereof;
(7) The prolactin secretion regulatory agent according to (3), comprising a partial peptide composed of amino acid residues 56 (Ser) to 92 (Phe) of SEQ ID NO:1, its amide or ester, or a salt thereof;
(8) The prolactin secretion regulatory agent according to (3), comprising an amide of a partial peptide of a polypeptide containing the same or substantially the same amino acid sequence as the amino acid sequence represented by SEQ ID NO:1, or a salt thereof;

(9) The prolactin secretion regulatory agent according to (8), comprising a partial peptide of a polypeptide containing the same or substantially the same amino acid sequence as the amino acid sequence represented by SEQ ID NO:1, the C-terminal carboxyl of which is amidated, or a salt thereof;

(10) The prolactin secretion regulatory agent according to (1) or (3), which is a prolactin secretion stimulant;

(11) The prolactin secretion regulatory agent according to (1) or (3), which is a prolactin secretion inhibitor;

(12) The prolactin secretion stimulant according to (10), which is a medicament for the prevention or treatment of hypoovarianism, spermatic underdevelopment, osteoporosis, menopausal symptoms, agalactosis, hypothyroidism or renal insufficiency;

(13) The a prolactin secretion inhibitor according to (11), which is a medicament for the prevention or treatment of hyperprolactinemia, pituitary tumor, diencephalon tumor, menstrual disorder, stress, autoimmune diseases, prolactinoma, sterility, impotence, amenorrhea, lactorrhea, acromegaly, Chiari-Frommel syndrome, Argonz-del Castilo syndrome, Forbes-Albright syndrome, breast cancer lymphoma or Sheehan's syndrome, or spermatogenesis disorder;

(14) The prolactin secretion regulatory agent according to (1) or (3), which is a lactation stimulant for livestock mammal;

(15) The prolactin secretion regulatory agent according to (I) or (3), which is a test agent for prolactin secretion function;

(16) A prolactin secretion regulatory agent comprising a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, which is obtainable using:

a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof; or,

a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof.

(17) A prolactin secretion regulatory agent comprising a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, which is obtainable using:

(I) a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof; or,
(II) a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof.

(18) (1) A peptide containing an amino acid sequence composed of 81 (Met) to 92 (Phe) of SEQ ID NO:1, its amide or ester, or a salt thereof, (2) a peptide containing an amino acid sequence composed of 101 (Ser) to 112 (Ser) of SEQ ID NO:1, its amide or ester, or a salt thereof, (3) a peptide containing an amino acid sequence composed of 124 (Val) to 131 (Phe) of SEQ ID NO: 1, its amide or ester, or a salt thereof, (4) a peptide containing an amino acid sequence composed of 56 (Ser) to 92 (Phe) of SEQ ID NO:1, its amide or ester, or a salt thereof, (5) a peptide containing an amino acid sequence composed of 81 (Met) to 92 (Phe) of SEQ ID NO: 14, its amide or ester, or a salt thereof, (6) a peptide containing an amino acid sequence composed of 101 (Ser) to 112 (Leu) of SEQ ID NO: 14, its amide or ester, or a salt thereof, (7) a peptide containing an amino acid sequence composed of 58(Ser) to 92 (Phe) of SEQ ID NO:14, its amide or ester, or a salt thereof, (8) a peptide containing an amino acid sequence composed of 83 (Val) to 94 (Phe) of SEQ ID NO:33, its amide or ester, or a salt thereof, (9) a peptide containing an amino acid sequence composed of 118 (Phe) to 125 (Phe) of SEQ ID NO:33, its amide or ester, or a salt thereof, (10) a peptide containing an amino acid sequence composed of 58 (Ser) to 94 (Phe) of SEQ ID NO:33, its amide or ester, or a salt thereof, or (11) a peptide containing an amino acid sequence composed of 58 (Ser) to 94 (Phe) of SEQ ID NO:50, its amide or ester, or a salt thereof;
(19) An amide of the peptide according to (18), or a salt thereof;
(20) The peptide according to (18) wherein the C-terminal carboxyl is amidated, its amide or ester, or a salt thereof;
(21) A DNA encoding the peptide according to (18);
(22) The DNA containing (1) a 241-276 base sequence of SEQ ID NO:2, (2) a 301-336 base sequence of SEQ ID NO:2, (3) a 370-393 base sequence of SEQ ID NO:2, (4) a 166-276 base sequence of SEQ ID NO:2, (5) a 241-276 base sequence of SEQ ID NO:15, (6) a 301-336 base sequence of SEQ ID NO:15, (7) a 172-276 base sequence of SEQ ID NO:15, (8) a 247-282 base sequence of SEQ ID NO:34, (9) a 352-375 base sequence of SEQ ID NO: 34, (10) a 172-282 base sequence of SEQ ID NO:34, or (11) a 172-282 base sequence of SEQ ID NO:51;
(23) An antibody to the peptide according to (18), or its amide or ester, or a salt thereof;
(24) A diagnostic comprising the DNA according to (21) or the antibody according to (23);
(25) An antisense DNA having a base sequence complementary or substantially complementary to the DNA according to (21) and capable of inhibiting expression of the DNA;
(26) An agent comprising the peptide according to (18), or its amide or ester, or a salt thereof;
(27) A pharmaceutical composition comprising the peptide according to (18), or its amide or ester, or a salt thereof;
(28) The pharmaceutical composition according to (27), which is a prolactin secretion regulatory agent;
(29) A method of screening a compound or its salt that promotes or inhibits the activity of the peptide according to (18), or its amide or ester, or a salt thereof, which comprises using the peptide according to (18), or its amide or ester, or a salt thereof;
(30) The method of screening according to (29), wherein a protein containing the same or substantially the same amino acid sequence represented by SEQ ID NO:37 or a salt thereof, or its partial peptide, its amide or ester, or a salt thereof is further used;
(31) A kit for screening a compound or its salt that promotes or inhibits the activity of the peptide according to (18), or its amide or ester, or a salt thereof, which comprises using the peptide according to (18), or its amide or ester, or a salt thereof;
(32) A compound or its salt that promotes or inhibits the activity of the peptide according to (18), or its amide or ester, or a salt thereof, which is obtainable using the screening method according to (29) or the screening kit according to (31);
(33) Use of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity;

(34) A method of regulating the secretion of prolactin, which comprises administering to a mammal (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof.

(35) Use of a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, for the manufacture of a medicament having a prolactin secretion regulating activity, which is obtainable using:

a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; or, a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof;

(36) Use of a compound or its salt that that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

(I) a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof; or,
(II) a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof;

(37) A method of regulating the secretion of prolactin, which comprises administering to a mammal a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; or, a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; and,

(38) A method of regulating the secretion of prolactin, which comprises administering to a mammal a compound or its salt that that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

(I) a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof; or,
(II) a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof.

Furthermore, the present invention provides the following.
(39) The agent according to (1), wherein substantially the same amino acid sequence as the amino acid sequence shown by SEQ ID NO: 1 is an amino acid sequence having homology of at least about 70%, preferably at least about 80%, more preferably at least about 90% and most preferably about 95%, to the amino acid sequence shown by SEQ ID NO:1;
(40) The agent according to (1), wherein substantially the same amino acid sequence as the amino acid sequence shown by SEQ ID NO:1 is (i) an amino acid sequence represented by SEQ ID NO:1, SEQ ID NO:8, SEQ ID NO: 14, SEQ ID NO:18, SEQ ID NO:33 or SEQ ID NO:50, of which 1 to 20 (preferably 1 to 15, more preferably 1 to 5 and most preferably 1 to 3) amino acids are deleted; (ii) an amino acid sequence represented by SEQ ID NO:1, SEQ ID NO:8, SEQ ID NO:14, SEQ ID NO:18, SEQ ID NO:33 or SEQ ID NO:50, to which 1 to 20 (preferably 1 to 15, more preferably 1 to 5 and most preferably 1 to 3) amino acids are added; (iii) an amino acid sequence represented by SEQ ID NO:1, SEQ ID NO:8, SEQ ID NO:14, SEQ ID NO:18, SEQ ID NO:33 or SEQ ID NO:50, into which 1 to 20 (preferably 1 to 15, more preferably 1 to 5 and most preferably 1 to 3) amino acids are inserted, (iv) an amino acid sequence represented by SEQ ID NO:1, SEQ ID NO:8, SEQ ID NO:14, SEQ ID NO:18, SEQ ID NO:33 or SEQ ID NO:50, in which 1 to 20 (preferably 1 to 15, more preferably 1 to 5 and most preferably 1 to 3) amino acids are substituted by other amino acids; and (v) a combination of the above amino acid sequences;

(36) The agent according to (17), wherein substantially the same amino acid sequence as the amino acid sequence shown by SEQ ID NO:37 is an amino acid sequence having homology of at least about 50%, preferably at least about 70%, more preferably at least about 80%, much more preferably at least about 90% and most preferably about 95%, to the amino acid sequence shown by SEQ ID NO:37;

(37) The agent according to (17), wherein substantially the same amino acid sequence as the amino acid sequence shown by SEQ ID NO:37 is (i) an amino acid sequence represented by SEQ ID NO:37, of which 1 to 20 (preferably 1 to 15, more preferably 1 to 5 and most preferably 1 to 3) amino acids are deleted; (ii) an amino acid sequence represented by SEQ ID NO:37, to which 1 to 20 (preferably 1 to 15, more preferably 1 to 5 and most preferably 1 to 3) amino acids are added; (iii) an amino acid sequence represented by SEQ ID NO:37, in which 1 to 20 (preferably 1 to 15, more preferably 1 to 5 and most preferably 1 to 3) amino acids are substituted by other amino acids; and (iv) a combination of the above amino acid sequences; etc.

## BRIEF DESCRIPTION OF THE DRAWINGS

FIG. 1 shows the base sequence of DNA encoding the polypeptide (human type) of the present invention obtained in REFERENCE EXAMPLE 2, and the amino acid sequence deduced from the base sequence.

FIG. 2 shows the hydrophobic plotting of the polypeptide of the present invention.

FIG. 3 shows the base sequence of DNA encoding the polypeptide (human type) of the present invention obtained in REFERENCE EXAMPLE 3, and the amino acid sequence deduced from the base sequence.

FIG. 4 shows the base sequence of DNA encoding the polypeptide (bovine type) of the present invention obtained in REFERENCE EXAMPLE 4, and the amino acid sequence deduced from the base sequence.

FIG. 5 shows the base sequence of DNA encoding the polypeptide (rat type) of the present invention obtained in REFERENCE EXAMPLE 5, and the amino acid sequence deduced from the base sequence.

FIG. 6 shows comparison among the amino acid sequences of the polypeptides of the present invention obtained in REFERENCE EXAMPLES 3, 4 and 5.

FIG. 7 shows the base sequence of DNA encoding the polypeptide (mouse type) of the present invention obtained in REFERENCE EXAMPLE 6, and the amino acid sequence deduced from the base sequence.

FIG. 8 shows the reactivity of peptides to r0T7T022L receptor-expressed CHO cells assayed by Cytosensor in REFERENCE EXAMPLE 7, in which •-• and Δ-Δ denote MPHSFANLPLRF amide (SEQ ID NO:39) and VPNLPQRF amide (SEQ ID NO:40), respectively.

FIG. 9 shows the activity of MPHSFANLPLRF amide (SEQ ID NO:39) and VPNLPQRF amide (SEQ ID NO: 40) for inhibiting cAMP production against the r0T7T022L-expressed CHO cells, assayed in REFERENCE EXAMPLE 10, in which □-□ and •-• denote MPHSFANLPLRF amide (SEQ ID NO:39) and VPNLPQRF amide (SEQ ID NO:40), respectively.

FIG. 10 shows the measurement results of prolactin level contained in plasma, which was carried out in EXAMPLE 1, wherein •-•denote the prolactin level in the group receiving PBS in which the peptide represented by SEQ ID NO:39 was dissolved, and o-o denotes the prolactin level in the control group receiving PBS alone.

Also, the time period for administration is made 0 minutes, and * indicates a risk percentage of $p<0.05$, and ** indicates a risk percentage of $p<0.01$.

FIG. 11 shows the results of the reactivity of an RF amide-related peptide in competitive EIA using anti-rat RFRP-1 monoclonal antibody 1F3, which was carried out in REFERENCE EXAMPLE 13.

To a 96-well plate coated with anti-mouse IgGAM antibody, 50 μl of anti-rat RFRP-1 monoclonal antibody and 50 μl of the peptide in concentrations indicated on the abscissa were added. After incubation at 4°C for 16 hours, HRP-rat RFRP-1 was added followed by incubation at room temperature for further 2 hours. After the plate was washed, the HRP activity was measured as absorbance at 450 nm. B denotes absorbance when the peptide was added and $B_0$ denotes absorbance when the peptide was not added.

In the figure, -•- denotes the peptide composed of 83 (Val) - 94 (Phe) amino acid sequence of the amino acid sequence shown by SEQ ID NO:50, in which the C-terminal carboxyl is amidated (VPHSAANLPLRF-$NH_2$), -▲- denotes

the peptide composed of 90 (Leu) - 94 (Phe) amino acid sequence of the amino acid sequence shown by SEQ ID NO: 50, in which the C-terminal carboxyl is amidated (LPLRF-$NH_2$), -■- denotes the peptide composed of 124 (Val) - 131 (Phe) amino acid sequence of the amino acid sequence shown by SEQ ID NO:50, in which the C-terminal carboxyl is amidated (VPNLPQRF-$NH_2$), and -◆- denotes the peptide composed of 128 (Pro) - 131 (Phe) amino acid sequence of the amino acid sequence shown by SEQ ID NO:50, in which the C-terminal carboxyl is amidated (PQRF-NH2).

FIG. 12 shows the chromatographic pattern of endogenous RFRP-1 finally purified from bovine hypothalamus, which was performed in EXAMPLE 2.

The chromatogram shows μRPC C2/dC18 SC 2.1/10 at the final purification step, wherein the ordinate indicates absorbance at 215 nm and the concentration of acetonitrile for elution, and the abscissa indicates retention time. In the figure, the black column shows RFRP-1-like immune activity when measured by competitive EIA using anti-rat RFRP-1 monoclonal antibody 1F3 in each fraction.

FIG. 13 shows the construction for plasmid pTFCRFRT-1 obtained in EXAMPLE 4.

FIG. 14 shows the activities of various peptides for inhibiting the increased cAMP production in cells by forskolin treatment performed in EXAMPLE 8, in which -o- denotes hRFRP-1-12 (peptide having the 81 (Met) to 92 (Phe) amino acid sequence in SEQ ID NO:1), -■- denotes hRFRP-1-37 (peptide having the 56 (Ser) to 92 (Phe) amino acid sequence in SEQ ID NO:1), -◊- denotes rRFRP-1-37 (peptide having the 58 (Ser) to 94 (Phe) amino acid sequence in SEQ ID NO:50), - ▲- denotes hRFRP-2-12 (peptide having the 101 (Phe) to 112 (Ser) amino acid sequence in SEQ ID NO:1), -□- denotes hRFRP-3-8 (peptide having the 124 (Val) to 131 (Phe) amino acid sequence in SEQ ID NO:1), -◆- denotes PQRFamide (peptide shown by Pro-Gln-Arg-Phe-$NH_2$), -●- denotes LPLRFamide (peptide shown by Leu-Pro-Leu-Arg-Phe-$NH_2$), and -▲- denotes NPFF (peptide shown by Asn-Pro-Phe-Phe).

FIG. 15 is the figure showing the effect of pertussis toxin on activation of human 0T7T022 receptor by RFRP peptide using as an indicator the cAMP production inhibiting activity, which was carried out in EXAMPLE 9.

BEST MODE OF EMBODIMENT OF THE INVENTION

The polypeptide of the present invention having the same or substantially the same amino acid sequence as the amino acid sequence shown by SEQ ID NO: 1 (hereinafter sometimes referred to as the polypeptide of the present invention) may be any polypeptide derived from any cells of human and other warm-blooded animals (e.g., guinea pig, rat, mouse, chicken, rabbit, swine, sheep, bovine, monkey, etc.) such as retina cells, liver cells, splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes, interstitial cells, etc., the corresponding precursor cells, stem cells, cancer cells, etc., or any tissues where such cells are present, such as brain or any of brain regions (e.g., retina, olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc.; polypeptides derived from hemocyte type cells or their cultured cells (e.g., MEL, M1, CTLL-2, HT-2, WEHI-3, HL-60, JOSK-1, K562, ML-1, MOLT-3, MOLT-4, MOLT-10, CCRF-CEM, TALL-1, Jurkat, CCRT-HSB-2, KE-37, SKW-3, HUT-78, HUT-102, H9, U937, THP-1, HEL, JK-1, CMK, KO-812, MEG-01, etc.); the polypeptides may also be synthetic polypeptides.

The amino acid sequence which has substantially the same amino acid sequence as the amino acid sequence represented by SEQ ID NO: 1 includes an amino acid sequence having at least about 70% homology, preferably at least about 80% homology, more preferably at least about 90% homology, and most preferably at least about 95% homology, to the amino acid sequence represented by SEQ ID NO:1.

Examples of substantially the same amino acid sequence as the amino acid sequence represented by SEQ ID NO: 1 include an amino acid sequence containing the 22-180 amino acid sequence of the amino acid sequence represented by SEQ ID NO:1, etc.

More specifically, substantially the same amino acid sequence as that represented by SEQ ID NO: 1 includes amino acid sequences represented by SEQ ID NO:8, SEQ ID NO:14, SEQ ID NO:18, SEQ ID NO:33 or SEQ ID NO: 50, and the like.

Examples of the polypeptide which has substantially the same amino acid sequence as that represented by SEQ ID NO: include a polypeptide having substantially the same amino acid sequence as that represented by SEQ ID NO: 1 (e.g., amino acid sequence shown by SEQ ID NO:8, SEQ ID NO:14, SEQ ID NO:18, SEQ ID NO:33 or SEQ ID NO:50) and having the prolactin secretion regulating activity substantially equivalent to the polypeptide containing the amino acid sequence represented by SEQ ID NO: 1.

The term substantially equivalent is used to mean that the nature of these activities is equivalent (for example, biochemically or pharmacologically). It is thus preferred that the prolactin secretion regulating activity is equivalent (e.g., about 0.1 to about 100 times, preferably about 0.5 to about 10 times, more preferably about 0.5 to about 2 times), and it is allowable that differences among grades such as the strength of these activities, molecular weight of the polypeptide, etc. are even present.

The prolactin secretion regulating activity can be determined according to a modification from publicly known methods, for example, in accordance with EXAMPLE 1, which will be later described.

Examples of the polypeptides of the present invention include polypeptides containing (i) an amino acid sequence represented by SEQ ID NO:1, SEQ ID NO:8, SEQ ID NO:14, SEQ ID NO:18, SEQ ID NO:33 or SEQ ID NO:50, of which 1 to 20 (preferably 1 to 15, more preferably 1 to 5 and most preferably 1 to 3) amino acids are deleted; (ii) an amino acid sequence represented by SEQ ID NO:1, SEQ ID NO:8, SEQ ID NO:14, SEQ ID NO:18, SEQ ID NO:33 or SEQ ID NO:50, to which 1 to 20 (preferably 1 to 15, more preferably 1 to 5 and most preferably 1 to 3) amino acids are added; (iii) an amino acid sequence represented by SEQ ID NO:1, SEQ ID NO:8, SEQ ID NO:14, SEQ ID NO:18, SEQ ID NO:33 or SEQ ID NO:50, into which 1 to 20 (preferably 1 to 15, more preferably 1 to 5 and most preferably 1 to 3) amino acids are inserted, (iv) an amino acid sequence represented by SEQ ID NO:1, SEQ ID NO:8, SEQ ID NO:14, SEQ ID NO:18, SEQ ID NO:33 or SEQ ID NO:50, in which 1 to 20 (preferably 1 to 15, more preferably 1 to 5 and most preferably 1 to 3) amino acids are substituted by other amino acids; and (v) so-called muteins such as polypeptides containing a combination of the above amino acid sequences.

When an amino acid sequence(s) are inserted, deleted or substituted as described above, the positions of such insertion, deletion or substitution are not particularly limited.

Specific examples of the polypeptide which contains substantially the same amino acid sequence as the amino acid sequence shown by SEQ ID NO:1 are a polypeptide containing substantially the same amino acid sequence as that shown by SEQ ID NO:8, a polypeptide containing substantially the same amino acid sequence as that shown by SEQ ID NO:14, a polypeptide containing substantially the same amino acid sequence as that shown by SEQ ID NO:18, a polypeptide containing substantially the same amino acid sequence as that shown by SEQ ID NO:33, a polypeptide containing substantially the same amino acid sequence as that shown by SEQ ID NO:50, and the like.

Throughout the present specification, the polypeptides are represented in accordance with the conventional way of describing polypeptides, that is, the N-terminus (amino terminus) at the left hand and the C-terminus (carboxyl terminus) at the right hand. In the polypeptides of the present invention including the polypeptide containing the amino acid sequence shown by SEQ ID NO:1, the C-terminus is usually in the form of a carboxyl group (-COOH) or a carboxylate ($-COO^-$) but may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; a $C_{7-14}$ aralkyl such as a phenyl-$C_{1-2}$ alkyl group, e.g., benzyl, phenethyl, etc.; an α-naphthyl-$C_{1-2}$ alkyl group such as α-naphthylmethyl, etc.; and the like. In addition, pivaloyloxymethyl or the like which is used widely as an ester for oral administration may also be used.

Where the polypeptide of the present invention contains a carboxyl group (or a carboxylate) at a position other than the C-terminus, it may be amidated or esterified and such an amide or ester is also included within the polypeptide of the present invention. The ester group may be the same group as that described with respect to the above C-terminal.

Furthermore, examples of the polypeptide of the present invention include variants of the above polypeptides, wherein the amino group at the N-terminus (e.g., methionine residue) of the polypeptide is protected with a protecting group (e.g., a $C_{1-6}$ acyl group such as a $C_{1-6}$ alkanoyl group e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{1-6}$ alkanoyl group , e.g., formyl group, acetyl group, etc.), or conjugated proteins such as glycoproteins having sugar chains. These polypeptides are sometimes merely referred to as the polypeptide of the present invention.

Specific examples of the polypeptide of the present invention include a human-derived polypeptide containing the amino acid sequence represented by SEQ ID NO:1 (FIG. 1), a human-derived polypeptide containing the amino acid sequence represented by SEQ ID NO:8 (FIG. 3), a bovine-derived polypeptide containing the amino acid sequence represented by SEQ ID NO:14 (FIG. 4), a rat-derived polypeptide containing the amino acid sequence represented by SEQ ID NO: 18 (FIG. 5), a mouse-derived polypeptide containing the amino acid sequence represented by SEQ ID NO:33 (FIG.7), a rat-derived polypeptide containing the amino acid sequence represented by SEQ ID NO:50, etc. Preferably employed are, for example, the human-derived polypeptide containing the amino acid sequence represented by SEQ ID NO:1, the human-derived polypeptide containing the amino acid sequence represented by SEQ ID NO:8, and the bovine-derived polypeptide containing the amino acid sequence represented by SEQ ID NO:14.

The partial peptides of the polypeptides of the present invention (hereinafter sometimes referred to as the partial peptide of the present invention) may be any partial peptides of the polypeptides of the present invention described above, and those having the prolactin secretion regulating activity, which is expressed by adding the receptors of the polypeptide of the present invention (specifically, proteins containing the same or substantially the same amino acid sequence as the amino acid sequence shown by SEQ ID NO:37, or salts thereof.

The partial peptide of the present invention may contain an amino acid sequence, wherein 1 to 5 (preferably 1 to 3) amino acids are deleted, an amino acid sequence, to which 1 to 5 (preferably 1 to 3) amino acids are added, an amino acid sequence, wherein 1 to 5 (preferably 1 to 3) amino acids are inserted, or an amino acid sequence, wherein 1 to 5 (preferably I to 3) amino acids are substituted by other amino acids. The partial peptide may contain a combination of the above amino acid sequences.

In the partial peptide of the present invention, the C-terminus is normally a carboxyl group (-COOH) or carboxylate ($-COO^-$) but the C-terminus may be in the form of an amide ($-CONH_2$) or an ester (-COOR)(wherein R has the same significance as defined above), as has been described with the polypeptide of the present invention. In particular, preferred are the partial peptides having an amide ($-CONH_2$) at the C-terminus.

Where the partial peptide of the receptor protein of the present invention contains a carboxyl group (or a carboxylate) at a position other than the C-terminus, it may be amidated or esterified and such an amide or ester is also included within the receptor protein of the present invention. The ester group may be the same group as the ester described with respect to the above C-terminal.

As in the polypeptide of the present invention described above, the partial peptide of the present invention further includes conjugated peptides such as those in which the amino group of the N-terminal amino acid residue (e. g., methionine residue) is protected by a protecting group, those in which the N-terminal residue is cleaved in vivo and the produced glutamine residue is pyroglutaminated, those in which substituents on the side chains of amino acids in the molecule are protected by appropriate protecting groups and conjugated proteins such as so-called glycoproteins having sugar chains. Hereinafter, these partial peptides are sometimes merely referred to as the partial peptide of the present invention.

As the partial peptide of the polypeptide of the present invention, preferred are peptides containing the RF amide, RS amide or RL amide structure, more preferably peptides containing the RF amide or RS amide structure, and most preferably peptides containing the RF amide structure.

The RF amide structure refers to the peptide structure, the C-terminus of which takes an arginine-phenylalanine-$NH_2$ structure. The RS amide structure is used to mean the peptide structure, the C-terminus of which takes an arginine-serine-$NH_2$ structure. In the RL amide structure, the C-terminus of the peptide takes an arginine-leucine-$NH_2$ structure.

Among these peptides, preferred examples include:

(1) a peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe), 124 (Val) - 131 (Phe), 1 (Met) - 92 (Phe), 1 (Met)-112 (Ser) or 1 (Met) - 131 (Phe) in the amino acid sequence shown by SEQ ID NO:1;
(2) a peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe), 124 (Val) - 131 (Phe), 1 (Met) - 92 (Phe), 1 (Met) - 112 (Ser) or 1 (Met) - 131 (Phe) in the amino acid sequence shown by SEQ ID NO:8;
(3) a peptide containing the amino acid sequence of 58 (Ser) - 92 (Phe), 81 (Met) - 92 (Phe), 101 (Ser) - 112 (Leu), 124 (Val) - 131 (Phe), 1 (Met) - 92 (Phe) or 1 (Met) - 131 (Phe) in the amino acid sequence shown by SEQ ID NO:14;
(4) a peptide containing the amino acid sequence of 58 (Ser) - 94 (Phe), 83 (Val) - 94 (Phe), 84 (Pro) - 94 (Phe) or 118 (Phe) - 125 (Phe) in the amino acid sequence shown by SEQ ID NO:33;
(5) a peptide containing the amino acid sequence of 58 (Ser) - 94 (Phe) or 84 (Pro) - 94 (Phe) in the amino acid sequence shown by SEQ ID NO:50.

Particularly preferred are:

(1) a peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe) or 124 (Val) - 131 (Phe) in the amino acid sequence shown by SEQ ID NO:1;
(2) a peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe) or 124 (Val) -131 (Phe) in the amino acid sequence shown by SEQ ID NO:8;
(3) a peptide containing the amino acid sequence of 58 (Ser) - 92 (Phe), 81 (Met) - 92 (Phe), 101 (Ser) - 112 (Leu) or 124 (Val) - 131 (Phe) in the amino acid sequence shown by SEQ ID NO:14;
(4) a peptide containing the amino acid sequence of 58 (Ser) - 94 (Phe), 83 (Val) - 94 (Phe), 84 (Pro) - 94 (Phe) or 118 (Phe) - 125 (Phe) in the amino acid sequence shown by SEQ ID NO:33;
(5) a peptide containing the amino acid sequence of 58 (Ser) - 94 (Phe) or 84 (Pro) - 94 (Phe) in the amino acid sequence shown by SEQ ID NO:50; and the like;

among others, preferred are:

(1) a peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe) or 124 (Val) - 131 (Phe) in the amino acid sequence shown by SEQ ID NO:1;
(2) a peptide containing the amino acid sequence of 56 (Ser) - 92 (Phe), 73 (Met) - 92 (Phe), 81 (Met) - 92 (Phe), 84 (Ser) - 92 (Phe), 101 (Ser) -112 (Ser), 115 (Asn) - 131 (Phe) or 124 (Val) - 131 (Phe) in the amino acid sequence shown by SEQ ID NO:8;
(3) a peptide containing the amino acid sequence of 58 (Ser) - 92 (Phe), 81 (Met) - 92 (Phe), 101 (Ser) - 112 (Leu) or 124 (Val) - 131 (Phe) in the amino acid sequence shown by SEQ ID NO:14;
(4) a peptide containing the amino acid sequence of 58 (Ser) - 94 (Phe), 83 (Val) - 94 (Phe) or 118 (Phe) - 125 (Phe) in the amino acid sequence shown by SEQ ID NO:33;
(5) a peptide containing the amino acid sequence of 58 (Ser) - 94 (Phe) in the amino acid sequence shown by SEQ ID NO:50; and the like;

among others, preferred are:

(1) a peptide containing the amino acid sequence of 56 (Ser) - 92 (Phe), 73 (Met) - 92 (Phe), 81 (Met) - 92 (Phe), 84 (Ser) - 92 (Phe), 115 (Asn) - 131 (Phe) or 124 (Val) - 131 (Phe) in the amino acid sequence shown by SEQ ID NO:1;
(2) a peptide containing the amino acid sequence of 56 (Ser) - 92 (Phe), 73 (Met) - 92 (Phe), 81 (Met) - 92 (Phe), 84 (Ser) - 92 (Phe), 115 (Asn) - 131 (Phe) or 124 (Val) - 131 (Phe) in the amino acid sequence shown by SEQ ID NO:8;
(3) a peptide containing the amino acid sequence of 58 (Ser) - 92 (Phe), 81 (Met) - 92 (Phe) or 124 (Val) - 131 (Phe) in the amino acid sequence shown by SEQ ID NO:14;

among others, preferred are:

(1) a peptide containing the amino acid sequence of 56 (Ser) - 92 (Phe), 73 (Met) - 92 (Phe), 81 (Met) - 92 (Phe) or 84 (Ser) - 92 (Phe) in the amino acid sequence shown by SEQ ID NO:1;
(2) a peptide containing the amino acid sequence of 56 (Ser) - 92 (Phe), 73 (Met) - 92 (Phe), 81 (Met) - 92 (Phe) or 84 (Ser) - 92 (Phe) in the amino acid sequence shown by SEQ ID NO:8;
(3) a peptide containing the amino acid sequence of 58 (Ser) - 92 (Phe) or 81 (Met) - 92 (Phe) in the amino acid sequence shown by SEQ ID NO:14;

among others, preferred are:

(1) a peptide containing the amino acid sequence of 81 (Met) - 92 (Phe) in the amino acid sequence shown by SEQ ID NO:1;
(2) a peptide containing the amino acid sequence of 81 (Met) - 92 (Phe), 84 (Ser) - 92 (Phe) in the amino acid sequence shown by SEQ ID NO:8;
(3) a peptide containing the amino acid sequence of 81 (Met) - 92 (Phe) in the amino acid sequence shown by SEQ ID NO:14;

among others, preferred are:

(1) a peptide containing the amino acid sequence of 81 (Met) - 92 (Phe) in the amino acid sequence shown by SEQ ID NO:1;
(2) a peptide containing the amino acid sequence of 81 (Met) - 92 (Phe), 84 (Ser) - 92 (Phe) in the amino acid sequence shown by SEQ ID NO:8.

Also, preferred examples of the partial peptide of the present invention include:

(a) a peptide containing the amino acid sequence of 81 (Met) - 92 (Phe) in the amino acid sequence shown by SEQ ID NO:1;
(b) a peptide containing the amino acid sequence of 101 (Ser) - 112 (Ser) in the amino acid sequence shown by SEQ ID NO:1;
(c) a peptide containing the amino acid sequence of 124 (Val) - 131 (Phe) in the amino acid sequence shown by SEQ ID NO:1;

(d) a peptide containing the amino acid sequence of 56 (Ser) - 92 (Phe) in the amino acid sequence shown by SEQ ID NO:1;

(e) a peptide containing the amino acid sequence of 81 (Met) - 92 (Phe) in the amino acid sequence shown by SEQ ID NO:14;

(f) a peptide containing the amino acid sequence of 101 (Ser) - 112 (Leu) in the amino acid sequence shown by SEQ ID NO:14;

(g) a peptide containing the amino acid sequence of 58 (Ser) - 92 (Phe) in the amino acid sequence shown by SEQ ID NO:14;

(h) a peptide containing the amino acid sequence of 83 (Val) - 94 (Phe) in the amino acid sequence shown by SEQ ID NO:33;

(i) a peptide containing the amino acid sequence of 118 (Phe) - 125 (Phe) in the amino acid sequence shown by SEQ ID NO:33;

(j) a peptide containing the amino acid sequence of 58 (Ser) - 94 (Phe) in the amino acid sequence shown by SEQ ID NO:33;

(k) a peptide containing the amino acid sequence of 58 (Ser) - 94 (Phe) in the amino acid sequence shown by SEQ ID NO:50; and the like.

In particular, the amides of these peptides are desirable (preferably, peptides wherein the C-terminal carboxyl group (-COOH) is amidated (-$CONH_2$)).

Specific examples include a peptide (SEQ ID NO:39) having the 81 (Met) -92 (Phe) amino acid sequence in SEQ ID NO:1, wherein the C-terminus is amidated (-$CONH_2$), a peptide (SEQ ID NO:41) having the 101 (Ser) - 112 (Ser) amino acid sequence in SEQ ID NO:1, wherein the C-terminus is amidated (-$CONH_2$); a peptide (SEQ ID NO: 40) having the 124 (Val) - 131 (Phe) amino acid sequence in SEQ ID NO: wherein the C-terminus is amidated (-$CONH_2$), and the like.

Among others, preferred are the peptide (SEQ ID NO:39) having the 81 (Met) - 92 (Phe) amino acid sequence in SEQ ID NO:1, wherein the C-terminus is amidated (-$CONH_2$) and the peptide (SEQ ID NO:40) having the 124 (Val) - 131 (Phe) amino acid sequence in SEQ ID NO: 1 wherein the C-terminus is amidated (-$CONH_2$), with particular preference of the peptide (SEQ ID NO:39) having the 81 (Met) - 92 (Phe) amino acid sequence in SEQ ID NO:1, wherein the C-terminus is amidated (-$CONH_2$).

As salts of the polypeptide of the present invention or the partial peptide of the present invention, there may be used salts with physiologically acceptable acids (e.g., inorganic acids or organic acids) or bases (e.g., alkali metal salts), preferably in the form of physiologically acceptable acid addition salts. Examples of such salts are salts with inorganic acids (e.g., hydrochloric acid, phosphoric acid, hydrobromic acid, sulfuric acid), salts with organic acids (e. g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The polypeptide of the present invention or salts thereof, or the partial peptide of the present invention or salts thereof may be manufactured by a publicly known method used to purify polypeptides from human or other warm-blooded animal cells or tissues described above. Alternatively, they may also be manufactured by culturing transformants containing DNAs encoding the polypeptides, which will be later described. Furthermore, they may also be manufactured by a modification of the methods for peptide synthesis, which will also be described hereinafter.

Where the polypeptide or partial peptide, or salts thereof are manufactured from human or mammalian tissues or cells, human or mammalian tissues or cells are homogenized, then extracted with an acid or the like, and the extract is isolated and purified by a combination of chromatography techniques such as reversed phase chromatography, ion exchange chromatography, and the like.

To synthesize the polypeptide of the present invention or salts thereof, or the partial peptide of the present invention or salts thereof, commercially available resins that are generally used for polypeptide synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmethylphenyl acetamidomethyl resin, polyacrylamide resin, 4-(2',4'-dimethoxyphenyl-hydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective polypeptide according to various condensation methods publicly known in the art. At the end of the reaction, the polypeptide is excised from the resin and at the same time, the protecting groups are removed. Then, intramolecular disulfide bond-forming reaction is performed in a highly diluted solution to obtain the objective polypeptide of the present invention or salts thereof, or the partial peptide of the present invention or salts thereof.

For condensation of the protected amino acids described above, a variety of activation reagents for polypeptide synthesis may be used, but carbodiimides are particularly preferably employed. Examples of such carbodiimides include DCC, N,N'-diisopropylcarbodiimide, N-ethyl-N'-(3-dimethylaminopropyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents that are known to be usable for polypeptide condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to polypeptide bond-forming reactions and is usually selected in the range of approximately -20°C to 50°C. The activated amino acid derivatives are used generally in an excess of 1.5 to 4 times. The condensation is examined using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole to cancel any possible adverse affect on the subsequent reaction.

Examples of the protecting groups used to protect the starting amino groups include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower $C_{1-6}$ alkanoyl group , such as acetyl group, an aroyl group such as benzoyl group, and a group derived from carbonic acid such as benzyloxycarbonyl group and ethoxycarbonyl group. Examples of a group appropriately used for the etherification include benzyl group, tetrahydropyranyl group, t-butyl group, etc.

Examples of groups for protecting the phenolic hydroxyl group of tyrosine include Bzl, $Cl_2$-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting amino acids include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], and the like. As the activated amino acids in which the amino groups are activated in the starting material, for example, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethanesulfonic acid or trifluoroacetic acid, or a mixture solution of these acids, etc.; a treatment with a base such as diisopropylethylamine, triethylamine, piperidine, piperazine, etc.; and reduction with sodium in liquid ammonia, etc. The elimination of the protecting group by the acid treatment described above is carried out generally at a temperature of approximately -20°C to 40°C, In the acid treatment, it is efficient to add a cation scavenger such as anisole, phenol, thioanisole, m-cresol, p-cresol, dimethylsulfide, 1,4-butanedithiol, 1,2-ethanedithiol, etc. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol, etc. as well as by a treatment with an alkali such as a dilute sodium hydroxide solution, dilute ammonia, etc.

Protection of functional groups that should not be involved in the reaction of the starting materials, protecting groups, elimination of the protecting groups, activation of functional groups participated in the reaction, etc. may be appropriately selected from publicly known groups or publicly known means.

In another method for obtaining the amides of the polypeptide or partial peptide of the present invention, for example, the α-carboxyl group of the carboxy terminal amino acid is first protected by amidation; the peptide (polypeptide) chain is then extended from the amino group side to a desired length. Thereafter, a polypeptide in which only the protecting group of the N-terminal α-amino group has been eliminated from the peptide chain and a polypeptide in which only the protecting group of the C-terminal carboxyl group has been eliminated are manufactured. The two polypeptides are condensed in a mixture of the solvents described above. The details of the condensation reaction are the same as described above. After the protected polypeptide obtained by the condensation is purified, all the protecting groups are eliminated by the method described above to give the desired crude polypeptide. This crude polypeptide is purified by various known purification means. Lyophilization of the major fraction gives the amide of the desired polypeptide or partial peptide.

To prepare the esterified polypeptide or partial peptide of the present invention, for example, the α-carboxyl group of the carboxy terminal amino acid is condensed with a desired alcohol to prepare the amino acid ester, which is followed by procedure similar to the preparation of the amidated polypeptide above to give the desired esterified polypeptide or partial peptide.

The partial peptide or salts of the present invention can be manufactured by publicly known methods for peptide synthesis, or by cleaving the polypeptide of the present invention with an appropriate peptidase. For the methods for peptide synthesis, for example, either solid phase synthesis or liquid phase synthesis may be used. That is, the partial peptide or amino acids that can construct the partial peptide of the present invention are condensed with the remaining part of the partial peptide. Where the product contains protecting groups, these protecting groups are removed to give the desired peptide. Publicly known methods for condensation and elimination of the protecting groups are described in 1) - 5) below.

1) M. Bodanszky & M.A. Ondetti: Peptide Synthesis, Interscience Publishers, New York (1966)
2) Schroeder & Luebke: The Peptide, Academic Press, New York (1965)
3) Nobuo Izumiya, et al.: *Peptide Gosei-no-Kiso to Jikken* (Basics and experiments of peptide synthesis), published by Maruzen Co. (1975)
4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
5) Haruaki Yajima ed.: *Zoku Iyakuhin no Kaihatsu* (A sequel to Development of Pharmaceuticals), Vol. 14, Peptide Synthesis, published by Hirokawa Shoten

After completion of the reaction, the product may be purified and isolated by a combination of conventional purification methods such as solvent extraction, distillation, column chromatography, liquid chromatography and recrystallization to give the partial peptide of the present invention. When the partial peptide obtained by the above methods is in a free form, the peptide can be converted into an appropriate salt by a publicly known method; when the protein is obtained in a salt form, it can be converted into a free form or a different salt form by a publicly known method.

The DNA encoding the polypeptide of the present invention may be any DNA, so far as it contains the base sequence encoding the polypeptide of the present invention described above. Such a DNA may also be any one of genomic DNA, genomic DNA library, cDNA derived from the cells or tissues described above, cDNA library derived from the cells or tissues described above and synthetic DNA.

The vector to be used for the library may be any of bacteriophage, plasmid, cosmid, phagemid and the like. In addition, the DNA can be amplified by reverse transcriptase polymerase chain reaction (hereinafter abbreviated as RT-PCR) with total RNA or mRNA fraction prepared from the above-described cells or tissues.

Specifically, the DNA encoding the polypeptide of the present invention may be any one of, for example, DNA having the base sequence represented by SEQ ID NO:2, SEQ ID NO:9,SEQ ID NO:15,SEQ ID NO:19, SEQ ID NO: 34 or SEQ ID NO:51 or any DNA having a base sequence hybridizable to the base sequence represented by SEQ ID NO:2, SEQ ID NO;9, SEQ ID NO:15, SEQ ID NO:19, SEQ ID NO:34 or SEQ ID NO:51 under high stringent conditions and encoding a polypeptide, which has the activity substantially equivalent to those of the polypeptide of the present invention (e.g., a cell stimulating activity, etc.).

Specific examples of the DNA that is hybridizable to the base sequence represented by SEQ ID NO:2, SEQ ID NO:9, SEQ ID NO:15, SEQ ID NO:19, SEQ ID NO:34 or SEQ ID NO:51 under high stringent conditions include DNA having at least about 70% homology, preferably at least about 80% homology, more preferably at least about 90% homology and most preferably at least about 95% homology, to the base sequence represented by SEQ ID NO:2.

The hybridization can be carried out by publicly known methods or by a modification thereof, for example, in accordance with the method described in Molecular Cloning, 2nd ed., J. Sambrook et al., Cold Spring Harbor Lab. Press, (1989). A commercially available library may also be used according to the instructions of the attached manufacturer's protocol. Preferably, the hybridization can be carried out under high stringent conditions.

The high stringent conditions used herein are, for example, those in a sodium concentration at about 19 to about 40 mM, preferably about 19 to about 20 mM at a temperature of about 50 to about 70°C, preferably about 60 to about 65°C. In particular, hybridization conditions in a sodium concentration at about 19 mM at a temperature of about 65°C are most preferred.

More specifically, for the DNA encoding the polypeptide having the amino acid sequence represented by SEQ ID NO:1, there may be employed DNA having the base sequence represented by SEQ ID NO:2, etc. and, DNA having the base sequence represented by SEQ ID NO:9, etc. may be used for the DNA encoding the polypeptide having the amino acid sequence represented by SEQ ID NO:8. For the DNA encoding the polypeptide having the amino acid sequence represented by SEQ ID NO:14, DNA having the base sequence represented by SEQ ID NO:15, etc. may be employed and, DNA having the base sequence represented by SEQ ID NO:19, etc. may be used as the DNA encoding the polypeptide having the amino acid sequence represented by SEQ ID NO:18. As the DNA encoding the polypeptide having the amino acid sequence represented by SEQ ID NO:33, there may be employed DNA having the base sequence represented by SEQ ID NO:34, etc., and DNA having the base sequence represented by SEQ ID NO: 51, etc. may be used for the DNA encoding the polypeptide having the amino acid sequence represented by SEQ ID NO:50.

The DNA encoding the partial peptide of the present invention may be any DNA, so long as it contains the base sequence encoding the partial peptide of the present invention described above. The DNA may also be any of genomic DNA, genomic DNA library, cDNA derived from the cells and tissues described above, cDNA library derived from the cells and tissues described above and synthetic DNA.

Specifically, the DNA encoding the partial peptide of the present invention may be any one of, for example, a DNA having a partial base sequence of the DNA having the base sequence represented by SEQ ID NO:2, SEQ ID NO:9 SEQ ID NO:15, SEQ ID NO:19, SEQ ID NO:34 or SEQ ID NO:51 or any DNA having a partial base sequence of the DNA having a base sequence hybridizable to the base sequence represented by SEQ ID NO:2, SEQ ID NO:9, SEQ ID NO:15, SEQ ID NO:19, SEQ ID NO:34 or SEQ ID NO:51 under high stringent conditions and encoding a polypeptide which has the activity substantially equivalent to that of the polypeptide of the present invention.

Specific examples of the DNA that is hybridizable to the base sequence represented by SEQ ID NO:2, SEQ ID NO:9, SEQ ID NO:15, SEQ ID NO:19, SEQ ID NO:34 or SEQ ID NO:51 are the same as those described above.

Methods for the hybridization and the high stringent conditions that can be used are also the same as described above.

The polypeptide encoded by the DNA that is hybridizable to the base sequence represented by SEQ ID NO: 2, SEQ ID NO:9, SEQ ID NO:15, SEQ ID NO:19, SEQ ID NO:34 or SEQ ID NO:51 can be manufactured in a manner similar to the method for manufacturing the polypeptide of the present invention, which will be described hereinafter. Examples of the amides, esters and salts of the polypeptide include those as described for the amides, esters and salts of the polypeptide of the present invention described above.

Specifically, the DNA encoding the partial peptide of the present invention includes:

(1) a DNA encoding the peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe), 124 (Val) - 131 (Phe), 1 (Met) - 92 (Phe), 1 (Met) - 112 (Ser) or 1 (Met) - 131 (Phe) in the amino acid sequence shown by SEQ ID NO:1; or a DNA containing the base sequence hybridizable thereto under high stringent conditions;
(2) a DNA encoding the peptide containing the amino acid sequence of 56 (Ser) - 92 (Phe), 73 (Met) - 92 (Phe), 81 (Met) - 92 (Phe), 84 (Ser) - 92 (Phe), 101 (Ser) - 112 (Ser), 115 (Asn) - 131 (Phe), 124 (Val) - 131 (Phe), 1 (Met) - 92 (Phe), 1 (Met) - 112 (Ser) or 1 (Met) - 131 (Phe) in the amino acid sequence shown by SEQ ID NO:8; or a DNA containing the base sequence hybridizable thereto under high stringent conditions;
(3) a DNA encoding the peptide containing the amino acid sequence of 58 (Ser) - 92 (Phe), 81 (Met) - 92 (Phe), 101 (Ser) - 112 (Leu), 124 (Val) - 131 (Phe), 1 (Met) - 92 (Phe) or 1 (Met) - 131 (Phe) in the amino acid sequence shown by SEQ ID NO:14; or a DNA containing the base sequence hybridizable thereto under high stringent conditions;
(4) a DNA encoding the peptide containing the amino acid sequence of 58 (Ser) - 94 (Phe), 83 (Val) - 94 (Phe), 84 (Pro) - 94 (Phe) or 118 (Phe) - 125 (Phe) in the amino acid sequence shown by SEQ ID NO:33; or a DNA containing the base sequence hybridizable thereto under high stringent conditions;
(5) a DNA encoding the peptide containing the amino acid sequence of 58 (Ser) - 94 (Phe) or 84 (Pro) - 94 (Phe) in the amino acid sequence shown by SEQ ID NO:50; and the like.

More specifically, the DNA includes:

as the DNA encoding the peptide containing the 81 (Met) - 92 (Phe) amino acid sequence in the amino acid sequence represented by SEQ ID NO:1, a DNA containing the base sequence represented by SEQ ID NO:42 (the 241-276 base sequence in the base sequence represented by SEQ ID NO:2);
as the DNA encoding the peptide containing the 101 (Ser) - 112 (Ser) amino acid sequence in the amino acid sequence represented by SEQ ID NO:1, a DNA containing the base sequence represented by SEQ ID NO:43 (the 301-336 base sequence in the base sequence represented by SEQ ID NO:2);
as the DNA encoding the peptide containing the 124 (Val) - 131 (Phe) amino acid sequence in the amino acid sequence represented by SEQ ID NO:1, a DNA containing the base sequence represented by SEQ ID NO:44 (the 370-393 base sequence in the base sequence represented by SEQ ID NO:2);

as the DNA encoding the peptide containing the 1 (Met) - 92 (Phe) amino acid sequence in the amino acid sequence represented by SEQ ID NO:1, a DNA containing the base sequence represented by SEQ ID NO:45 (the 1-276 base sequence in the base sequence represented by SEQ ID NO:2);
as the DNA encoding the peptide containing the 1 (Met) - 112 (Ser) amino acid sequence in the amino acid sequence represented by SEQ ID NO:1, a DNA containing the base sequence represented by SEQ ID NO:46 (the 1-336 3 base sequence in the base sequence represented by SEQ ID NO:2); and,
as the DNA encoding the peptide containing the 1 (Met) - 131 (Phe) amino acid sequence in the amino acid sequence represented by SEQ ID NO:1, a DNA containing the base sequence represented by SEQ ID NO:47 (the 1-393 base sequence in the base sequence represented by SEQ ID NO:2); and the like.

The DNA further includes:

as the DNA encoding the peptide containing the 56 (Ser) - 92 (Phe) amino acid sequence in the amino acid sequence represented by SEQ ID NO:1, a DNA containing the 166-276 base sequence represented by SEQ ID NO:2;
as the DNA encoding the peptide containing the 73 (Met) - 92 (Phe) amino acid sequence in the amino acid sequence represented by SEQ ID NO:1, a DNA containing the 217-276 base sequence represented by SEQ ID NO:2;
as the DNA encoding the peptide containing the 84 (Ser) - 92 (Phe) amino acid sequence in the amino acid sequence represented by SEQ ID NO:1, a DNA containing the 253-276 base sequence represented by SEQ ID NO:2;
as the DNA encoding the peptide containing the 115 (Asn) - 131 (Phe) amino acid sequence in the amino acid sequence represented by SEQ ID NO:1, a DNA containing the 346-393 base sequence represented by SEQ ID NO:2;
as the DNA encoding the peptide containing the 56 (Ser) - 92 (Phe) amino acid sequence in the amino acid sequence represented by SEQ ID NO:8, a DNA containing the 169-276 base sequence represented by SEQ ID NO:9;
as the DNA encoding the peptide containing the 73 (Met) - 92 (Phe) amino acid sequence in the amino acid sequence represented by SEQ ID NO:8, a DNA containing the 220-276 base sequence represented by SEQ ID NO:9;
as the DNA encoding the peptide containing the 81 (Met) - 92 (Phe) amino acid sequence in the amino acid sequence represented by SEQ ID NO:8, a DNA containing the 244-276 base sequence represented by SEQ ID NO:9;
as the DNA encoding the peptide containing the 84 (Ser) - 92 (Phe) amino acid sequence in the amino acid sequence represented by SEQ ID NO:8, a DNA containing the 253-276 base sequence represented by SEQ ID NO:9;
as the DNA encoding the peptide containing the 101 (Ser) - 112 (Ser) amino acid sequence in the amino acid sequence represented by SEQ ID NO:8, a DNA containing the 304-336 base sequence represented by SEQ ID NO:9;
as the DNA encoding the peptide containing the 115 (Asn) - 131 (Phe) amino acid sequence in the amino acid sequence represented by SEQ ID NO:8, a DNA containing the 346-393 base sequence represented by SEQ ID NO:9;
as the DNA encoding the peptide containing the 124 (Val) - 131 (Phe) amino acid sequence in the amino acid sequence represented by SEQ ID NO:8, a DNA containing the 373-393 base sequence represented by SEQ ID NO:9;
as the DNA encoding the peptide containing the 1 (Met) - 92 (Phe) amino acid sequence in the amino acid sequence represented by SEQ ID NO:8, a DNA containing the 1-276 base sequence represented by SEQ ID NO:9;
as the DNA encoding the peptide containing the 1 (Met) - 112 (Ser) amino acid sequence in the amino acid sequence represented by SEQ ID NO:8, a DNA containing the 1-336 base sequence represented by SEQ ID NO:9;
as the DNA encoding the peptide containing the 1 (Met) - 131 (Phe) amino acid sequence in the amino acid sequence represented by SEQ ID NO:8, a DNA containing the 1-393 base sequence represented by SEQ ID NO:9;
as the DNA encoding the peptide containing the 58 (Ser) - 92 (Phe) amino acid sequence in the amino acid sequence represented by SEQ ID NO: 14, a DNA containing the 172-276 base sequence represented by SEQ ID NO:15;
as the DNA encoding the peptide containing the 81 (Met) - 92 (Phe) amino acid sequence in the amino acid sequence represented by SEQ ID NO: 14, a DNA containing the 241-276 base sequence represented by SEQ ID NO:15;
as the DNA encoding the peptide containing the 101 (Ser) - 112 (Leu) amino acid sequence in the amino acid sequence represented by SEQ ID NO:14, a DNA containing the 301-336 base sequence represented by SEQ ID NO:15;
as the DNA encoding the peptide containing the 124 (Val) - 131 (Phe) amino acid sequence in the amino acid sequence represented by SEQ ID NO:14, a DNA containing the 370-393 base sequence represented by SEQ ID NO:15;

as the DNA encoding the peptide containing the 1 (Met) - 92 (Phe) amino acid sequence in the amino acid sequence represented by SEQ ID NO:14, a DNA containing the 1-276 base sequence represented by SEQ ID NO:15;

as the DNA encoding the peptide containing the 1 (Met) - 131 (Phe) amino acid sequence in the amino acid sequence represented by SEQ ID NO:14, a DNA containing the 1-393 base sequence represented by SEQ ID NO:15;

as the DNA encoding the peptide containing the 58 (Ser) - 94 (Phe) amino acid sequence in the amino acid sequence represented by SEQ ID NO:33, a DNA containing the 172-282 base sequence represented by SEQ ID NO:34;

as the DNA encoding the peptide containing the 83 (Val) - 94 (Phe) amino acid sequence in the amino acid sequence represented by SEQ ID NO:33, a DNA containing the 247-282 base sequence represented by SEQ ID NO:34;

as the DNA encoding the peptide containing the 84 (Pro) - 94 (Phe) amino acid sequence in the amino acid sequence represented by SEQ ID NO:33, a DNA containing the 250-282 base sequence represented by SEQ ID NO:34;

as the DNA encoding the peptide containing the 118 (Phe) - 125 (Phe) amino acid sequence in the amino acid sequence represented by SEQ ID NO:33, a DNA containing the 352-375 base sequence represented by SEQ ID NO:34;

as the DNA encoding the peptide containing the 58 (Ser) - 94 (Phe) amino acid sequence in the amino acid sequence represented by SEQ ID NO:50, a DNA containing the 172-282 base sequence represented by SEQ ID NO:51;

as the DNA encoding the peptide containing the 84 (Pro) - 94 (Phe) amino acid sequence in the amino acid sequence represented by SEQ ID NO:50, a DNA containing the 250-282 base sequence represented by SEQ ID NO:51; and the like.

The polypeptide of the present invention or its partial peptide and the receptor protein of the present invention or its partial peptide, which will be described hereinafter, as well as the DNAs encoding these proteins or peptides may be labeled in a publicly known manner. Specific examples include those labeled with an isotope, those labeled with fluorescence (labeling with, e.g., fluorescein, etc.), those biotinated, those labeled with enzymes, and the like.

As a means for cloning the DNA that completely encodes the polypeptide of the present invention or its partial peptide (hereinafter sometimes collectively referred to as the polypeptide of the present invention in the following description of cloning and expression of the DNAs encoding these polypeptides or the like), the DNA may be either amplified by publicly known PCR using synthetic DNA primers containing a part of the base sequence of the polypeptide of the present invention, or the DNA inserted into an appropriate vector can be selected by hybridization with a labeled DNA fragment or synthetic DNA that encodes a part or entire region of the polypeptide of the present invention. The hybridization can be carried out, for example, according to the method described in Molecular Cloning, 2nd (J. Sambrook et al., Cold Spring Harbor Lab. Press, 1989). The hybridization may also be performed using commercially available library in accordance with the protocol described in the attached instructions.

Conversion of the base sequence of DNA can be effected by publicly known methods such as the Gapped duplex method or the Kunkel method or its modification by using a publicly known kit available as Mutan™-super Express Km (manufactured by Takara Shuzo Co., Ltd.), Mutan™-K (manufactured by Takara Shuzo Co., Ltd.), or the like.

The cloned DNA encoding the polypeptide can be used as it is, depending upon purpose or, if desired, after digestion with a restriction enzyme or after addition of a linker thereto. The DNA may contain ATG as a translation initiation codon at the 5' end thereof and TAA, TGA or TAG as a translation termination codon at the 3' end thereof. These translation initiation and termination codons may also be added by using an appropriate synthetic DNA adapter.

The expression vector of the polypeptide of the present invention can be manufactured, for example, by (a) excising the desired DNA fragment from the DNA encoding the polypeptide of the present invention, (b) and then ligating the DNA fragment with an appropriate expression vector downstream a promoter in the vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as λ phage, etc., animal viruses such as retrovirus, vaccinia virus, baculovirus, etc. as well as pA1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRα promoter, SV40 promoter, HIV·LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, λPL promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, and the like.

In addition to the foregoing examples, the expression vector may further optionally contain an enhancer, a splicing signal, a poly A addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as $Amp^r$), neomycin resistant gene (hereinafter sometimes abbreviated as Neo, G418 resistance), etc. In particular, when dhfr gene is used as the selection marker together with dhfr gene, selection can also be made on thymidine free media.

If necessary, a signal sequence that matches with a host is added to the N-terminus of the polypeptide of the present invention. Examples of the signal sequence that can be used are Pho A signal sequence, OmpA signal sequence, etc. in the case of using bacteria of the genus Escherichia as the host; α-amylase signal sequence, subtilisin signal sequence, etc. in the case of using bacteria of the genus Bacillus as the host; MFα signal sequence, SUC2 signal sequence, etc. in the case of using yeast as the host; and insulin signal sequence, α-interferon signal sequence, antibody molecule signal sequence, etc. in the case of using animal cells as the host, respectively.

Using the vector containing the DNA encoding the polypeptide of the present invention thus constructed, transformants can be manufactured.

Examples of the host, which may be employed, are bacteria belonging to the genus Escherichia, bacteria belonging to the genus Bacillus, yeast, insect cells, insects and animal cells, etc.

Specific examples of the bacteria belonging to the genus Escherichia include Escherichia coli K12 DH1 [Proc. Natl. Acad. Sci. U.S.A., 60, 160 (1968)], JM103 [Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cerevisiae AH22, $AH22R^-$, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. is used. Examples of the Sf cells which can be used are Sf9 cells (ATCC CRL1711) and Sf21 cells (both cells are described in Vaughn, J. L. et al., In Vivo, 13, 213-217 (1977).

As the insect, for example, a larva of Bombyx mori can be used [Maeda et al., Nature, 315, 592 (1985)].

Examples of animal cells include monkey cells COS-7, Vero, Chinese hamster cells CHO (hereinafter referred to as CHO cells), dhfr gene deficient Chinese hamster cells CHO (hereinafter simply referred to as CHO (dhfr) cells), mouse L cells, mouse AtT-20, mouse myeloma cells, rat GH 3, human FL cells, etc.

Bacteria belonging to the genus Escherichia can be transformed, for example, by the method described in Proc. Natl. Acad. Sci. U.S.A., 69, 2110 (1972), Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc..

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the polypeptide can be obtained.

Where the host is bacteria belonging to the genus Escherichia or the genus Bacillus, the transformant can be appropriately cultured in a liquid medium which contains materials required for growth of the transformant such as carbon sources, nitrogen sources, inorganic materials, etc. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast, vitamins, growth promoting factors etc. may also be added to the medium. Preferably, pH of the medium is adjusted to about 5 to about 8.

A preferred example of the medium for culturing the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15 to about 43°C for about 3 to about 24 hours. If necessary, the culture may be aerated or agitated.

Where the bacteria belonging to the genus Bacillus are used as the host, the transformant is cultivated generally at about 30 to about 40°C for about 6 to about 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20 to about 35°C for about 24 to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary, the culture can be aerated or agitated.

Where animal cells are employed as the host, the transformant is cultivated in, for example, MEM medium containing about 5% to about 20% fetal bovine serum [Science, 122, 501 (1952)), DMEM medium (Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30 to about 40°C for about 15 to about 60 hours and, if necessary, the culture can be aerated or agitated.

As described above, the polypeptide of the present invention can be produced in the cell membrane of the transformant, etc.

The polypeptide of the present invention can be separated and purified from the culture described above by the following procedures.

When the polypeptide of the present invention is extracted from the culture or cells, after cultivation the transformant or cell is collected by a publicly known method and suspended in a appropriate buffer. The transformant or cell is then disrupted by publicly known methods such as ultrasonication, a treatment with lysozyme and/or freeze-thaw cycling, followed by centrifugation, filtration, etc. Thus, the crude extract of the polypeptide or its partial peptide of the present invention can be obtained. The buffer used for the procedures may contain a protein modifier such as urea or guanidine hydrochloride, or a surfactant such as Triton X-100™, etc. When the polypeptide or its partial peptide of the present invention is secreted in the culture broth, after completion of the cultivation the supernatant can be separated from the transformant or cell to collect the supernatant by a publicly known method.

The polypeptide of the present invention contained in the supernatant or the extract thus obtained can be purified by appropriately combining the publicly known methods for separation and purification. Such publicly known methods for separation and purification include a method utilizing difference in solubility such as salting out, solvent precipitation, etc.; a method mainly utilizing difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reversed phase high performance liquid chromatography, etc.; a method utilizing difference in isoelectric point such as isoelectrofocusing electrophoresis; and the like.

When the polypeptide thus obtained is in a free form, it can be converted into the salt by publicly known methods or modifications thereof. On the other hand, when the polypeptide is obtained in the form of a salt, it can be converted into the free form or in the form of a different salt by publicly known methods or modifications thereof.

The polypeptide produced by the recombinant can be treated, prior to or after the purification, with an appropriate protein-modifying enzyme so that the protein or partial peptide can be appropriately modified to partially remove a polypeptide. Examples of the protein-modifying enzyme include trypsin, chymotrypsin, arginyl endopeptidase, protein kinase, glycosidase and the like.

The presence or activity of the thus produced polypeptide of the present invention or salts thereof can be determined by a binding test to a labeled ligand and by an enzyme immunoassay using a specific antibody.

Specific examples of the receptor for the polypeptide of the present invention, its amides or esters or salts thereof, or the partial peptide or its esters, amides or salts thereof (hereinafter sometimes collectively referred to as the receptor protein of the present invention) include receptor proteins containing the same or substantially the same amino acid sequence as the amino acid sequence shown by, e.g., SEQ ID NO:37, and the like.

The receptor protein of the present invention may be any polypeptide derived from any cells of mammals (e. g., human, guinea pig, rat, mouse, rabbit, swine, sheep, bovine, monkey, etc.) (such as splenocytes, nerve cells, glial cells, β cells of pancreas, bone marrow cells, mesangial cells, Langerhans' cells, epidermic cells, epithelial cells, endothelial cells, fibroblasts, fibrocytes, myocytes, fat cells, immune cells (e.g., macrophage, T cells, B cells, natural killer cells, mast cells, neutrophils, basophils, eosinophils, monocytes), megakaryocytes, synovial cells, chondrocytes, bone cells, osteoblasts, osteoclasts, mammary gland cells, hepatocytes or interstitial cells, etc.; the corresponding precursor cells, stem cells, cancer cells, etc.) or hemocyte type cells; or any tissues where such cells are present, such as brain or any of brain regions (e.g., olfactory bulb, amygdaloid nucleus, basal ganglia, hippocampus, thalamus, hypothalamus, subthalamic nucleus, cerebral cortex, medulla oblongata, cerebellum, occipital pole, frontal lobe, temporal lobe, putamen, caudate nucleus, corpus callosum, substantia nigra), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, peripheral blood cells, prostate, testis, ovary, placenta, uterus, bone, joint, skeletal muscle, etc. (especially brain or any of brain regions). The receptor protein may also be a synthetic protein.

The amino acid sequence which has substantially the same amino acid sequence as the amino acid sequence represented by SEQ ID NO:37 includes an amino acid sequence having at least about 50% homology, preferably at least about 70% homology, more preferably at least about 80% homology, much more preferably at least about 90% homology and most preferably at least about 95% homology, to the amino acid sequence represented by SEQ ID NO:37.

The protein containing substantially the same amino acid sequence as that shown by SEQ ID NO:37 is preferably a protein having substantially the same amino acid sequence shown by SEQ ID NO:37 and having the activities substantially equivalent to the activity of the amino acid sequence shown by SEQ ID NO:37. Specific examples include a protein containing the amino acid sequence represented by SEQ ID NO:54, etc.

The substantially equivalent activities include, for example, a ligand binding activity, a signal transduction activity, etc. The term substantially equivalent is used to mean that the nature of the activities is equivalent. It is thus preferred that the activities such as a ligand binding activity, a signal transduction activity, etc. are equivalent in strength (e.g., about 0.1 to about 100 times, preferably about 0.5 to about 20 times, more preferably about 0.5 to about 2 times), and it is allowable that differences among grades such as the strength of these activities and molecular weight of the polypeptide are even present.

The activities such as a ligand binding activity, a signal transduction activity, a somatostatin secretion regulating activity or the like can be assayed by a modification of publicly known methods, for example, by means of ligand determination or screening, which will be later described.

As the receptor protein of the present invention, there can be employed proteins containing (i) an amino acid sequence represented by SEQ ID NO:37 or SEQ ID NO:54, of which at least 1 or 2 (preferably 1 to 30, more preferably 1 to 10 and most preferably several (1 or 2)) amino acids are deleted; (ii) an amino acid sequence represented by SEQ ID NO:37 or SEQ ID NO:54, to which at least 1 or 2 (preferably 1 to 30, more preferably 1 to 10 and most preferably several (1 or 2)) amino acids are added; (iii) an amino acid sequence represented by SEQ ID NO:37 or SEQ ID NO: 54, in which at least 1 or 2 (preferably 1 to 30, more preferably 1 to 10 and most preferably several (1 or 2)) amino acids are substituted by other amino acids; and (iv) a combination of the above amino acid sequences.

The receptor proteins in the present specification are represented in accordance with the conventional way of describing peptides, that is, the N-terminus (amino terminus) at the left hand and the C-terminus (carboxyl terminus) at the right hand. In the receptor proteins of the present invention including the receptor proteins containing the amino acid sequence shown by SEQ ID NO:37, the C-terminus is usually in the form of a carboxyl group (-COOH) or a carboxylate ($-COO^-$) but may be in the form of an amide ($-CONH_2$) or an ester (-COOR).

Examples of the ester group shown by R include a $C_{1-6}$ alkyl group such as methyl, ethyl, n-propyl, isopropyl, n-butyl, etc.; a $C_{3-8}$ cycloalkyl group such as cyclopentyl, cyclohexyl, etc.; a $C_{6-12}$ aryl group such as phenyl, α-naphthyl, etc.; an aralkyl having 7 to 14 carbon atoms such as a phenyl-$C_{1-2}$ alkyl group, e.g., benzyl, phenethyl, etc.; an α-naphthyl-$C_{1-2}$ alkyl group such as α-naphthylmethyl, etc.; and the like. In addition, pivaloyloxymethyl or the like which is used widely as an ester for oral administration may also be used.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the above C-terminal.

Furthermore, examples of the receptor protein of the present invention include variants of the above receptor protein, wherein the amino group at the N-terminal methionine residue in the polypeptide described above is protected with a protecting group (e.g., a $C_{1-6}$ acyl group such as a $C_{1-6}$ alkanoyl group, e.g., formyl group, acetyl group, etc.); those wherein the N-terminal region is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a $C_{1-6}$ acyl group such as a $C_{2-6}$ alkanoyl group, e.g., formyl group, acetyl group, etc.), conjugated proteins such as glycoproteins having sugar chains, and the like.

Specific examples of the receptor protein of the present invention include a rat-derived receptor protein containing the amino acid sequence represented by SEQ ID NO:37, a human-derived receptor protein containing the amino acid sequence represented by SEQ ID NO:54, etc.

As the partial peptide of the receptor protein of the present invention, any partial peptide described for the receptor protein can be used. For example, a part of the receptor protein molecule of the present invention, which is exposed to the outside of a cell membrane or the like, can be used, so long as it has a receptor binding activity.

Specifically, the partial peptide of the receptor protein having the amino acid sequence represented by SEQ ID NO:37 or SEQ ID NO:54 is a peptide containing the parts, which have been analyzed to be extracellular domains (hydrophilic domains) in the hydrophobic plotting analysis. A peptide containing a hydrophobic domain part can be used as well. In addition, the peptide may contain each domain separately or a plurality of domains together.

In the receptor protein of the present invention, the partial peptide is a peptide having at least 20, preferably at least 50 and more preferably at least 100 amino acids, in the amino acid sequence, which constitutes the receptor protein of the present invention.

The substantially the same amino acid sequence includes an amino acid sequence having at least about 50% homology, preferably at least about 70% homology, more preferably at least about 80% homology, much more preferably at least about 90% homology and most preferably at least about 95% homology, to the amino acid sequence represented.

Herein the term "substantially equivalent activities" refers to the same significance as defined hereinabove. The "substantially equivalent activities" can be assayed by the same method as described above.

In the partial peptide of the receptor protein of the present invention, at least 1 or 2 (preferably 1 to 10, more preferably several (1 or 2)) amino acids may be deleted; at least 1 or 2 (preferably 1 to 20, more preferably 1 to 10 and most preferably several (1 or 2)) amino acids may be added; or at least 1 or 2 (preferably 1 to 10, more preferably several (1 or 2)), amino acids may be substituted by other amino acids.

In the partial peptide in the receptor protein of the present invention, the C-terminus is usually in the form of a carboxyl group (-COOH) or a carboxylate ($-COO^-$) but may be in the form of an amide ($-CONH_2$) or an ester (-COOR) (wherein R has the same significance as described above), as in the polypeptide of the present invention described above.

Where the receptor protein of the present invention contains a carboxyl group (or a carboxylate) at a position other than the C-terminus, it may be amidated or esterified and such an amide or ester is also included within the receptor protein of the present invention. The ester group may be the same group as that described with respect to the above C-terminal.

Furthermore, examples of the partial peptide of the receptor protein in the present invention include peptides wherein the amino group at the N-terminal methionine residue is protected with a protecting group, those wherein the N-terminal region is cleaved in vivo and the Gln formed is pyroglutaminated, those wherein a substituent on the side chain of an amino acid in the molecule is protected with a suitable protecting group, or conjugated proteins such as glycoproteins having sugar chains, etc., as in the receptor protein of the present invention described above.

As the salts of the receptor protein or its partial peptide in the present invention, physiologically acceptable acid addition salts are particularly preferred. Examples of such salts are salts with inorganic acids (e.g., hydrochloric acid, phosphoric acid, hydrobromic acid, sulfuric acid), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid), and the like.

The receptor protein of the present invention or salts thereof may be manufactured by a publicly known method used to purify receptor proteins from human or other mammalian cells or tissues described above, or by preparing a transformant containing the DNA encoding the receptor protein of the present invention (a host similar to the host of the transformant containing the DNA encoding the polypeptide of the present invention described above may be used) in a manner similar to the aforesaid method for preparing the transformant containing the DNA encoding the polypeptide of the present invention, culturing the resulting transformant in a manner similar to the aforesaid method for preparing the transformant containing the DNA encoding the polypeptide of the present invention. Furthermore, the receptor protein or salts of the present invention may also be manufactured by the aforesaid methods for synthesizing polypeptides or by modified methods.

Where the receptor protein or salts thereof are manufactured from human or mammalian tissues or cells, human or mammalian tissues or cells are homogenized, then extracted with an acid or the like, and the extract is isolated and purified by a combination of chromatography techniques such as reversed phase chromatography, ion exchange chromatography, and the like.

The partial peptide of the receptor protein of the present invention or salts thereof can be manufactured by a publicly known method used to synthesize a peptide or, by cleaving the receptor protein of the present invention with an appropriate peptidase.

The receptor protein of the present invention or salts thereof, its partial peptide, amides or esters, or salts thereof can be synthesized by the aforesaid method for synthesizing the polypeptide of the present invention, its amides or esters, or salts thereof.

For the polynucleotide encoding the receptor protein of the present invention, any polynucleotide can be used as long as it contains the base sequence (DNA or RNA, preferably DNA) encoding the receptor protein of the present invention. Such a polynucleotide may be DNA and RNA including mRNA encoding the receptor protein of the present invention. The polynucleotide of the present invention may be double-stranded or single-stranded. Where the polynucleotide is double-stranded, it may be double-stranded DNA, double-stranded RNA or DNA:RNA hybrid. Where the polynucleotide is single-stranded, it may be a sense strand (i.e., a coding strand) or an antisense strand (i.e., a non-coding strand).

Using the polynucleotide encoding the receptor protein of the present invention, mRNA of the. receptor protein of the present invention can be quantified by, for example, the publicly known method published in separate volume *of Jikken Igaku* 15 (7) "New PCR and its application" (1997) or the modified method.

The DNA encoding the receptor protein of the present invention may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using the total RNA or mRNA fraction prepared from the cells and tissues described above.

Specifically, the DNA encoding the receptor protein of the present invention may be any DNA having the base sequence shown by SEQ ID NO:38, SEQ ID NO:55 or SEQ ID NO:56, or the base sequence hybridizable to the base sequence represented by SEQ ID NO:38, SEQ ID NO:55 or SEQ ID NO:56 under high stringent conditions and encoding a receptor protein which has the activities substantially equivalent to those of the receptor protein of the present invention (e.g., a ligand binding activity, a signal transduction activity, etc.).

Specific examples of the DNA that is hybridizable to the base sequence represented by SEQ ID NO:38, SEQ ID NO:55 or SEQ ID NO:56 include DNA having at least about 70% homology, preferably at least about 80% homology, more preferably at least about 90% homology and most preferably at least about 95% homology, to the base sequence represented by SEQ ID NO:38, SEQ ID NO:55 or SEQ ID NO:56.

The hybridization can be carried out by publicly known methods or by a modification thereof, for example, according to the method described in Molecular Cloning, 2nd Ed., J. Sambrook et al., Cold Spring Harbor Lab. Press, (1989). A commercially available library may also be used according to the instructions of the attached manufacturer's protocol. The hybridization can be carried out preferably under high stringent conditions.

The high stringent conditions used herein refer to the conditions, for example, in a sodium concentration of about 19 to about 40 mM, preferably about 19 to about 20 mM at a temperature of about 50 to about 70°C, preferably about 60 to about 65°C. In particular, hybridization conditions in a sodium concentration of about 19 mM at a temperature of about 65°C are most preferred.

The polypeptide encoded by the DNA, which is hybridizable to the base sequence shown by SEQ ID NO:38, SEQ ID NO:55 or SEQ ID NO:56 can be manufactured by methods similar to those for manufacturing the polypeptide of the present invention, described above. Examples of the amides, esters or salts of the polypeptide are the same as those for the amides, esters or salts of the polypeptide of the present invention described above.

More specifically, for the DNA encoding the receptor protein having the amino acid sequence represented by SEQ ID NO:37, DNA having the base sequence represented by SEQ ID NO:38 may be employed; and DNA having the base sequence represented by SEQ ID NO:55 or SEQ ID NO:56 may be used for the DNA encoding the receptor protein having the amino acid sequence represented by SEQ ID NO:54.

The polynucleotide containing a part of the base sequence of DNA encoding the receptor protein of the present invention or a part of the base sequence complementary to the DNA is used to mean that not only the DNA encoding the partial peptide of the present invention described below but also RNA are included.

Any DNA can be used as the DNA encoding the partial peptide of the receptor protein of the present invention so long as the DNA contains the base sequence encoding the partial peptide of the present invention described above. The DNA may be any of genomic DNA, genomic DNA library, cDNA derived from the cells and tissues described above, cDNA library derived from the cells and tissues described above and synthetic DNA. The vector to be used for the library may be any of bacteriophage, plasmid, cosmid and phagemid. The DNA may also be directly amplified by reverse transcriptase polymerase chain reaction (hereinafter abbreviated as RT-PCR) using mRNA fraction prepared from the cells and tissues described above.

Specifically, as the DNA encoding the partial peptide of the present invention, there are employed, for example, (1) a DNA having a part of the base sequence of a DNA containing the base sequence shown by SEQ ID NO:38, SEQ ID NO:55 or SEQ ID NO:56, (2) a DNA containing the base sequence hybridizable to the base sequence represented by SEQ ID NO:38, SEQ ID NO:55 or SEQ ID NO:56 under high stringent conditions and containing a part of the base sequence of the DNA encoding a receptor protein which has the activities substantially equivalent to those of the receptor protein of the present invention (e.g., a ligand binding activity, a signal transduction activity, etc.).

(1) The polypeptide of the present invention, its amides or esters, or salts thereof, or the partial peptide of the present invention, its amides or esters, or salts thereof (hereinafter sometimes collectively referred to as the polypeptide of the present invention) has the activities of regulating the secretion of prolactin, namely, prolactin secretion-stimulating and -inhibiting activities. That is, first, the polypeptide of the present invention has prolactin secretion-stimulating activity and thus finds application as a prophylactic and therapeutic drug for various diseases associated with prolactin hyposecretion. On the other hand, the polypeptide of the present invention has a high affinity to the receptor proteins and, therefore, when used in an increased dose, causes desensitization for prolactin secretion so that the polypeptide also exhibits the prolactin secretion-inhibiting activity. In this sense, the polypeptide can be used as a prophylactic and therapeutic drug for various diseases associated with prolactin hypersecretion.

Thus, the polypeptide of the present invention as the prolactin secretion stimulant is useful as prophylactic and therapeutic drugs for various diseases associated with prolactin secretion, such as hypoovarianism, spermatic underdevelopment, osteoporosis, menopausal symptoms, agalactosis, hypothyroidism, renal insufficiency, etc.

In addition, the polypeptide of the present invention has an effect of arousing sexual desire (pheromone-like activity) based on the prolactin secretion stimulating activity, and is useful also as a sexual desire-stimulating agent.

Moreover, the polypeptide of the present invention as the prolactin secretion inhibitor is useful as prophylactic and therapeutic drugs for various diseases associated with prolactin secretion, such as hyperprolactinemia, pituitary tumor, diencephalon tumor, menstrual disorder, stress, autoimmune diseases, prolactinoma, sterility, impotence, amenorrhea, lactorrhea, acromegaly, Chiari-Frommel syndrome, Argonz-del Castilo syndrome, Forbes-Albright syndrome, breast cancer lymphoma or Sheehan's syndrome, or spermatogenesis disorder, etc.

Furthermore, the polypeptide of the present invention is also useful as an anticonceptive, based on the prolactin secretion inhibiting activity.

In addition, the polypeptide of the present invention is useful not only as a testing agent to examine the function of prolactin secretion but also as a veterinary drug such as a lactation stimulant for livestock mammals including bovine, goat, swine, etc. Furthermore, an application of the polypeptide to the production of useful substances is expected, which involves producing a useful substance in the body of livestock mammals and secreting the substance into milk, or the like.

Furthermore, the polypeptide of the present invention has the activity of regulating placenta functions and is thus useful as a prophylactic or therapeutic agent for ciliary tumor, hydatid mole, invasive mole, miscarriage, fetal underdevelopment, glucose metabolism abnormality, lipid metabolism abnormality or labor induction.

The prolactin secretion regulating activities of the polypeptide of the present invention can be attained in accordance with the method described in Neuroendocrinology, 62, 1995, 198-206 or Neuroscience Letters, 203, 1996, 164-170, or its modifications. It is desired to perform the method described in EXAMPLES hereinafter.

In the case that the polypeptide of the present invention is used as the aforesaid pharmaceuticals or veterinary drugs, it may be implemented by a conventional means. The polypeptide may be used orally, for example, in the form of tablets, if necessary, sugarcoated, capsules, elixirs, microcapsules etc., or parenterally in the form of injectable preparations such as a sterile solution or a suspension in water or with other pharmaceutically acceptable liquid. These preparations can be manufactured by mixing the polypeptide or its salts with a physiologically acceptable known carrier, a flavoring agent, an excipient, a vehicle, an antiseptic agent, a stabilizer, a binder, etc., in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80(™) and HCO-50), etc. Examples of the oily medium include sesame oil and soybean oil, which may also be used in combination with a dissolution aid such as benzyl benzoate and benzyl alcohol. The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule aseptically.

Since the thus obtained pharmaceutical preparation is safe and low toxic, the preparation can be administered to mammal (e.g., human, mouse, rat, guinea pig, rabbit, sheep, swine, bovine, cat, dog, monkey, hamadryad, chimpanzee, etc.).

The dose of the polypeptide of the present invention varies depending on conditions, etc.; in oral administration, generally to the adult patient with hypothyroidism (as 60 kg body weight), the dose is normally about 0.1 to about 100 mg, preferably about 1.0 to about 50 mg, and more preferably about 1.0 to about 20 mg per day. In parenteral administration, the single dose varies depending on subject to be administered, conditions, route for administration, etc. but it is advantageous to administer the active ingredient intravenously to the adult patient with hypothyroidism (as 60 kg body weight) at a daily dose of about 0.01 to about 30 mg, preferably about 0.1 to about 20 mg, and more preferably about 0.1 to about 10 mg. For other animal species, the corresponding dose as converted per 60 kg body weight can be administered.

(2) Also, the compound or its salts, which are obtainable using the method of screening the compound or its salts that promote or inhibit the activity of the polypeptide of the present invention characterized by using the polypeptide of the present invention, or using the kit for screening the compound or its salts that promote or inhibit the activity of the polypeptide of the present invention, comprising the polypeptide of the present invention; or, the compound or its salts, which are obtainable using the method of screening the compound or its salts that promote or inhibit the activity of the polypeptide of the present invention characterized by using the polypeptide of the present invention and the receptor protein of the present invention or its partial peptide, its amides or esters or salts thereof (hereinafter sometimes merely referred to as the receptor protein of the present invention), or using the kit for screening the compound or its salts that promote or inhibit the activity of the polypeptide of the present invention, comprising the receptor protein of the present invention; may also be used as prophylactic and therapeutic agents for various diseases associated with prolactin hyposecretion when they have the prolactin secretion stimulating activity, and when they have the prolactin secretion inhibiting activity, as prophylactic and therapeutic agents for various diseases associated with prolactin hypersecretion.

When the compound or its salts thus obtained have the prolactin secretion stimulating activity, they can be used as prophylactic and therapeutic agents for various diseases associated with prolactin hyposecretion;

the compound or its salts as the prolactin secretion stimulants are useful as prophylactic and therapeutic drugs for various diseases associated with prolactin secretion, such as hypoovarianism, spermatic underdevelopment, osteoporosis, menopausal symptoms, agalactosis, hypothyroidism, renal insufficiency, etc.

In addition, the compound or its salts have the effect of arousing sexual desire (pheromone-like activity) based on the prolactin secretion stimulating activity, and is useful as a sexual desire-stimulating agent.

On the other hand, when the compound or its salts thus obtained have the prolactin secretion inhibiting activity, they can be used as prophylactic and therapeutic agents for various diseases associated with prolactin hypersecretion; the compound or its salts as the prolactin secretion inhibitors are useful as prophylactic and therapeutic drugs for various diseases associated with prolactin secretion, such as hyperprolactinemia, pituitary tumor, diencephalon tumor, menstrual disorder, stress, autoimmune diseases, prolactinoma, sterility, impotence, amenorrhea, lactorrhea, acromegaly, Chiari-Frommel syndrome, Argonz-del Castilo syndrome, Forbes-Albright syndrome, breast cancer lymphoma or Sheehan's syndrome, or spermatogenesis disorder, etc.

Furthermore, the compound or its salts thus obtained are useful as anticonceptives, based on the prolactin secretion inhibiting activity.

In addition, the compound or its salts thus obtained are useful not only as testing agents to examine the function of prolactin secretion but also as a veterinary drug such as lactation stimulants for livestock mammals including bovine, goat, swine, etc. Furthermore, an application to the production of useful substances is expected, which involves producing a useful substance in the body of livestock mammals and secreting the substance into milk, or the like.

Furthermore, the compound or its salts thus obtained have the activity of regulating the functions of placenta and are thus useful as prophylactic or therapeutic agents for ciliary tumor, hydatid mole, invasive mole, miscarriage, fetal underdevelopment, glucose metabolism abnormality, lipid metabolism abnormality or labor induction.

The prolactin secretion regulating activities of the compound or its salts obtained can be attained in accordance with the method described in Neuroendocrinology, 62, 1995, 198-206 or Neuroscience Letters, 203, 1996, 164-170, or its modifications. It is desired to perform the method described in EXAMPLES hereinafter.

In the case that the compound or its salts obtained is used as the aforesaid pharmaceuticals or veterinary drugs, it may be implemented by a conventional means. The compound or its salts may be used orally, for example, in the form of tablets, if necessary, sugarcoated, capsules, elixirs, microcapsules etc., or parenterally in the form of injectable preparations such as a sterile solution or a suspension in water or with other pharmaceutically acceptable liquid. These preparations can be manufactured by mixing the compound or its salts with a physiologically acceptable known carrier, a flavoring agent, an excipient, a vehicle, an antiseptic agent, a stabilizer, a binder, etc., in a unit dosage form required in a generally accepted manner that is applied to making pharmaceutical preparations. The active ingredient in the preparation is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, and a flavoring agent such as peppermint, akamono oil and cherry. When the unit dosage is in the form of capsules, liquid carriers such as oils and fats may further be used together with the additives described above. A sterile composition for injection may be formulated according to a conventional manner used to make pharmaceutical compositions, e.g., by dissolving or suspending the active ingredients in a vehicle such as water for injection with a naturally occurring vegetable oil such as sesame oil, coconut oil, etc. to prepare the pharmaceutical composition.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol), a polyalcohol (e.g., propylene glycol and polyethylene glycol), a nonionic surfactant (e.g., polysorbate 80(TM) and HCO-50), etc. Examples of the oily medium include sesame oil and soybean oil, which may also be used in combination with a dissolution aid such as benzyl benzoate and benzyl alcohol. The prophylactic/therapeutic agent described above may further be formulated with a buffer (e.g., phosphate buffer, sodium acetate buffer), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule aseptically.

Since the thus obtained pharmaceutical preparation is safe and low toxic, the preparation can be administered to mammal (e.g., human, mouse, rat, guinea pig, rabbit, sheep, swine, bovine, cat, dog, monkey, hamadryad, chimpanzee, etc.).

The dose of the compound or its salts obtained varies depending on conditions, etc.; in oral administration, generally to the adult patient with hypothyroidism (as 60 kg body weight), the dose is normally about 0.1 to about 100 mg, preferably about 1.0 to about 50 mg, and more preferably about 1.0 to about 20 mg per day. In parenteral administration, the single dose varies depending on subject to be administered, conditions, route for administration, etc. but it is advantageous to administer the active ingredient intravenously to the adult patient with hypothyroidism (as 60 kg body weight) at a daily dose of about 0.01 to about 30 mg, preferably about 0.1 to about 20 mg, and more preferably about 0.1 to about 10 mg. For other animal species, the corresponding dose as converted per 60 kg body weight can be administered.

(3) The method and the kit for screening the compound or its salt that promotes or inhibits the activity of the polypeptide of the present invention will be described below in detail.

The method for screening the compound or its salts that promote or inhibit the activities of the polypeptide of the present invention which comprises using the polypeptide of the present invention, is a method for screening the compound or its salts that promote or inhibit the activities of the polypeptide of the present invention which comprises using, preferably, the polypeptide of the present invention and the receptor protein of the present invention or its partial peptide, its amides or esters, or salts thereof, the partial peptide, its amides or esters, or salts thereof (hereinafter sometimes merely referred to as the receptor protein of the present invention).

The screening method is carried out specifically by measuring the activities of the polypeptide of the present invention (i) when the receptor protein of the present invention is brought in contact with the polypeptide of the present invention and (ii) when the receptor protein of the present invention and a test compound are brought in contact with the polypeptide of the present invention, and comparing them.

Specifically, the screening method described above is characterized by measuring the cell stimulating activities of the polypeptide of the present invention and a test compound or the binding amount of the polypeptide of the present invention and a test compound to the receptor protein of the present invention, in the cases (i) and (ii), and comparing these activities. The cell stimulating activity, etc. of the polypeptide in the present invention can be measured in accordance with publicly known methods, for example, Dockray, G.J., et al., Nature, 305, 328-330, 1983, Fukusumi, S., et al., Biochem. Biophys. Res. Commun., 232, 157-163, 1997, Hinuma, S., et al., Nature, 393, 272-276, 1998, Tatemoto, K., et al., Biochem. Biophys. Res. Commun., 251, 471-476, 1998, etc., or modifications thereof.

The binding amounts of the polypeptide of the present invention and a test compound to the receptor protein of the present invention or the cell stimulating activities can be measured by the methods described below, or a modification thereof.

Examples of such a test compound are a peptide, a protein, a non-peptide compound, a synthetic compound, a fermentation product, a cell extract, a plant extract, an animal tissue extract and the like. These compounds may be novel compounds or publicly known compounds.

To perform the screening method described above, the polypeptide of the present invention is suspended in a buffer suitable for screening to prepare a specimen of the polypeptide of the present invention. Any buffer having pH of approximately 4 to 10 (desirably a pH of approximately 6 to 8) such as a phosphate buffer, Tris-hydrochloride buffer, etc. may be used, so far as it does not interfere the reaction between the polypeptide of the present invention and the receptor protein of the present invention.

For example, when a test compound increases the cell stimulating activity, etc. in (ii) described above by at least about 20%, preferably at least 30%, more preferably at least about 50%, as compared to the case of (i) above, the test compound can be selected to be a compound that promotes the cell stimulating activity, etc. of the polypeptide of the present invention. On the other hand, a test compound can be selected to be a compound that inhibits the cell stimulating activity, etc. of the polypeptide of the present invention, when the test compound inhibits the cell stimulating activity, etc. in (ii) described above by at least about 20%, preferably at least 30%, more preferably at least about 50%, as compared to the case of (i) above.

It is desirable, before conducting these tests, to examine the binding ability of a test compound to the receptor protein of the present invention to see if the test compound is capable of binding to the receptor protein of the present invention, which is effected by the methods (1) to (3) later described for the "determination of the polypeptide of the present invention and a test compound to the receptor protein of the present invention".

As an indicator that the test compound described above is judged to be such a compound or its salts that promote or inhibit the activities of the polypeptide of the present invention, there are binding amounts of the polypeptide of the present invention and a test compound to the receptor protein of the present invention, and the activity that inhibits the binding between the polypeptide of the present invention and the labeled compound. According to the binding test system described in, e.g., Hosoya, M. et al., Biochem. Biophys. Res. Commun., 194 (1), 133-134, 1993, a test compound that inhibits the binding of the labeled compound by 10% or more in a concentration of 1 x $10^{-2}$ M or less is highly likely to be the compound or its salts that promote or inhibit the activities of the polypeptide of the present invention. However, since the binding inhibition activity is a relative value based on the binding of the labeled compound, the activity is not essential for judging the test compound to be a compound or its salts that promote or inhibit the activities of the polypeptide of the present invention.

The screening kit according to the present invention comprises the polypeptide of the present invention. Preferably, the screening kit of the present invention further contains the receptor to the polypeptide of the present invention, that is, the receptor protein of the present invention (specifically, the protein containing the same or substantially the same amino acid sequence as that shown by SEQ ID NO:37, or its salts, or its partial peptide, amides or esters, or salts thereof).

Examples of the screening kit according to the present invention include the following:

1. Reagent for screening

(1) Assay and wash buffers

Hanks' Balanced Salt Solution (manufactured by Gibco) supplemented with 0.05% of bovine serum albumin (manufactured by Sigma).

The buffers may be sterilized by filtration through a membrane filter with a 0.45 μm pore size and stored at 4°C, or may be prepared at use.

(2) A receptor preparation

CHO cells in which the receptor protein of the present invention is expressed are subcultured at 5 x $10^5$ cells/well on a 12-well plate followed by culturing at 37°C under a 5% $CO_2$ and 95% air for 2 days.

(3) Labeled ligand

The polypeptide of the present invention is labeled with commercially available [$^3$H], [$^{125}$I], [$^{14}$C], [$^{35}$S], etc. The product in the form of an aqueous solution is stored at 4°C or at -20°C, which will be diluted at use to 1 μM with a buffer for the assay.

(4) Standard ligand solution

The polypeptide of the present invention is dissolved in PBS containing 0.1 % of bovine serum albumin (manufactured by Sigma) to make the volume 1 mM and then stored at -20°C.

2. Method for assay

(1) CHO cells are cultured in a 12-well tissue culture plate to express the receptor protein of the present invention. After washing the CHO cells twice with 1 ml of buffer for the assay, 490 μl of the buffer for assay is added to each well.

(2) After 5 μl of a test compound solution of $10^{-3}$ to $10^{-10}$M is added, 5 μl of a labeled ligand is added to the system followed by culturing at room temperature for an hour. To determine the amount of the non-specific binding, 5 μl of the ligand of $10^{-3}$ M is added to the system, instead of the test compound.

(3) The reaction mixture is removed from the well, which is washed three times with 1 ml each of the buffer for assay. The labeled ligand bound to the cells is dissolved in 0.2N NaOH-1% SDS and mixed with 4 ml of a liquid scintillator A (manufactured by Wako Pure Chemical).

(4) Radioactivity is measured using a liquid scintillation counter (manufactured by Beckman) and percent of the maximum binding (PMB) is calculated in accordance with the following [equation 1]:

[equation 1]

$$PMB = [(B-NSB)/(B_0 - NSB)] \times 100$$

wherein:

PMB: percent of the maximum binding
B: value when a sample is added
NSB: non-specific binding
$B_0$: maximum binding

The compound or its salt, which is obtainable by the screening method or by the screening kit of the present invention, is the compound selected from the test compounds described above, such as peptides, proteins, non-peptide compounds, synthetic compounds, fermentation products, cells extracts, plant extracts, animal tissue extracts, plasma, etc. and the compound that promotes or inhibits the activities (e.g., a cell stimulating activity, etc.) of the polypeptide of the present invention.

As the salts of the compound, there may be employed similar salts to those of the polypeptide of the present invention described above.

(4) Assay of the binding amount or the cell stimulating activity of the polypeptide of the present invention and a test compound to the receptor protein of the present invention

Either by using the receptor protein of the present invention or by constructing an expression system of the receptor protein of recombinant type and making use of the receptor-binding assay system using the expression system, the binding amount or the cell stimulating activity of the compound (e.g., a peptide, protein, non-peptide compound, synthetic compound, fermentation product, etc.) that binds to the receptor protein of the present invention to exhibit the cell stimulating activity (e.g., the activity that promotes or inhibits arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.), can be assayed.

In the assay method above, the characteristic feature comprises contacting the receptor protein of the present invention with a compound to be tested, and measuring, e.g., the binding amount, the cell stimulating activity, etc. of the test compound to the receptor protein of the present invention.

More specifically, the present invention provides the following:

(1) A method for determining a ligand to the receptor protein of the present invention, which contacting a labeled test compound with the receptor protein of the present invention and measuring the amount of the labeled test compound bound to the receptor protein;

(2) A method for determining a ligand to the receptor protein of the present invention, which comprises contacting a labeled test compound with a cell containing the receptor protein of the present invention or with a membrane fraction of the cell and measuring the amount of the labeled test compound bound to the cell or the membrane fraction;

(3) A method for determining a ligand to the receptor protein of the present invention, which comprises culturing a transformant containing the DNA encoding the receptor protein of the present invention, contacting a labeled test compound with the receptor protein expressed on the cell membrane by said culturing, and measuring the amount of the labeled test compound bound to the receptor protein;

(4) A method for determining a ligand to the receptor protein of the present invention, which comprises contacting a test compound with a cell containing the receptor protein of the present invention and measuring the receptor protein-mediated cell stimulating activity (e.g., the activity that promotes or inhibits arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.); and,

(5) A method for determining a ligand to the receptor protein of the present invention, which comprises culturing a transformant containing DNA encoding the receptor protein of the present invention, contacting a labeled test compound with the receptor protein expressed on the cell membrane by said culturing, and measuring the receptor protein-mediated cell stimulating activity (e.g., the activity that promotes or inhibits arachidonic acid release, ace-

tylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.).

In particular, it is preferred to perform the methods (1) to (3) described above, thereby to confirm that a test compound can bind to the receptor protein of the present invention, followed by the methods (4) and (5) described above.

Any protein exemplified to be usable as the receptor protein of the present invention can be used for determining ligands. However, the receptor protein that is abundantly expressed using animal cells is appropriate.

The receptor protein of the present invention can be manufactured by the method for expression described above, preferably by expressing DNA encoding the receptor protein in mammalian or insect cells. DNA fragments encoding the desired portion of the protein include, but are not limited to, complementary DNA. For example, gene fragments or synthetic DNA may also be used. For introducing a DNA fragment encoding the receptor protein of the present invention into host animal cells and efficiently expressing the same, it is preferred to insert the DNA fragment downstream the polyhedrin promoter of nuclear polyhedrosis virus (NPV), which is a baculovirus having insect hosts, an SV40-derived promoter, a retrovirus promoter, a metallothionein promoter, a human heat shock promoter, a cytomegalovirus promoter, an SRα promoter or the like. The amount and quality of the receptor expressed can be determined by a publicly known method. For example, this determination can be made by the method described in the literature [Nambi, P. et al., J. Biol. Chem., Vol. 267, pp. 19555-19559 (1992)].

Accordingly, the subject containing the receptor protein in the method for determining the ligand may be the receptor protein purified by publicly known method, a cell containing the receptor protein or membrane fraction of such a cell.

Where cells containing the receptor protein of the present invention are used in the method of the present invention for determination of ligands, the cells may be fixed using glutaraldehyde, formalin etc. he fixation can be made by a publicly known method.

The cells containing the receptor protein of the present invention are host cells that have expressed the receptor protein of the present invention, which host cells include Escherichia coli, Bacillus subtilis, yeast, insect cells, animal cells and the like.

The cell membrane fraction is a fraction abundant in cell membrane obtained by cell disruption and subsequent fractionation by a publicly known method. Useful cell disruption methods include cell squashing using a Potter-Elvehjem homogenizer, disruption using a Waring blender or Polytron (manufactured by Kinematica Inc.), disruption by ultrasonication, and disruption by cell spraying through thin nozzles under an increased pressure using a French press or the like. Cell membrane fractionation is effected mainly by fractionation using a centrifugal force, such as centrifugation for fractionation and density gradient centrifugation. For example, cell disruption fluid is centrifuged at a low speed (500 rpm to 3,000 rpm) for a short period of time (normally about 1 to about 10 minutes), the resulting supernatant is then centrifuged at a higher speed (15,000 rpm to 30,000 rpm) normally for 30 minutes to 2 hours. The precipitate thus obtained is used as the membrane fraction. The membrane fraction is rich in the receptor protein expressed and membrane components such as cell-derived phospholipids and membrane proteins.

The amount of the receptor protein in the cell containing the receptor protein and in the membrane fraction is preferably $10^3$ to $10^8$ molecules per cell, more preferably $10^5$ to $10^7$ molecules per cell. As the amount of expression increases, the ligand binding activity per unit of membrane fraction (specific activity) increases so that not only the highly sensitive screening system can be constructed but also large quantities of samples can be assayed with the same lot.

To perform the methods (1) through (3) for determination of a ligand to the receptor protein of the present invention, an appropriate receptor fraction and a labeled test compound are required.

The receptor protein fraction is preferably a fraction of naturally occurring receptor protein or a recombinant receptor fraction having an activity equivalent to that of the natural protein. Herein, the equivalent activity is intended to mean a ligand binding activity, a signal transduction activity or the like that is equivalent to that possessed by naturally occurring receptor proteins.

Preferred examples of labeled test compounds include angiotensin, bombesin, canavinoid, cholecystokinin, glutamine, serotonin, melatonin, neuropeptide Y, opioid, purines, vasopressin, oxytocin, PACAP, secretin, glucagon, calcitonin, adrenomedulin, somatostatin, GHRH, CRF, ACTH, GRP, PTH, VIP (vasoactive intestinal and related polypeptide), somatostatin, dopamine, motilin, amylin, bradykinin, CGRP (calcitonin gene-related peptide), leukotrienes, pancreastatin, prostaglandins, thromboxane, adenosine, adrenaline, α and β-chemokines (e.g., IL-8, GROα, GROβ, GROγ, NAP-2, ENA-78, PF4, IP10, GCP-2, MCP-1, HC14, MCP-3, I-309, MIP1α, MIP-1β, RANTES, etc.), endothelin, enterogastrin, histamine, neurotensin, TRH, pancreatic polypeptide, galanin, etc., which are labeled with [$^{3}H$], [$^{125}I$], [$^{14}C$], [$^{35}S$], etc.

Specifically, first, a standard receptor preparation is prepared by suspending a cell containing the receptor protein of the present invention or the membrane fraction thereof in a buffer appropriate for use in the determination method. Any buffer can be used so long as it does not interfere with ligand-receptor binding, such buffers including a phosphate buffer or a Tris-HCl buffer having pH of 4 to 10 (preferably pH of 6 to 8). For the purpose of minimizing non-specific binding, a surfactant such as CHAPS, Tween-80™ (manufactured by Kao-Atlas Inc.), digitonin or deoxycholate, and various proteins such as bovine serum albumin or gelatin, may optionally be added to the buffer. Further for the purpose of suppressing the degradation of the receptor or ligand by a protease, a protease inhibitor such as PMSF, leupeptin, E-64 (manufactured by Peptide Institute, Inc.) and pepstatin may also be added. A given amount (5,000 to 500,000 cpm) of a test compound labeled with [$^3$H], [$^{125}$I], [$^{14}$C], [$^{35}$S] or the like is added to 0.01 ml to 10 ml of the receptor solution. To determine the amount of non-specific binding (NSB), a reaction tube containing an unlabeled test compound in a large excess is also provided. The reaction is carried out at approximately 0°C to 50°C, preferably about 4°C to 37°C for about 20 minutes to about 24 hours, preferably about 30 minutes to 3 hours. After completion of the reaction, the reaction mixture is filtrated through glass fiber filter paper, etc. and washed with an appropriate volume of the same buffer. The residual radioactivity in the glass fiber filter paper is then measured by means of a liquid scintillation counter or γ-counter. A test compound exceeding 0 cpm in count obtained by subtracting nonspecific binding (NSB) from the total binding (B) (B minus NSB) may be selected as the compound that promotes the activity of the polypeptide of the present invention.

The method (4) or (5) above for determination of a ligand to the receptor protein of the present invention can be performed as follows. The receptor protein-mediated cell stimulating activity (e.g., the activity that promotes or inhibits arachidonic acid release, acetylcholine release, intracellular $Ca^{2+}$ release, intracellular cAMP production, intracellular cGMP production, inositol phosphate production, change in cell membrane potential, phosphorylation of intracellular proteins, activation of c-fos, pH reduction, etc.) can be determined by a publicly known method, or using an assay kit commercially available. Specifically, cells containing the receptor protein are first cultured on a multi-well plate, etc. Prior to the ligand determination, the medium is replaced with fresh medium or with an appropriate non-cytotoxic buffer, followed by culturing for a given period of time in the presence of a test compound, etc. Subsequently, the cells are extracted or the supernatant is recovered and the resulting product is quantified by appropriate procedures. Where it is difficult to detect the production of the indicator substance for the cell stimulating activity (e.g., arachidonic acid) due to a degrading enzyme contained in the cells, an inhibitor against such a degrading enzyme may be added prior to the assay. For detecting activities such as the activity of inhibiting the cAMP production, the baseline production in the cells is increased by forskolin or the like and the suppressing effect on the increased baseline production can then be detected.

The kit of the present invention for determination of the ligand that binds to the receptor protein of the present invention comprises the receptor protein of the present invention, cells containing the receptor protein of the present invention, or the membrane fraction, etc. of the cells containing the receptor protein of the present invention.

Examples of the ligand determination kit of the present invention are given below.

1. Reagents for determining ligands

(1) Assay and wash buffers

Hanks' Balanced Salt Solution (manufactured by Gibco Co.) supplemented with 0.05% bovine serum albumin (Sigma Co.).

The solution is sterilized by filtration through a 0.45 μm filter and stored at 4°C. Alternatively, the solution may be prepared at use.

(2) Standard G protein-coupled receptor protein

CHO cells on which the receptor protein of the present invention has been expressed are subjected to passage culture on a 12-well plate in a density of $5 \times 10^5$ cells/well followed by culturing at 37°C under 5% $CO_2$ and 95% air for 2 days.

(3) Labeled test compounds

Compounds labeled with commercially available [$^3$H], [$^{125}$I], [$^{14}$C], [$^{35}$S], etc. or compounds labeled by appropriate methods.

An aqueous solution of the compound is stored at 4°C or -20°C. The solution is diluted to 1 μM with an assay buffer at use. A sparingly water-soluble test compound is dissolved in dimethylformamide, DMSO, methanol, etc.

(4) Non-labeled compounds

A non-labeled form of the same compound as the labeled compound is prepared in a concentration 100 to 1,000-fold higher than that of the labeled compound.

2. Method for assay

(1) CHO cells expressing the receptor protein of the present invention are cultured in a 12-well culture plate. After washing twice with 1 ml of an assay buffer, 490 µl of the assay buffer is added to each well.
(2) After 5 µl of the labeled test compound is added, the resulting mixture is cultured at room temperature for an hour. To determine the non-specific binding, 5 µl of the non-labeled compound is added to the system.
(3) The reaction mixture is removed and the wells are washed 3 times with 1 ml of washing buffer. The labeled test compound bound to the cell is dissolved in 0.2N NaOH-1% SDS and then mixed with 4 ml of liquid scintillator A (manufactured by Wako Pure Chemical Industries, Ltd.).
(4) The radioactivity is measured using a liquid scintillation counter (manufactured by Beckman Co.).

In the specification and drawings, the codes of bases and amino acids are denoted in accordance with the IUPAC-IUB Commission on Biochemical Nomenclature or by the common codes in the art, examples of which are shown below. For amino acids that may have the optical isomer, L form is presented unless otherwise indicated.

| | |
|---|---|
| DNA | : deoxyribonucleic acid |
| cDNA | : complementary deoxyribonucleic acid |
| A | : adenine |
| T | : thymine |
| G | : guanine |
| C | : cytosine |
| I | : inosine |
| R | : adenine (A) or guanine (G) |
| Y | : thymine (T) or cytosine (C) |
| M | : adenine (A) or cytosine (C) |
| K | : guanine (G) or thymine (T) |
| S | : guanine (G) or cytosine (C) |
| W | : adenine (A) or thymine (T) |
| B | : guanine (G), guanine (G) or thymine (T) |
| D | : adenine (A), guanine (G) or thymine (T) |
| V | : adenine (A), guanine (G) or cytosine (C) |
| N | : adenine (A), guanine (G), cytosine (C) or thymine (T), or unknown or other base |
| RNA | : ribonucleic acid |
| mRNA : | messenger ribonucleic acid |
| dATP | : deoxyadenosine triphosphate |
| dTTP | : deoxythymidine triphosphate |
| dGTP | : deoxyguanosine triphosphate |
| dCTP | : deoxycytidine triphosphate |
| ATP | : adenosine triphosphate |
| EDTA | : ethylenediaminetetraacetic acid |
| SDS | : sodium dodecyl sulfate |
| BHA | : benzhydrylamine |
| pMBHA : | p-methyobenzhydrylamine |
| Tos | : p-toluenesulfonyl |
| Bzl | : benzyl |
| Bom | : benzyloxymethyl |
| Boc | : t-butyloxycarbonyl |
| DCM | : dichloromethane |
| HOBt | : 1-hydroxybenztriazole |
| DCC | : N,N'-dicyclohexylcarbodiimide |
| TFA | : trifluoroacetic acid |
| DIEA | : diisopropylethylamine |
| Gly | : glycine |
| Ala or A : | alanine |
| Val or B | valine |
| Leu or L : | leucine |
| Ile or I | : isoleucine |
| Ser or S | : serine |
| Thr or T : | threonine |
| Cys or C : | cysteine |

| | |
|---|---|
| Met or M : | methionine |
| Glu or E : | glutamic acid |
| Asp or D : | aspartic acid |
| Lys or K | : lysine |
| Arg or R | : arginine |
| His or H | : histidine |
| Phe or F | : phenylalanine |
| Tyr or Y | : tyrosine |
| Trp or W | : tryptophan |
| Pro or P | : proline |
| Asn or N | : asparagine |
| Gln or Q | : glutamine |
| pGlu | : pyroglutamic acid |

The sequence identification numbers in the sequence listing of the specification indicates the following sequence, respectively.

[SEQ ID NO:1]

This shows the amino acid sequence of the polypeptide (human type) of the present invention, obtained in REFERENCE EXAMPLE 1, which will be later described.

[SEQ ID NO:2]

This shows the base sequence of DNA encoding the polypeptide of the present invention shown by SEQ ID NO:1.

[SEQ ID NO:3]

This shows the base sequence of primer F5 used in REFERENCE EXAMPLE 1 later described.

[SEQ ID NO:4]

This shows the base sequence of primer F6 used in REFERENCE EXAMPLE 1 later described.

[SEQ ID NO:5]

This shows the base sequence of primer F I used in REFERENCE EXAMPLE 1 later described.

[SEQ ID NO:6]

This shows the base sequence of primer R5 used in REFERENCE EXAMPLE 1 later described.

[SEQ ID NO:7]

This shows the base sequence of primer hR1 used in REFERENCE EXAMPLE 3 later described.

[SEQ ID NO:8]

This shows the amino acid sequence of the polypeptide (human type) of the present invention obtained in REFERENCE EXAMPLE 3 later described.

[SEQ ID NO:9]

This shows the base sequence of DNA encoding the polypeptide of the present invention represented by SEQ ID NO:8.

[SEQ ID NO:10]

This shows the base sequence of primer bF6 used in REFERENCE EXAMPLE 4 later described.

[SEQ ID NO:11]

This shows the base sequence of primer bF7 used in REFERENCE EXAMPLE 4 later described.

[SEQ ID NO:12]

This shows the base sequence of primer bR6 used in REFERENCE EXAMPLE 4 later described.

[SEQ ID NO: 13]

This shows the base sequence of primer bR7 used in REFERENCE EXAMPLE 4 later described.

[SEQ ID NO:14]

This shows the amino acid sequence of the polypeptide (bovine type) obtained in REFERENCE EXAMPLE4, which will be later described.

[SEQ ID NO:15]

This shows the base sequence of the DNA encoding the polypeptide of the present invention shown by SEQ ID NO:14.

[SEQ ID NO:16]

This shows the base sequence of primer rLPR1 used in REFERENCE EXAMPLE 5, which will be later described.

[SEQ ID NO:17]

This shows the base sequence of primer rLPF1 employed in REFERENCE EXAMPLE 5, which will be later described.

[SEQ ID NO:18]

This shows the amino acid sequence of the polypeptide (rat type) of the present invention obtained in REFERENCE EXAMPLE 5, which will be later described (before re-cloning).

[SEQ ID NO:19]

This shows the base sequence of the DNA encoding the polypeptide of the present invention shown by SEQ ID NO: 18.

[SEQ ID NO:20]

This shows the base sequence encoding RFGK sequence.

[SEQ ID NO:21]

This shows the base sequence encoding RFGR sequence.

[SEQ ID NO:22]

This shows the base sequence encoding RSGK sequence.

[SEQ ID NO:23]

This shows the base sequence encoding RSGR sequence.

[SEQ ID NO:24]

This shows the base sequence encoding RLGK sequence.

[SEQ ID NO:25]

This shows the base sequence encoding RLGR sequence.

[SEQ ID NO:26]

This shows the base sequence of primer FF2 used in REFERENCE EXAMPLE 6, which will be later described.

[SEQ ID NO:27]

This shows the base sequence of primer rR4 used in REFERENCE EXAMPLE 6, which will be later described.

[SEQ ID NO:28]

This shows the base sequence of primer mF1 used in REFERENCE EXAMPLE 6, which will be later described.

[SEQ ID NO:29]

This shows the base sequence of primer mF3 used in REFERENCE EXAMPLE 6, which will be later described.

[SEQ ID NO:30]

This shows the base sequence of primer mR1 used in REFERENCE EXAMPLE 6, which will be later described.

[SEQ ID NO:31]

This shows the base sequence of primer moF used in REFERENCE EXAMPLE 6, which will be later described.

[SEQ ID NO:32]

This shows the base sequence of primer moR used in REFERENCE EXAMPLE 6, which will be later described.

[SEQ ID NO:33]

This shows the amino acid sequence of the polypeptide (mouse type) of the present invention obtained in REFERENCE EXAMPLE 6, which will be later described.

[SEQ ID NO:34]

This shows the base sequence of the DNA encoding the polypeptide of the present invention having the amino acid sequence shown by SEQ ID NO:33.

[SEQ ID NO:35]

This shows the base sequence of primer 1 used for cloning the cDNA encoding the rat cerebellum-derived novel G protein-coupled receptor protein r0T7T022L obtained in REFERENCE EXAMPLE 7, which will be later described.

[SEQ ID NO:36]

This shows the base sequence of primer 2 used for cloning the cDNA encoding the rat cerebellum-derived novel G protein-coupled receptor protein r0T7T022 obtained in REFERENCE EXAMPLE 7, which will be later described.

[SEQ ID NO:37]

This shows the amino acid sequence of the rat cerebellum-derived novel G protein-coupled receptor protein r0T7T022L obtained in REFERENCE EXAMPLE 7, which will be later described.

[SEQ ID NO:38]

This shows the base sequence of the cDNA encoding the rat cerebellum-derived novel G protein-coupled receptor protein r0T7T022L obtained in REFERENCE EXAMPLE 7, which will be later described.

[SEQ ID NO:39]

This shows the amino acid sequence of the peptide obtained in REFERENCE EXAMPLE 7 (3), which will be later described.

[SEQ ID NO:40]

This shows the amino acid sequence of the peptide obtained in REFERENCE EXAMPLE 7 (4), which will be later described.

[SEQ ID NO:41]

This shows the amino acid sequence of the peptide obtained in REFERENCE EXAMPLE 7 (5), which will be later described.

[SEQ ID NO:42]

This shows the base sequence encoding the peptide containing the 81 (Met) - 92 (Phe) amino acid sequence in the amino acid sequence shown by SEQ ID NO:1.

[SEQ ID NO:43]

This shows the base sequence encoding the peptide containing the 101 (Ser) - 112 (Ser) amino acid sequence in the amino acid sequence shown by SEQ ID NO: 1.

[SEQ ID NO:44]

This shows the base sequence encoding the peptide containing the 124 (Val) - 131 (Phe) amino acid sequence in the amino acid sequence shown by SEQ ID NO: 1.

[SEQ ID NO:45]

This shows the base sequence encoding the peptide containing the 1 (Met) -92 (Phe) amino acid sequence in the amino acid sequence shown by SEQ ID NO:1.

[SEQ ID NO:46]

This shows the base sequence encoding the peptide containing the 1 (Met) -112 (Ser) amino acid sequence in the amino acid sequence shown by SEQ ID NO: 1.

[SEQ ID NO:47]

This shows the base sequence encoding the peptide containing the amino acid sequence of the 1 (Met) - 131 (Phe) in the amino acid sequence shown by SEQ ID NO:1.

[SEQ ID NO:48]

This shows the base sequence of primer ratF2 used in REFERENCE EXAMPLE 5.

[SEQ ID NO:49]

This shows the base sequence of primer ratR used in REFERENCE EXAMPLE 5.

[SEQ ID NO:50]

This shows the amino acid sequence of the polypeptide (rat type) of the present invention obtained in REFERENCE EXAMPLE 5, which will be later described (after re-cloning).

[SEQ ID NO:51]

This shows the base sequence of the DNA encoding the polypeptide of the present invention containing the amino acid sequence shown by SEQ ID NO:50.

[SEQ ID NO:52]

This shows the base sequence of primer bFF used in REFERENCE EXAMPLE 9.

[SEQ ID NO:53]

This shows the base sequence of primer bFR used in REFERENCE EXAMPLE 9.

[SEQ ID NO:54]

This shows the amino acid sequence coding the protein (polypeptide) represented by h0T7T022 obtained REFERENCE EXAMPLE 11.

[SEQ ID NO:55]

This shows the base sequence of the DNA encoding the protein (polypeptide) represented by h0T7T022 containing the amino acid sequence shown by SEQ ID NO:54.

[SEQ ID NO:56]

This shows the base sequence of the DNA encoding the protein (polypeptide) represented by h0T7T022 containing the amino acid sequence shown by SEQ ID NO:54.

[SEQ ID NO:57]

This shows the base sequence of primer 1 used in REFERENCE EXAMPLE 11.

[SEQ ID NO:58]

This shows the base sequence of primer 2 used in REFERENCE EXAMPLE 11.

[SEQ ID NO:59]

This shows the base sequence of primer #1 used in EXAMPLE 4.

[SEQ ID NO:60]

This shows the base sequence of primer #2 used in EXAMPLE 4.

[SEQ ID NO:61]

This shows the base sequence of primer #3 used in EXAMPLE 4.

[SEQ ID NO:62]

This shows the base sequence of primer #4 used in EXAMPLE 4.

Transformant *Escherichia coli* JM109/phRF1 obtained in REFERENCE EXAMPLE 2 later described was on deposit with the Ministry of International Trade and Industry, Agency of Industrial Science and Technology, National Institute of Bioscience and Human Technology (NIBH) as the Accession Number FERM BP-6702 on April 14, 1999 and with Institute for Fermentation (IFO) as the Accession Number IFO 16265 on March 5,1999.

Transformant *Escherichia coli* DH10B/pAK-rOT022L obtained in REFERENCE EXAMPLE 7 later described was on deposit with the Ministry of International Trade and Industry, Agency of Industrial Science and Technology, National Institute of Bioscience and Human Technology (NIBH) as the Accession Number FERM BP-6558 on November 2, 1998 and with Institute for Fermentation (IFO) as the Accession Number IFO 16211 on October 16, 1998.

Transformant *Escherichia coli* JM109/pbRF2 obtained in REFERENCE EXAMPLE 9 later described was on deposit with the Ministry of International Trade and Industry, Agency of Industrial Science and Technology, National Institute of Bioscience and Human Technology (NIBH) as the Accession Number FERM BP-6811 on August 2, 1999 and with Institute for Fermentation (IFO) as the Accession Number IFO 16288 on June 18, 1999.

Transformant *Escherichia coli* JM109/phRF2 obtained in REFERENCE EXAMPLE 8 later described was on deposit with the Ministry of International Trade and Industry, Agency of Industrial Science and Technology, National Institute of Bioscience and Human Technology (NIBH) as the Accession Number FERM BP-6812 on August 2, 1999 and with Institute for Fermentation (IFO) as the Accession Number IFO 16289 on June 18, 1999.

Transformant *Escherichia coli* JM109/pmLP4 obtained in REFERENCE EXAMPLE 6 later described was on deposit with the Ministry of International Trade and Industry, Agency of Industrial Science and Technology, National Institute of Bioscience and Human Technology (NIBH) as the Accession Number FERM BP-6813 on August 2, 1999 and with Institute for Fermentation (IFO) as the Accession Number IFO 16290 on June 18, 1999.

Transformant *Escherichia coli* JM109/prLPL6 obtained in REFERENCE EXAMPLE 5 later described was on deposit with the Ministry of International Trade and Industry, Agency of Industrial Science and Technology, National Institute of Bioscience and Human Technology (NIBH) as the Accession Number FERM BP-6814 on August 2, 1999 and with Institute for Fermentation (IFO) as the Accession Number IFO 16291 on June 18,1999.

Transformant *Escherichia coli* DH5α/pCR2.1-h0T022T obtained in REFERENCE EXAMPLE 11 later described was on deposit with the Ministry of International Trade and Industry, Agency of Industrial Science and Technology, National Institute of Bioscience and Human Technology (NIBH) as the Accession Number FERM BP-6930 on November 8, 1999 and with Institute for Fermentation (IFO) as the Accession Number IFO 16330 on October 27, 1999.

Transformant *Escherichia coli* DH5α/pCR2.1-h0T022G obtained in REFERENCE EXAMPLE 11 later described was on deposit with the Ministry of International Trade and Industry, Agency of Industrial Science and Technology, National Institute of Bioscience and Human Technology (NIBH) as the Accession Number FERM BP-6931 on November 8, 1999 and with Institute for Fermentation (IFO) as the Accession Number IFO 16331 on October 27, 1999.

Transformant *Escherichia coli* MM294(DE3)/pTFCRFRP-1 obtained in EXAMPLE 5 later described was on deposit with the Ministry of International Trade and Industry, Agency of Industrial Science and Technology, National Institute of Bioscience and Human Technology (NIBH) as the Accession Number FERM BP-7313 on September 28, 2000 and with Institute for Fermentation (IFO) as the Accession Number IFO 16476 on September 19, 2000.

Anti-rat type RFRP-1 monoclonal antibody IF3 obtained in REFERENCE EXAMPLE 12 later described was on deposit with the Ministry of International Trade and Industry, Agency of Industrial Science and Technology, National Institute of Bioscience and Human Technology (NIBH) as the Accession Number FERM BP-7463 on February 21, 2001 and with Institute for Fermentation (IFO) as the Accession Number IFO 50527 on January 16, 2001.

EXAMPLES

The present invention is described in detail below with reference to EXAMPLES and REFERENCE EXAMPLES, but not limited thereto. The gene manipulation procedures using *Escherichia coli* were performed according to the methods described in the Molecular Cloning.

REFERENCE EXAMPLE 1 Synthesis of cDNA from human fetal brain poly(A)$^+$RNA fraction and amplification of physiologically active peptide cDNA by RT-PCR

Oligo dT primer (Gibco BRL Inc.) was added as a primer to 1μg of human fetal brain poly(A)$^+$RNA fraction available from Clontech and cDNA was synthesized with reverse transcriptase from Moloney murine leukemia virus (Gibco BRL Inc.) using a buffer attached thereto. After completion of the reaction, the product was extracted with phenol: chloroform (1:1) and the extract was precipitated with ethanol. The precipitate was dissolved in 30 μl of TE. Using a 1 μl aliquot of the thus prepared cDNA as a template, amplification was performed by PCR using the following two primers (F5 and F6).

F5: 5'-GGGCTGCACATAGAGACTTAATTTTAG-3' (SEQ ID NO:3)

F6: 5'-CTAGACCACCTCTATATAACTGCCCAT-3' (SEQ ID NO:4)

The reaction solution was composed of 20 pM each of the synthetic DNA primers (F5 and F6), 0.25 mM dNTPs, 0.5 μl of Ex Taq DNA polymerase and 5 μl of a buffer attached to the enzyme, which were mixed together to make the total volume of the reaction solution 50 μl. Using Thermal Cycler (Perkin-Elmer Co.) for amplification, one cycle was set to include 98°C for 10 seconds, 63°C for 20 seconds and 72°C for 40 seconds. This cycle was repeated 40 times in total.

Using a 1 μl aliquot of the PCR product as a template, the following two primers (F1 and R5) were amplified by nested PCR.

F1: 5'-GCACATAGAGACTTAATTTTAGATTTAGAC-3' (SEQ ID NO:5)

R5: 5'-CATGCACTTTGACTGGTTTCCAGGTAT-3' (SEQ ID NO:6)

The reaction solution was composed of 20 pM each of the synthetic DNA primers (F1 and R5), 0.25 mM dNTPs, 0.5 μl of Ex Taq DNA polymerase and 5 μl of a buffer attached to the enzyme, which were mixed together to make the total volume of the reaction solution 50 μl. Using Thermal Cycler (Perkin-Elmer Co.) for amplification, one cycle was set to include 98°C for 10 seconds, 60°C for 20 seconds and 72°C for 40 seconds. This cycle was repeated 40 times in total. The amplification product was confirmed by 1.2% agarose electrophoresis and ethidium bromide staining.

REFERENCE EXAMPLE 2 Subcloning of the PCR products into plasmid vectors and selection of novel physiologically active peptide candidate clone by analyzing base sequence of the inserted cDNA region

The PCR products obtained after the PCR procedure in REFERENCE EXAMPLE 1 were separated by using a 1.2% agarose gel. After DNA fragments were proven to be amplified to the desired size, the DNAs were recovered using Quigen PCR purification kit (Quiagen). According to the protocol attached to TA Cloning Kit (Invitrogen Co.), the recovered DNAs were subcloned to plasmid vector pCR™2.1. The recombinant vectors were introduced into *Escherichia coli* JM109 competent cells (Takara Shuzo Co., Ltd.) for transformation. Then, the resulting transformant clones bearing a cDNA-inserted fragment were selected in an LB agar culture medium supplemented with ampicillin, IPTG and X-gal. Only transformant clones that showed white color were picked up with a sterilized toothpick to obtain transformant *Escherichia coli* JM109/phRF1.

After the individual clones were cultured overnight in an LB culture medium containing ampicillin, the clones were treated with an automated plasmid extracting machine (Kurabo Co., Ltd.) to prepare plasmid DNAs. An aliquot of the DNAs thus prepared was cleaved by EcoRI to confirm the size of the cDNA fragment inserted. An aliquot of the remaining DNAs was further treated with RNase, extracted with phenol/chloroform followed by concentrating the aliquot through ethanol precipitation. Sequencing was carried out by using DyeDeoxy Terminator Cycle Sequencing Kit (ABI Inc.), the DNAs were decoded using an automated fluorescent sequencer. The data of the base sequences obtained were read by DNASIS (Hitachi System Engineering Co., Ltd.). The base sequence determined is shown in FIG. 1.

The base sequence thus determined was subjected to homology search and sequence analysis based on FIG. 1. The results reveal that the novel physiologically active peptide was encoded by the cDNA fragment inserted in the plasmid of the transformant *Escherichia coli* JM109/phRF1.

REFERENCE EXAMPLE 3 Acquisition of splicing variant of the physiologically active peptide cDNA from human fetal brain cDNA

Using as a template 1 ml of the human fetal brain cDNA prepared in REFERENCE EXAMPLE 1, amplification was performed by PCR using the following two primers (F5 and hR1).

F5: 5'-GGGCTGCACATAGAGACTTAATTTTAG-3' (SEQ ID NO:3)

hR1: 5'-CAGCTTTAGGGACAGGCTCCAGGTTTC-3' (SEQ ID NO:7)

The reaction solution was composed of 20 pM each of the synthetic DNA primers (F5 and hR1), 0.25 mM dNTPs, 0.5 ml of Ex Taq DNA polymerase and a buffer attached to the enzyme, which were mixed together to make the total volume of the reaction solution 50 ml. Using Thermal Cycler (Perkin-Elmer Co.) for amplification, one cycle was set to include 98°C for 10 seconds, 65°C for 20 seconds and 72°C for 20 seconds. This cycle was repeated 40 times in total. The amplification product was confirmed by 1.2% agarose electrophoresis and ethidium bromide staining. After the PCR product was proven to be amplified, the reaction product was purified using QIA Quick PCR Purification Kit (Quiagen), followed by sequencing. The sequencing reaction was conducted using BigDye Deoxy Terminator Cycle Sequence Kit (ABI Inc.). The DNAs were analyzed using an automated fluorescent sequencer (ABI377). The data of the base sequences obtained were read by DNASIS (Hitachi System Engineering Co., Ltd.). As a result, cDNA with the 3' terminus different from the cDNA obtained in REFERENCE EXAMPLE 2 was obtained. The cDNA thus obtained in this REFERENCE EXAMPLE was found to be a splicing variant of the cDNA obtained in REFERENCE EXAMPLE 2. The base sequence determined (SEQ ID NO:9) and the deduced amino acid sequence (SEQ ID NO:8) are shown in FIG. 3.

REFERENCE EXAMPLE 4 Acquisition of physiologically active peptide cDNA from bovine hypothalamus poly(A)$^+$RNA

Bovine type physiologically active peptide cDNA was obtained from bovine hypothalamus poly(A)$^+$RNA using Marathon cDNA Amplification Kit (Clontech). Using as a template bovine hypothalamus cDNA prepared in accordance with the manual attached to the Kit, the following four primers (bF6, bF7, bR6 and bR7) were synthesized and employed in combination with two primers AP 1 and AP2 attached to the Kit to effect amplification by PCR.

bF6: 5'-GCCTAGAGGAGATCTAGGCTGGGAGGA-3' (SEQ ID NO:10)

bF7: 5'-GGGAGGAACATGGAAGAAGAAAGGAGC-3' (SEQ ID NO:11)

bR6: 5'-GATGGTGAATGCATGGACTGCTGGAGC-3' (SEQ ID NO:12)

bR7: 5'-TTCCTCCCAAATCTCAGTGGCAGGTTG-3' (SEQ ID NO:13)

For amplification of the 5' terminus (N-terminal region), a first PCR was carried out using the synthetic primers (bR6 and AP1). The reaction solution composed of 20 pM each of the synthetic DNA primers, 0.25 mM dNTPs, 0.5 ml of Klen Taq. DNA polymerase and a buffer attached to the enzyme was made the total volume of the reaction solution 25 ml. Using Thermal Cycler (Perkin-Elmer Co.) for amplification, one cycle was set to include 98°C for 10 seconds and 72°C for 2 minutes, which cycle was repeated 5 times, further cycle was set to include 98°C for 10 seconds and 70°C for 2 minutes, which cycle was repeated 5 times, and another cycle set to include 98°C for 10 seconds and 68°C for 2 minutes and 30 seconds, which cycle was repeated 25 times. Then, the reaction solution of the first PCR was diluted to 10-fold, 1 ml of the aliquot was used as a template to perform a second PCR using (bR7 and AP2) as primers.

The reaction solution composed of 20 pM each of the primers, 0.25 mM dNTPs, 0.5 ml of Klen Taq DNA polymerase and a buffer attached to the enzyme was made the total volume of the reaction solution 25 ml. Using a Thermal Cycler (Perkin-Elmer Co.) for amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds and 68°C for 2 minutes and 30 seconds, which cycle was repeated 35 times.

For amplification of the 3' terminus (C-terminal region), a first PCR was carried out using the synthetic primers (bF6 and AP1). The reaction solution composed of 20 pM each of the primers, 0.25 mM dNTPs, 0.5 ml of Klen Taq DNA polymerase and a buffer attached to the enzyme was made the total volume of the reaction solution 25 ml. Using Thermal Cycler (Perkin-Elmer Co.) for amplification, one cycle was set to include 98°C for 10 seconds and 72°C for 2 minutes, which cycle was repeated 5 times, and another cycle set to include 98°C for 10 seconds and 70°C for 2 minutes, which cycle was repeated 5 times and a further cycle set to include 98°C for 10 seconds and 68°C for 2 minutes and 30 seconds, which cycle was repeated 25 times. Then, the reaction solution of the first PCR was diluted to 10-fold, 1 ml of the aliquot was used as a template to perform a second PCR using (bF7 and AP2) as primers. The reaction solution composed of 20 pM each of the primers, 0.25 mM dNTPs, 0.5 ml of Klen Taq DNA polymerase and a buffer attached to the enzyme was made the total volume of the reaction solution 25 ml. Using Thermal Cycler (Perkin-Elmer Co.) for amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds and 68°C for 2 minutes and 30 seconds, which cycle was repeated 35 times. The amplification products at the 5' and 3' termini were confirmed by 1.2% agarose gel electrophoresis and ethidium bromide staining, respectively. After the PCR product was confirmed to be amplified, the reaction product was purified using QIA quick PCR purification Kit (Quiagen), followed by sequencing. The sequencing reaction was conducted using BigDye Deoxy Terminator Cycle Sequence Kit (ABI). The DNAs were analyzed using an automated fluorescent sequencer (ABI377).

The data of the base sequences obtained were read by DNASIS (Hitachi System Engineering Co., Ltd.). The base sequence determined (SEQ ID NO: 15) and the deduced amino acid sequence (SEQ ID NO:14) are shown in FIG. 4.

REFERENCE EXAMPLE 5 Acquisition of physiologically active peptide cDNA from rat brain poly(A)$^+$RNA

Rat type physiologically active peptide cDNA was obtained from rat brain poly(A)$^+$RNA using Marathon cDNA Amplification Kit (Clontech). Using as a template rat brain cDNA prepared in accordance with the manual attached to the Kit, the following two primers were synthesized and employed in combination with two primers AP1 and AP2 attached to the Kit to effect amplification by PCR.

rLPR1: 5'-CCCTGGGGCTTCTTCTGTCTTCTATGT-3' (SEQ ID NO:16)

rLPF1: 5'-AGCGATTCATTTTATTGACTTTAGCA-3' (SEQ ID NO:17)

For amplification of the 5' terminus (N-terminal region), a first PCR was carried out using the primer set of rLPR1 and AP1. The reaction solution composed of 200 pM each of the primers, 0.1 mM dNTPs, 0.25 ml of Klen Taq DNA polymerase was made the total volume of the reaction solution 25 ml with a buffer attached to the enzyme. Using Thermal Cycler (Perkin-Elmer Co.) for amplification, one cycle was set to include 98°C for 10 seconds and 72°C for 2 minutes, which cycle was repeated 5 times, another cycle set to include 98°C for 10 seconds and 70°C for 2 minutes, which cycle was repeated 5 times and then a further cycle set to include 98°C for 10 seconds and 68°C for 2 minutes and 30 seconds, which cycle was repeated 25 times. Then, a second PCR was performed using the first PCR solution as a template, the first set of primers and the same compositions of he reaction solution. For amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds (68°C for 2 minutes and 30 seconds), which cycle was repeated 38 times.

For amplification of the 3' terminus (C-terminal region), a first PCR was carried out using the primer set of rLPF1 and AP1. The composition of the reaction solution was the same as that for amplification of the 5'-terminus (N-terminal region). For amplification, one cycle was set to include 98°C for 10 seconds and 72°C for 2 minutes, which cycle was repeated 5 times, another cycle set to include 98°C for 10 seconds and 72°C for 2 minutes, which cycle was repeated 5 times and a further cycle set to include 98°C for 10 seconds, 65°C for 20 seconds and 72°C for 2 minutes, which cycle was repeated 25 times. Then, the reaction solution of the first PCR was used as a template to perform a second PCR using rLPF1 and AP2 primers. The composition of the reaction solution was the same as that for the first PCR. Using Thermal Cycler (Perkin-Elmer Co.) for amplification, one cycle was set to include 98°C for 10 seconds and 72°C for 2 minutes, which cycle was repeated 5 times, followed by another cycle set to include 98°C for 10 seconds and 70°C for 2 minutes, which cycle was repeated 5 times and then a further cycle set to 98°C for 10 seconds, 65°C for 20 seconds and 72°C for 2 minutes, which cycle was repeated 38 times. The amplification products at the 5' and 3' termini were confirmed by 1.2% agarose gel electrophoresis and ethidium bromide staining, respectively. The PCR product band was purified using QIA quick Gel Extrication Kit (Quiagen), followed by sequencing. The sequencing was conducted in a manner similar to REFERENCE EXAMPLE 3. The base sequence determined (SEQ ID NO:19) and the deduced amino acid sequence (SEQ ID NO: 18) are shown in FIG. 5. Based on the sequences, two primers were synthesized around the initiation and termination codons.

ratF2: 5'-AATGGAAATTATTTCATCAAAGCGATTCAT-3' (SEQ ID NO:48)

ratR: 5'-CACCTATACTGACAGGAATGATGGCTCTCC-3' (SEQ ID NO:49)

Using as a template cDNA that was synthesized from rat hypothalamus poly(A)$^+$ RNA using AMV reverse transferase (Takara Shuzo Co., Ltd.) and random 9 mer (Takara Shuzo Co., Ltd.), PCR was carried out by repeating 33 times a cycle set to include 98°C for 10 seconds and 68°C for 40 seconds. Using the reaction solution as a template, PCR was carried out by repeating 38 times a cycle set to include 98°C for 10 seconds and 68°C for 1 minute to obtain the PCR product of about 690 bp. The PCR product was inserted to cloning vector pCR2.1 TOPO following the instructions attached to TA cloning kit (Invitrogen Inc.), which was then introduced into *Escherichia coli* JM109 to obtain transformant E. *coli* JM109/prLPL6. The base sequence was determined in a manner similar to REFERENCE EXAMPLE 3 (SEQ ID NO:51), from which the amino acid sequence (SEQ ID NO:50) was deduced.

REFERENCE EXAMPLE 6 Acquisition of mouse type physiologically active peptide cDNA from mouse brain poly (A)$^+$RNA by the Marathon PCR method and confirmation of its sequence

To acquire mouse type physiologically active peptide cDNA from mouse brain poly(A)$^+$RNA, firstly 1μg of mouse brain poly(A)$^+$RNA was reacted with Superscript II RNase H-reverse transcriptase (GIBCO BRL) at 42°C for an hour in the presence of 2.5 pmols of oligo d(T) primer (Takara Shuzo Co., Ltd.), 0.5 mM dNTPs and 10 mM DTT to synthesize cDNA. Using the cDNA as a template, PCR was carried out using the following primers and Klen Taq DNA polymerase (Clontech), while repeating 39 times a cycle set to include 98°C for 10 seconds, 56°C for 20 seconds and 72°C for 25 seconds.

FF2: 5'-GACTTAATTTTAGATTTAGACAAAATGGAA-3' (SEQ ID NO:26)

rR4: 5'-TTCTCCCAAACCTTTGGGGCAGGTT-3' (SEQ ID NO:27)

Further using the same primer set, PCR was carried out by repeating 39 times a cycle set to include 98°C for 10 seconds, 60°C for 20 seconds and 72°C for 25 seconds. The amplification product was confirmed by 1.2% agarose gel electrophoresis and ethidium bromide staining. The band was purified using QIA quick Gel Extrication Kit (Quiagen), followed by sequencing in a manner similar to REFERENCE EXAMPLE 3. To obtain the 5' and 3' terminal sequences of the mouse type physiologically active peptide cDNA fragment, cDNA was synthesized from 1 μg of mouse brain poly(A)$^+$ RNA in a manner similar to REFERENCE EXAMPLE 5, using Marathon cDNA Amplification Kit (Clontech) to use the cDNA as a template. The following three primers were synthesized and used for PCR in combination with AP 1 primer attached to the kit.

mF1: 5'-ACAGCAAAGAAGGTGACGGAAAATACTC-3' (SEQ ID NO:28)

mF3: 5'-ATAGATGAGAAAAGAAGCCCCGCAGCAC-3' (SEQ ID NO:29)

mR1: 5'-GTGCTGCGGGGCTTCTTTTCTCATCTAT-3' (SEQ ID NO:30)

For amplification of the 5' terminus, a first PCR was carried out using the primer set of mR1 and AP1. For amplification of the 3' terminus (C-terminal region), a first PCR was carried out using the primer set of mF 1 and AP 1. The reaction solution composed of 200 pM each of the primers, 0.1 mM dNTP, 0.25 ml of Klen Taq DNA polymerase was made the total volume of the reaction solution 25 ml with a buffer attached to the enzyme. For amplification, one cycle was set to include 98°C for 10 seconds and 72°C for 2 minutes, which cycle was repeated 5 times, another cycle set to include 98°C for 10 seconds and 70°C for 2 minutes, which cycle was repeated 5 times and a further cycle set to include 98°C for 10 seconds and 68°C for 2 minutes and 30 seconds, which cycle was repeated 25 times. Then, the reaction solution of the first PCR was used as a template to perform a second PCR. Amplification at the 5' terminus was performed using the same primer set as in the first PCR and for amplification at the 3' terminus, the same composition of the reaction solution as in the first PCR was prepared, using the primer set of mF3 and AP1. PCR was carried out by repeating 5 times a cycle set to include 98°C for 10 seconds and 72°C for 2 minutes, 5 times another cycle set to include 98°C for 10 seconds and 70°C for 2 minutes, and then 38 times a further cycle set to 98°C for 10 seconds and 68°C for 2 minutes and 30 seconds.

The amplification products at the 5' and 3' termini were confirmed by 1.2% agarose gel electrophoresis and ethidium bromide staining, respectively. The PCR product band was purified using QIA quick Gel Extrication Kit (Quiagen), followed by sequencing. The sequencing was conducted in a manner similar to REFERENCE

EXAMPLE 3.

Based on the sequences, two primers were further synthesized.

moF: 5'-TTTAGACTTAGACGAAATGGA-3' (SEQ ID NO:31)

moR: 5'-GCTCCGTAGCCTCTTGAAGTC-3' (SEQ ID NO:32)

Using as a template the above-described cDNA that was synthesized from mouse brain poly(A)$^+$ RNA using Superscript II RNase H-reverse transcriptase, PCR was carried out to amplify a fragment containing mouse physiologically active peptide full-length cDNA. The reaction was carried out using Klen Taq DNA polymerase (Clontech), by repeating 35 times a cycle set to include 98°C for 10 seconds, 56°C for 20 seconds and 72°C for 15 seconds. The amplification product of about 600 bp was confirmed by 2% agarose electrophoresis and ethidium bromide staining. The band was purified using QIA quick Gel Extrication Kit (Quiagen), subcloned to cloning vector pCR2.1-TOPO (TOPO TA cloning kit, Invitrogen Inc.) and then introduced into *Escherichia coli* JM109 to obtain transformant E. coli JM 109/pmLP4. The base sequence was determined in a manner similar to REFERENCE EXAMPLE 3. The base sequence thus determined (SEQ ID NO:34) and the deduced amino acid sequence (SEQ ID NO:33) therefrom are shown in FIG. 7.

REFERENCE EXAMPLE 7

(1) Cloning of the cDNA encoding the rat cerebellum-derived G protein-coupled receptor protein and determination of the base sequence

Using rat cerebellum-derived cDNA as a template and two primers, namely, primer 1 (SEQ ID NO :35) and primer 2 (SEQ ID NO :36), PCR was carried out. The reaction solution in the above reaction comprised of 1/10 volume of the cDNA, 1/50 volume of Advantage cDNA Polymerase Mix (CLONTEC Inc.), 0.2 µM of primer 1 (SEQ ID NO :35), 0.2 µM of primer 2 (SEQ ID NO :36), 200 µM dNTPs and a buffer attached to the enzyme to make the final volume 50 µl. The PCR was carried out by cycles of (1) 94°C for 2 minutes, (2) then a cycle set to include 94°C for 30 seconds followed by 72°C for 2 minutes, which was repeated 3 times, (3) a cycle set to include 94°C for 30 seconds followed by 68°C for 2 minutes, which was repeated 3 times, (4) a cycle set to include 94°C for 30 seconds followed by 64°C for 30 seconds and 68°C for 2 minutes, which was repeated 30 times, and (5) finally, extension reaction at 68°C for 8 minutes. After completion of the PCR reaction, the product was subcloned to plasmid vector pCR2.1 (Invitrogen Inc.)

following the instructions attached to the TA cloning kit (Invitrogen Inc.), which was then introduced into *Escherichia coli* DH5α, and the clones containing the cDNA were selected on LB agar plates containing ampicillin. The sequence of each clone was analyzed to give the cDNA sequence (SEQ ID NO :38) encoding the novel G protein-coupled receptor protein. The novel G protein-coupled receptor protein containing the amino acid sequence (SEQ ID NO :1) deduced therefrom was designated rOT7T022L.

Plasmid pAK-rOT7T022L in which the cDNA (SEQ ID NO:38) encoding the rat cerebellum-derived G protein-coupled receptor protein rOT7T022L of the present invention was subcloned was introduced into *Escherichia coli* DH10B according to a publicly known method to give transformant *Escherichia coli* DH10B/pAK-rOT7T022L.

(2) Establishment of G protein-coupled receptor protein rOT7T022L-expressing CHO cells

CHOdhfr⁻ cells of 1 x $10^6$ were inoculated on Petri's dish of a 10 mm diameter for tissue culture followed by culturing for 24 hours. Using 20 μg of rOT7T022L-expressing vector pAK-rOT7T022L obtained (1), DNA-liposome complex was formed by the liposome method using a gene transfer kit (Gene Transfer, Nippon Gene Co.). After a fresh medium was exchanged for the medium, the DNA-liposome complex was added to the medium and cultured overnight. After the medium was replaced with a fresh medium and further culturing was performed for one day, the medium was exchanged for transformant selection followed by culturing for further 2 days. The cells in the Petri's dish were recovered by treatment with trypsin-EDTA. By culturing again in a dilute cell density, the ratio of transformants was increased thereby to obtain the clones of cell line CHO-rOT7T022L capable of stably expressing rOT7T022L in a high level.

(3) Synthesis of Met-Pro-His-Ser-Phe-Ala-Asn-Leu-Pro-Leu-Arg-Phe-$NH_2$ (SEQ ID NO:39)

Commercially available p-methyl BHA resin, 0.5 mmol, (manufactured by Applied Biosystems, now Perkin-Elmer Inc.) was charged in a reaction tank of peptide synthesizer (430A manufactured by Applied Biosystems). After swelling with DCM, first amino acid Boc-Phe was activated with the HOBt/DCC method and then introduced into p-methyl BHA resin. The resin was treated with 50% TFA/DCM to remove Boc, wherein the amino group was liberated and neutralized with DIEA. Next amino acid Boc-Arg(Tos) was condensed to the amino group by the HOBt/DCC method. Ninhydrin test was conducted to examine if any unreacted amino group was present. After it was confirmed that the reaction was completed, Boc-Leu, Boc-Pro, Boc-Leu, Boc-Asn, Boc-Ala, Boc-Phe, Boc-Ser(Bzl), Boc-His(Bom), Boc-Pro and Boc-Met were introduced in this order.

The resin in which all amino acids of the sequence were introduced was treated with 50% TFA/DCM to remove the Boc groups on the resin. Thereafter, the resin was dried to give 0.73 g of Met-Pro-His(Bom)-Ser(Bzl)-Phe-Ala-Asn-Leu-Pro-Leu-Arg(Tos)-Phe-pMBHA-resin.

In a Teflon-made hydrogen fluoride reactor, 0.25 g of the resin was reacted in 15 ml of hydrogen fluoride together with 5.1 g of p-cresol at 0°C for 60 minutes. After removing the hydrogen fluoride by distillation in vacuum, 100 ml of diethyl ether was added to the residue, stirred and filtered through a glass filter followed by drying. The dried product was suspended in 50 ml of 50% acetic acid aqueous solution and stirred. After the peptide was extracted, it was separated from the resin and concentrated to about 5 ml in vacuum. The concentrate was applied to a column of Sephadex G-25 (2 x 90 cm) and developed with 50% acetic acid aqueous solution. Main fractions were collected and lyophilized. Next, the crudely purified peptide was dissolved in 1.5 ml of 5% thioglycolic acid/50% acetic acid. The solution was kept at 50°C for 12 hours to reduce the Met-oxidized peptide. The peptide was applied to a reversed phase column filled up with LiChroprep (trade name) RP-18 (manufactured by MERCK Inc.) followed by repeating purification with gradient elution using 0.1% aqueous TFA and 33% acetonitrile aqueous solution containing 0.1% TFA. Fractions eluted at the acetonitrile concentration of about 27% were collected and lyophilized to give 26 mg of white powders.
Mass spectrum $(M+H)^+$ 1428.7 (calcd. 1428.8)
Elution time on HPLC: 18.0 mins.
Column conditions:

Column: Wakosil (trademark) 5C18 (4.6 x 100 mm)
Eluant: linear density gradient elution (25 mins.) with solution A to solution B, using solution A (5% aqueous acetonitrile solution containing 0.1% TFA) and solution B (55% aqueous acetonitrile solution containing 0.1 % TFA)
Flow rate: 1.0 ml/min.

(4) Synthesis of Val-Pro-Asn-Leu-Pro-Gln-Arg-Phe-$NH_2$ (SEQ ID NO:40)

As in REFERENCE EXAMPLE 7 (3) described above, Boc-Phe, Boc-Arg(Tos), Boc-Gln, Boc-Pro, Boc-Leu, Boc-Asn, Boc-Pro and Boc-Val were condensed in this order to give 0.43 g of Boc-Val-Pro-Asn-Leu-Pro-Gln-Arg(Tos)-Phe-pMBHA-resin. In a manner similar to the above, 0.22 g of the resin was treated with hydrogen fluoride and purified by column chromatography to give 46 mg of the product as white powders.
Mass spectrum $(M+H)^+$ 969.5 (calcd. 969.6)
Elution time on HPLC: 11.8 mins.
Column conditions:

Column: Wakosil (trademark) 5C18 (4.6 x 100 mm)
Eluant: linear density gradient elution (25 mins.) with solution A to solution B, using solution A (5% aqueous acetonitrile solution containing 0.1% TFA) and solution B (55% aqueous acetonitrile solution containing 0.1 % TFA)
Flow rate: 1.0 ml/min.

(5) Synthesis of Ser-Ala-Gly-Ala-Thr-Ala-Asn-Leu-Pro-Arg-Ser-$NH_2$ (SEQ ID NO:41)

As in REFERENCE EXAMPLE 7 (3) described above, Boc-Ser(Bzl), Boc-Arg(Tos), Boc-Leu, Boc-Pro, Boc-Leu, Boc-Asn, Boc-Ala, Boc-Thr(Bzl), Boc-Ala, Boc-Gly, Boc-Ala and Boc-Ser(Bzl) were condensed in this order to give 0.62 g of Boc-Ser(Bzl)-Ala-Gly-Ala-Thr(Bzl)-Ala-Asn-Leu-Pro-Leu-Arg(Tos)-Ser(Bzl)-pMBHA-resin. In a manner similar to the above, 0.23 g of the resin was treated with hydrogen fluoride and purified by column chromatography to give 71 mg of the product as white powders.
Mass spectrum $(M+H)^+$ 1156.4 (calcd. 1156.6)
Elution time on HPLC: 11.8 mins.
Column conditions:

Column: Wakosil (trademark) 5C18 (4.6 x 100 mm)
Eluant: linear density gradient elution (25 mins.) with solution A to solution B, using solution A (5% aqueous acetonitrile solution containing 0.1% TFA) and solution B (55% aqueous acetonitrile solution containing 0.1% TFA)
Flow rate: 1.0 ml/min.

(6) Reaction of r0T7T022L (SEQ ID NO:37) and peptide MPHSFANLPLRFamide (SEQ ID NO:39) and peptide VPNLPQRFamide (SEQ ID NO:40) with cytosensor

The r0T7T022L receptor-expressing CHO cells obtained in REFERENCE EXAMPLE 7 (2) above were inoculated on capsules for cytosensor in 2.7 x $10^5$ cells/capsule. After culturing overnight, the cytosensor was mounted to a work station for the cytosensor. A medium for assay (low buffered RMPI1640 medium supplemented with 0.1% bovine serum albumin), that was set in the flow path of the cytosensor, was supplied to the cells in a pump cycle of ON (80 seconds) and OFF (40 seconds). A rate of change in extracellular cells from 8 to 30 seconds after the pump stopped was calculated as an acidification rate. A change of the acidification rate with passage of time was monitored; when stable reading was obtained, the flow path was switched to expose each peptide to the cells for 7 minutes and 2 seconds. In the acidification rate of each well, the data for 3 cycles immediately before the peptide exposure was made 100% for standardization. Comparison of cell reactions reveals that r0T7T022L-expressing CHO cells strongly showed dose-dependent reaction with peptide MPHSFANLPLRFamide (SEQ ID NO:39) and peptide VPNLPQRFamide (SEQ ID NO:40) (FIG. 8).

REFERENCE EXAMPLE 8 Construction of transformant bearing splicing variant cDNA for human novel physiologically active peptide candidate

The reaction product obtained after PCR in REFERENCE EXAMPLE 3 *supra* was separated using 1.2% agarose gel. After DNA fragments were proven to be amplified to the desired size, the DNAs were recovered using Quigen PCR purification kit (Quiagen). According to the protocol attached to TA Cloning Kit (Invitrogen Co.), the recovered DNAs were subcloned to plasmid vector pCR™2.1. The recombinant vectors were introduced into *Escherichia coli* JM109 competent cells (Takara Shuzo Co.) for transformation. Then, the resulting clones bearing the cDNA-inserted fragment were selected in an LB agar culture medium supplemented with ampicillin, IPTG and X-gal. Only transformant clones that showed white color were picked up with a sterilized toothpick. Each clone was cultured overnight in an LB culture medium supplemented with ampicillin and plasmid DNA was prepared using an automated plasmid extracting machine (Kurabo Co., Ltd.). An aliquot of the DNAs thus prepared was cleaved by EcoRI to confirm the size of the cDNA fragment inserted. An aliquot of the remaining DNAs was further treated with RNase, extracted with phenol/chloroform followed by concentrating the aliquot through ethanol precipitation. The reaction for sequencing was carried out by using DyeDeoxy Terminator Cycle Sequencing Kit (ABI Inc.), the DNAs were decoded using an automated fluorescent sequencer to obtain transformant *Escherichia coli* JM109/phRF2.

REFERENCE EXAMPLE 9 Construction of transformant bovine novel physiologically active peptide cDNA

Using as a template 1 ml of the bovine hypothalamus cDNA prepared in REFERENCE EXAMPLE 4, amplification was performed by PCR using the following two primers (bFF and bFR).

bFF: 5'-TTCTAGATTTTGGACAAAATGGAAATT-3' (SEQ ID NO:52)

bFR: 5'-CGTCTTTAGGGACAGGCTCCAGATTTC-3' (SEQ ID NO:53)

The reaction solution was composed of 20 pM each of the synthetic primers (bFF and bFR), 0.25 mM dNTPs, 0.5 ml of Ex Taq DNA polymerase and a buffer attached to the enzyme, which were mixed together to make the total volume 50 ml. Using Thermal Cycler (Perkin-Elmer Co.) for amplification, one cycle was set to include 98°C for 10 seconds, 65°C for 20 seconds and 72°C for 20 seconds, which cycle was repeated 40 times. The amplification product was confirmed by 1.2% agarose electrophoresis and ethidium bromide staining. The reaction product obtained after PCR in REFERENCE EXAMPLE 3 was separated using 1.2% agarose gel. After the DNA fragments were proven to be amplified to the desired size, the DNAs were recovered using Quigen PCR Purification Kit (Quiagen). According to the protocol attached to TA Cloning Kit (Invitrogen Co.), the recovered DNAs were subcloned to plasmid vector pCR™2.1. The recombinant DNA was introduced into *Escherichia coli* JM109 competent cells (Takara Shuzo Co., Ltd.) for transformation. Then, the resulting clones bearing a cDNA-inserted fragment were selected in an LB agar culture medium supplemented with ampicillin, IPTG and X-gal. Only clones that showed white color were picked up with a sterilized toothpick. Each clone was cultured overnight in an LB culture medium supplemented with ampicillin and plasmid DNA was prepared using an automated plasmid extracting machine (Kurabo Co., Ltd.). An aliquot of the DNAs thus prepared was cleaved by EcoRI to confirm the size of the cDNA fragment inserted. The DNAs prepared were further treated with RNase, extracted with phenol/chloroform and the extract was concentrated by ethanol precipitation. The reaction for sequencing was carried out by using DyeDeoxy Terminator Cycle Sequencing Kit (ABI Inc.), the DNAs were decoded using an automated fluorescent sequencer to obtain transformant *Escherichia coli* JM109/pbRF2.

REFERENCE EXAMPLE 10 Activity of inhibiting the cAMP production of peptide MPHSFANLPLRFamide (SEQ ID NO:39) and peptide VPNLPQRFamide (SEQ ID NO:40) for r0T7T022L(SEQ ID NO:37)-expressing CHO cells

It was confirmed by the site sensor experiment of REFERENCE EXAMPLE 7 (6) that peptide MPHSFANLPLRFamide (SEQ ID NO:39) and peptide VPNLPQRFamide (SEQ ID NO:40) synthesized in REFERENCE EXAMPLE 7 (3) and (4) specifically reacted with the r0T7T022L receptor. Next, the cAMP production-inhibiting activity of the peptides for the r0T7T022L-expressing CHO cells were evaluated.

The r0T7T022L-expressing CHO cells obtained in REFERENCE EXAMPLE 7 (2) above were inoculated on a 24-well plate in a concentration of 1.0 x $10^5$ cells/well, followed by culturing at 37°C for 2 days. After the cells were rinsed with Hanks' buffer (HBSS) supplemented with 0.05% BSA and 0.2 mM IBMX, the system was allowed to stand at 37°C for 30 minutes in the same buffer.

Thirty minutes after, an assay buffer was prepared by adding the cells to Hanks' buffer supplemented with $10^{-6}$ M forskolin and at the same time, the peptides described above were added thereto in various concentrations. Culturing was performed at 37°C for 30 minutes. According to the method given in cAMP EIA Kit (Amersham Inc.), the cAMP level in the cells of each well was measured 30 minutes after. As shown in FIG. 9, peptide MPHSFANLPLRFamide (SEQ ID NO:39) and peptide VPNLPQRFamide (SEQ ID NO:40) showed a potent effect of inhibiting the cAMP production on r0T7T022L receptor-expressing CHO cells at $IC_{50}$ of 0.5 nM and 0.7 nM, respectively, indicating that the peptide concentrations were very low.

REFERENCE EXAMPLE 11 Cloning of the cDNA encoding human hypothalamus G protein-coupled receptor protein and determination of its base sequence

Using human hypothalamus cDNA (CLONTECH Inc.) as a template and two primers: primer 1, 5'-GTCGACATGG AGGGGGAGCC CTCCCAGCCT C-3' (SEQ ID NO:57) and primer 2, 5'-ACTAGTTCAG ATATCCCAGG CTGGAATGG-3' (SEQ ID NO :58), PCR was carried out. The reaction solution in the above reaction comprised of 1/10 volume of the cDNA, which was used as a template, 1/50 volume of Advantage cDNA Polymerase Mix (CLONTECH

Inc.), 0.2 μM of primer 1 (SEQ ID NO:57), 0.2 μM of primer 2 (SEQ ID NO:58) 200 μM dNTPs, 4% dimethylsulfoxide and a buffer attached to the enzyme to make the final volume 25 μl. The PCR was carried out by (1) a cycle of 94°C for 2 minutes, (2) then a cycle set to include 94°C for 20 seconds followed by 72°C for 1 minute and 30 seconds, which was repeated 3 times, (3) a cycle set to include 94°C for 20 seconds followed by 67°C for 1 minute and 30 seconds, which was repeated 3 times, (4) a cycle set to include 94°C for 20 seconds followed by 62°C for 20 seconds and 68°C for 1 minute and 30 seconds, which was repeated 38 times, and finally extension reaction at 68°C for 7 minutes. After completion of the PCR reaction, the reaction product was subcloned to plasmid vector pCR2.1 (Invitrogen Inc.) following the instructions attached to the TA cloning kit (Invitrogen Inc.), which was then introduced into *Escherichia coli* DH5α, and the clones carrying the cDNA were selected in an LB agar medium containing ampicillin. The sequence of each clone was analyzed to give the cDNA sequences (SEQ ID NO:55 and SEQ ID NO:56) encoding the novel G protein-coupled receptor protein. The two sequences are different by one base in the 597th residue but the deduced amino acid sequences are the same (SEQ ID NO:57). Novel G protein-coupled receptor protein containing the amino acid sequence was designated hOT7T022. The two transformants were named *Escherichia coli* DH5α/pCR2.1-hOT022T (containing cDNA shown by SEQ ID NO:55) and *Escherichia coli* DH5α/pCR2.1-hOT022G (containing cDNA shown by SEQ ID NO:56).

REFERENCE EXAMPLE 12

Preparation of anti-rat RFRP-1 monoclonal antibody

A monoclonal antibody was prepared using a peptide added with one Cys residue to the C-terminal 12 amino acids (the C-terminal carboxyl group has been amidated: the 83 (Val) - 94 (Phe) amino acid sequence in the amino acid sequence shown by SEQ ID NO:50) at the N-terminus (C-VPHSAANLPLRF-$NH_2$) of rat type RFRP-1 as an antigen. Using maleimide, 0.6 mg of an antigen peptide was conjugated to bovine serum albumin (BSA). After 100 μg of the conjugate was subcutaneously injected in mouse 3 times for immunization, 50 μg of the conjugate was injected for booster immunization via the tail vein. On 4 days following the final immunization, mouse splenocytes were collected and fused with mouse myeloma cells (P3-X63Ag8-U1: Matsumoto et al., BBRC (1999), vol. 257, 264-268) using polyethylene glycol. Following the cell fusion, hybridoma cell 1F3 was selected and mass culture was conducted using INTREGRA CL-1000 to give the supernatant of 1F3. From the culture supernatant, anti-rat REAP- 1 monoclonal antibody was obtained using HiTrap rProtein A column (Pharmacia). Detection with Mouse mAb isotyping kit (Amersham) revealed that the subtype of this monoclonal antibody was IgG1 κ chain.

REFERENCE EXAMPLE 13

Construction of competitive EIA

First, the peptide used as an antigen in REFERENCE EXAMPLE 12 was conjugated with horse radish peroxidase (HRP) using maleimide to prepare HRP-rat RFRP-1. Using this HRP-rat RFRP-1 and anti-rat RFRP-1 monoclonal antibody prepared in REFERENCE EXAMPLE 12, competitive EIA was constructed.

To each well of a 96-well plate coated with 1.5 μg/well of anti-mouse IgGAM (Cappel) and blocked with Block ACE (Dai-Nippon Pharmaceutical Co., Ltd.), 50 μl of anti-rat RFRP-1 monoclonal antibody, which had been diluted with buffer (phosphate buffered saline (PBS) containing 2 mM EDTA, 0.4% BSA, 0.1M NaCl and 0.1% micro-O-protect), was added and 50 ml of a sample dissolved in the same buffer was also added to the plate. After incubation at 4°C for 16 hours, 50 μl of anti-rat RFRP-1 monoclonal antibody diluted with the buffer was added to each well, followed by incubation at room temperature for 2 hours. After the plate was washed with PBS containing 0.1% Tween 20 (Sigma), the activity of HRP bound to each well was detected via color-forming reaction using TMB microwell peroxidase substrate system (Kirkegaard & Perry Labs), and absorbance was assayed at 450 nm. Change in absorbance when the RFRP-1-related peptide was added as the sample is shown in FIG. 11.

EXAMPLE 1

Effects of ligand polypeptide on pituitary hormone level in plasma

The effect of the peptide shown by SEQ ID NO:39 administered into the third ventricle on pituitary hormone level in plasma was explored. Mature male Wistar rats (body weights at operation: about 290-350 g) were anesthetized with 50 mg/kg of pentobarbital, i.p., and each rat was immobilized in a rat brain stereotaxic apparatus. The incisor bar was set 3.3 mm lower from the interaural line. The skull was exposed, and using a dental drill a hole was made on the bone for implantation of a guide cannula. In addition, an anchor screw was buried in one position around the hole. A stainless-steel guide cannula, AG-12 (0.4 mm inner diameter, 0.5 mm outer diameter, EICOM Co., Ltd.), was inserted in such a manner that its tip would be situated in the upper part of the third ventricle. Following the atlas of Paxinos and Watson (1986), the stereotaxic coordinates were set to AP: +7.2 mm (from the interaural line), L: 0.0 mm, and H: +2.0 mm (from the interaural line). The guide cannula was secured to the skull using an instant adhesive, a dental cement and an anchor screw. A stainless-steel dummy cannula, AD-12 (0.35 mm outer diameter, EICOM Co., Ltd.), was then passed through the guide cannula and locked in position with a cap nut (EICOM Co., Ltd.). After the operation the rats were housed in individual cages and kept for at least a week for postoperative recuperation before starting the experiment.

The operated rat was anesthetized with 50 mg/kg of pentobarbital, i.p., and immobilized in the dorsal position on a necropsy pad. A catheter (SP35, Natsume Seisakusho Co., Ltd.) was inserted into the right jugular vein. On the following day, 400 μl of blood was drawn through the jugular vein catheter. To prevent clotting, a syringe was filled beforehand with 20 μl of saline containing 200 U/ml of heparin. The cap nut and dummy cannula were removed from the rat skull and instead, a stainless steel microinjection cannula AMI13 (0.17 mm inner diameter, 0.35 mm outer diameter, EICOM Co., Ltd.) connected to a Teflon tube (50 cm long, 0.1 mm inner diameter, 0.4 mm outer diameter, EICOM Co., Ltd.) was inserted. The length of the microinjection cannula was adjusted beforehand so that its tip would be exposed from the guide cannula by 1 mm. One end of the Teflon tube was connected to a microsyringe pump and either PBS or the peptide shown by SEQ ID NO:39 dissolved in PBS was injected, in a total volume of 10 μl, into the third ventricle at a flow rate of 5 μl/min. After a 1 minute standby time following the infusion, the microinjection cannula was disconnected and the dummy cannula was locked in position again with a cap nut. Immediately before the initiation of intraventricular administration and 10, 20, 30, 40, and 60 minutes after the initiation of administration, 400 μl aliquots of blood were collected via the cannula inserted into the jugular vein. Each blood sample collected was centrifuged (5,000 rpm, 10 min.) with a high-speed refrigerated microcentrifuge (MR-150, Tommy Seiko) and the supernatant (plasma) was recovered. The amount of prolactin in the plasma was determined by radioimmunoassay.

The results were shown in terms of a mean ± S.E.M. To examine if there was a significant difference between the group administered with the peptide shown by SEQ ID NO:39 dissolved in PBS and the control group administered with PBS alone, Student's t-test was used. According to the two-tailed test, the risk percentage of 5% or less was assumed to be statistically significant. As shown in FIG. 10, the plasma prolactin level tended to increase at 10 minutes after the administration of 10 nmols of the peptide shown by SEQ ID NO:39 into the third ventricle and significantly increased at 20, 30 and 40 minutes. Even at 60 minutes after the administration, a significant difference was noted, as compared to the control group. As to the levels of GH, LH, ACTH, and TSH in plasma, none showed any significant change.

EXAMPLE 2

Purification endogenous RGRP-1 from bovine hypothalamus

An RFRP-1-like immune activity was detected in crude fraction of the peptide from bovine hypothalamus by the competitive EIA constructed in REFERENCE EXAMPLE 13. Using this RFRP-1-like immune activity as an indicator, endogenous RFRP-1 was purified from bovine hypothalamus.

First, 2.0 kg of frozen bovine hypothalamus was ground and boiled in 8.0 L of ultra pure water (milliQ water), acetic acid was added at a concentration of 1 M and the mixture was homogenized using a Polytron. Trifluoroacetic acid (TFA) was added to the supernatant at a concentration of 0.05%. The mixture was then applied to reversed phase C 18 column (Prep C18 125 angstrom; Waters). The peptide bound to the column was stepwise eluted with 10, 30 and 50% acetonitrile containing 0.5% TFA. The 30% acetonitrile fraction was diluted with 2-fold volume of 20 mM ammonium acetate (pH 4.7), and the dilution was applied to ion exchange column HiPrep CM-Sepharose FF (Pharmacia). The peptide bound to the ion exchange column was stepwise eluted with 0.1, 0.2, 0.5 and 1.0 M NaCl in 20 mM ammonium acetate (pH 4.7) containing 10% acetonitrile. Then, 3-fold volume of chilled acetone was added to the 0.1 M NaCl fraction with the most abundant RFRP-1-like immune activity, and the precipitates were removed by centrifugation. The supernatant was concentrated in an evaporator. To the concentrated fraction was added TFA in a concentration of 0.1%, and the mixture was applied to reversed phase HPLC column RESOURCE RPC (Pharmacia) for further fractionation. The fractionation of RESOURCE RPC was carried out on a concentration gradient of 10-30% acetonitrile, in which the main RFRP-1-like activity was detected in the fraction of about 22% acetonitrile. The active fraction was separated by cation exchange column TSK gel CM-SW (Toso) using a concentration gradient of 0.2-0.6 M NaCl in 20 mM ammonium acetate (pH 4.7) containing 10% acetonitrile. The main RFRP-1-like activity was detected in the fraction of about 0.3 M NaCl. TFA was added to the fraction of CM-2SW column containing the RFRP-1-like immune activity in a concentration of 0.1%, and the mixture was applied to reversed phase column diphenyl 219TP5415 (Vydac) for further fractionation. By fractionation on a concentration gradient of 21-25% acetonitrile, the RFRP-1-like immune activity was eluted with 23% acetonitrile. The fraction containing this RFRP-1-like immune activity was subjected to final purification on reversed phase column μRPC C2/C18 SC 2.1/10, using a concentration gradient of 22-23% acetonitrile. Thus, a single peak coincident with the RFRP-1-like immune activity was detected (FIG. 12).

EXAMPLE 3

N-Terminal amino acid sequencing of the finally purified product and determination of its molecular weight by mass spectrum

The N-terminal amino acids of the finally purified product obtained in EXAMPLE 2 were sequenced with a protein sequencer (model 491 cLC; Applied Biosystems). As a result, the amino acid sequence shown by S-L-T-F-E-E-V-K-D-X-A-P-K-I-K-M-N-K-P-V- (wherein X is an unidentified amino acid residue) was obtained.

Also, the molecular weight of the finally purified product using ESI-MS (Thermoquest) was found to be 3997.0.

These analytical results reveal that the finally purified product from bovine hypothalamus coincided with the peptide composed of 35 amino acids from 58 (Ser) to 92 (Phe) in amino acid sequence shown by SEQ ID NO:14.

EXAMPLE 4

Preparation of structural gene of a peptide having the amino acid sequence shown by SEQ ID NO:1, wherein the C-terminal carboxyl group has been amidated (hereinafter sometimes referred to as hRFRP-1(37))

Using 4 DNA fragments (#1: SEQ ID NO: 15; #4: SEQ ID NO: 18; #1: SEQ ID NO:18; manufactured by Kikotech Co., Ltd.) (#2: SEQ ID NO:16; #3: SEQ ID NO:17; manufactured by Amersham Pharmacia Biotech, Inc.) shown by SEQ ID NO:59 through SEQ ID NO:62, the structural gene of hRFRP-1(37) was prepared by a publicly known method.

a) Phosphorylation of DNA oligomers

In 100 μL of a reaction solution for phosphorylation [50 mM Tris-HCl (pH 7.6), 10 mM $MgCl_2$, 1 mM spermidine, 10 mM dithiothreitol, 0.1 mg/mL bovine serum albumin, 1 mM ATP, 10 U T4 polynucleotide kinase (Nippon Gene)], 1 μg each of the two oligomers, except for #1 and #4 supposed to form the 5' ends, were reacted at 37°C for 1 hour to phosphorylate the 5'-ends. After phenol treatment, the aqueous phase was recovered and 2-fold volume of ethanol was added thereto. Then, the mixture was cooled to -70°C and centrifuged to precipitate DNA.

b) Ligation of DNA fragments

The phosphorylated DNA fragments obtained in a) above and 1 μg each of #1 and #4 were combined and added to 10 mM Tris/HCl, 2 mM EDTA (pH 8.0) to make a total volume of 120 μL. The mixture was kept at 80°C for 10 minutes and then gradually cooled to room temperature for annealing. Using TaKaRa DNA Ligation Kit ver. 2 (Takara Shuzo), ligation was carried out. To 30 μL of the annealing solution, 30 μL of Solution II supplied with the kit was added. After thoroughly mixing them, 60 μL of Solution I supplied with the kit was added and the mixture was reacted at 37°C for 1 hour to effect ligation. After phenol treatment, the aqueous phase was recovered and 2-fold volume of ethanol was added thereto. Then, the mixture was cooled to -70°C and centrifuged to precipitate DNA.

c) Phosphorylation of the 5' ends

The precipitate was dissolved in 10 μL of TE buffer (10 mM Tris-HCl (pH 8.0), 1 mM EDTA), and the solution was reacted at 37°C for 1 hour in 100 μL of a reaction solution for phosphorylation [50 mM Tris-HCl (pH 7.6), 10 mM $MgCl_2$, 1 mM spermidine, 10 mM dithiothreitol, 0.1 mg/mL bovine serum albumin, 1 mM ATP, 10 U T4 polynucleotide kinase (Nippon Gene)] to phosphorylate the 5' ends. After phenol treatment, the aqueous phase was recovered and 2-fold volume of ethanol was added thereto. Then, the mixture was cooled to -70°C and centrifuged to precipitate DNA.

EXAMPLE 5

Preparation of hRFEP-1(37) expression plasmid

pTFC (described in JPA 2000-270871) as the expression vector was digested with NdeI and AvaI (Takara Shuzo Co., Ltd.) at 37°C for 4 hours and electrophoresed on a 1% agarose gel. A 4.4 kb DNA fragment was extracted using QIAquick Gel Extraction Kit (Qiagen) and dissolved in 25 μL of TE buffer. The NedI and AvaI fragment of this pTFC and the structural gene of hRFRP-1(37) prepared as above were ligated using TaKaRa DNA Ligation Kit ver. 2 (Takara Shuzo). Using a 10 µl aliquot of the reaction solution, E. coli JM 109 competent cells (Toyobo Co., Ltd.) were transformed and the transformants were seeded on an LB agar medium containing 10 µg/ml of tetracycline followed by incubation at 37°C overnight. The tetracycline-resistant colony formed was selected. This transformant was incubated overnight in LB medium, and plasmid pTFCRFRP-1 was prepared using QIAprep8 Miniprep Kit (Qiagen). The base sequence of this pTFCRFRP-1 structural gene part was verified using Model 377 DNA sequencer of Applied Systems, Inc. E. coli MM294 (DE3) was transformed by plasmid pTFCRFRP-1 to give RFRP-1-CS23-fused protein expressed Escherichia coli MM294 (DE3)/pTFCRFRP-1 (FIG. 13).

EXAMPLE 6

The transformant obtained in EXAMPLE 5 was subjected to shaking culture at 37°C for 8 hours in a 2 liter volume of flask charged with 1 L of LB medium containing 5.0 mg/L of tetracycline (1% peptone, 0.5% yeast extract, 0.5% sodium chloride). The culture broth obtained was transferred to a fermentation tank of 50 L volume charged with 19 liters of the main fermentation medium (1.68% sodium monohydrogenphosphate, 0.3% potassium dihydrogenphosphate, 0.1% ammonium chloride, 0.05% sodium chloride, 0.05% magnesium sulfate, 0.02% defoaming agent, 0.00025% ferrous sulfate, 0.00025% thiamine hydrochloride, 1.5% glucose, 1.5% Casamino acid) to initiate aeration spinner culture at 30°C. At the point of time when the turbidity showed a Klett value of about 500 was obtained, isopropyl-β-D-thiogalactopyranoside was added at a final concentration of 12 mg/L followed by incubation for additional 4 hours. After the incubation, the culture was centrifuged to give about 500 g of the wet cells. The cells were frozen and stored at -80°C.

EXAMPLE 7

Acquisition of hRFRP-1 (37)

To 500 g of the cells obtained in EXAMPLE 6 was added 1000 ml of 0.2 mM Tris/HCl (pH 8.0) solution, and the mixture was agitated for about 4 hours followed by centrifugation (10000 rpm, 60 minutes). The supernatant was diluted with 29 liters of 50 mM Tris/HCl (pH 8.0) supplemented with 0.6 M arginine and 1 mM dithiothreitol. After allowing to stand at 10°C overnight, the pH was adjusted to 6.0 with conc. hydrochloric acid. The mixture was passed through an AF-Heparin Toyopearl 650M column (11.3 cm ID x 13 cmL, Toso) equilibrated with 50 mM phosphate buffer (pH 6.0) for adsorption. After the column was rinsed, elution was carried out with 50 mM phosphate buffer, 2M NaCl, pH 6.0 to recover 1000 ml of the polypeptide of the present invention hRFRP-1(37)-CS23 fusion protein-containing eluate.

This eluate was concentrated using Pellicon Mini Cassette (Millipore Corp.) with constant addition of 0.1 M acetic acid to give a solution of hRFRP-1 (37)-CS23 fusion protein in 0.1 M acetic acid. Urea was added to this solution at a final concentration of 6 M, followed by addition of 445 mg of 1-cyano-4-dimethylaminopyridinium (DMAP-CN), and the reaction was carried out at room temperature for 15 minutes. After completion of the reaction, the reaction mixture was applied onto a Sephadex G-25 column (46 mm ID x 600 mmL, Pharmacia) equilibrated with 10% acetic acid and elution was carried out with the same 10% acetic acid as used for the equilibration at a flow rate of 6 ml/min to give an S-cyanated hRFRP-1(37)-CS23 fusion protein fraction. This eluate was concentrated and desalted using Pellicon Mini Cassette (Millipore Corp.) to give a desalted solution of hRFRP-1(37)-CS23 fusion protein. Urea was added to this desalted solution urea at a final concentration of 6 M, followed by further addition of 25% aqueous ammonia at a final concentration of 3 M. The reaction was carried out at room temperature for 15 minutes. After completion of the reaction, the reaction mixture was adjusted to pH 6.0 with acetic acid to give hRFRP-1(37). This reaction mixture was applied onto an SP-5PW column (5.5 mm ID x 30 mmL, Toso) equilibrated with 50 mM phosphate buffer (pH 6.5) containing 3 M urea for adsorption. After the column was rinsed, elution was carried out on a gradient of 0 to 50% B (B = 50 mM MES buffer + 1 M NaCl + 3 M urea) to recover hRFRP-1(35). This hRFRP-1(37) fraction was further applied onto an ODS-120T column (21.5 mm ID x 300 mmL, Toso) equilibrated with 0.1% trifluoroacetic acid (TFA) for adsorption. After the column was rinsed, elution was carried out a gradient concentration of 30 to 60% B (B = 80% acetonitrile/0.1% TFA). The resulting hRFRP-1(37) fractions were pooled (elution time: 45 minutes) and lyophilized to give about lyophilized powders of hRFEP-1(37).

EXAMPLE 8

Comparison in the agonist activity between various RF amide peptides on human 0T7T022 receptor-expressing CHO cells

Human 0T7T022 receptor-expressing CHO cells obtained by a modification of the method described in WO 00/29441 were inoculated on a 24-well plate in a concentration of 3 x $10^5$ cells/well. After incubation overnight, the cells were rinsed with Hanks' buffer (HBSS) supplemented with 0.05% BSA and 0.2 mM IBMX, and then preincubated at 37°C for 30 minutes in the same buffer. Next, the buffer was exchanged by Hanks' buffer (HBSS) supplemented with 0.05% BSA and 0.2 mM IBMX, or HBSS further added with 1 μM forskolin only, or HBSS added with 1 μM forskolin and peptide of various concentrations, followed by incubation at 37°C for 30 minutes. After completion of the incubation, cAMP in the cells of each well was extracted and quantified according to the method of cAMP EIA Kit (Amersham Inc.). An inhibition rate of the increased cAMP level in the cells by the forskolin treatment was calculated with the respective concentrations of the peptide. The dose-response curve as shown in FIG. 14 was obtained. The $ED_{50}$ levels of the peptides were hRFRP-1-12 (peptide having the 81 (Met) to 92 (Phe) amino acid sequence in SEQ ID NO:1 (o)) (4.5 nM); hRFRP-1-37 (peptide having the 56 (Ser) to 92 (Phe) amino acid sequence in SEQ ID NO:1 (■)) (21 nM); rRFRP-1-37 (peptide having the 58 (Ser) to 94 (Phe) amino acid sequence in SEQ ID NO:50 (◊)) (30 nM); hRFRP-2-12 (peptide having the 101 (Phe) to 112 (Ser) amino acid sequence in SEQ ID NO:1 (▲)); hRFRP-3-8 (peptide having the 124 (Val) to 131 (Phe) amino acid sequence in SEQ ID NO:1 (□)) (9.9 nM); PQRFamide (peptide shown by Pro-Gln-Arg-Phe-$NH_2$ (♦)) (1000 nM or more); LPLRFamide (peptide shown by Leu-Pro-Leu-Arg-Phe-$NH_2$ (•)) (36 nM); and NPFF (peptide shown by Asn-Pro-Phe-Phe (Δ)) (140 nM), respectively.

EXAMPLE 9

Study of the effects of pertussis toxin by RFRP peptide on the activation of human 0T7T022 receptor

Human 0T7T022 receptor-expressing CHO cells obtained in EXAMPLE 8 were inoculated on a 24-well plate in a concentration of 1 x $10^5$ cells/well. After culturing overnight, the medium was exchanged by a medium added with 100 ng/ml pertussis toxin (Sigma, Inc.) or with a control medium followed by further incubation overnight. The cells were rinsed with Hanks' buffer (HBSS) supplemented with 0.05% BSA and 0.2 mM IBMX and then preincubated at 37°C for 30 minutes in the same buffer. Next, the cells were incubated at 37°C for 30 minutes after the buffer was exchanged by Hanks' buffer alone (HBSS) supplemented with 0.05% BSA and 0.2 mM IBMX (black column), or by HBSS further added with 1 μM forskolin only (white column), or by HBSS added with 1 μM forskolin and 0.1 μM RFRP-1-12 (peptide having the 81 (Met) to 92 (Phe) amino acid sequence in SEQ ID NO:1) (hatched column). After completion of the incubation, cAMP in the cells of each well was extracted and quantified according to the method of cAMP EIA Kit (Amersham, Inc.). As shown in FIG. 15, since the cAMP production inhibiting activity by RFRP-1-12 was lost in the cells treated with pertussis toxin, it was demonstrated that the 0T7T022 receptor-mediated cAMP production inhibiting activity is conjugated to pertussis toxin-sensitive G protein α subunit, Gi (suppressive) or Go.

INDUSTRIAL APPLICABILITY

The polypeptide of the present invention exhibits the activities of promoting and inhibiting the secretion of prolactin. That is, as is evident from EXAMPLES described above, the polypeptide of the present invention has prolactin secretion-stimulating activity and thus finds application as a prophylactic and therapeutic drug for various diseases associated with prolactin hyposecretion. On the other hand, the polypeptide of the present invention has a high affinity to the receptor proteins, and therefore, when used in an increased dose, causes desensitization for prolactin secretion so that the polypeptide also exhibits the prolactin secretion-inhibiting activity. In this sense, the polypeptide can be used as a prophylactic and therapeutic drug for various diseases associated with prolactin hypersecretion.

Thus, the polypeptide of the present invention as the prolactin secretion stimulant is useful as a prophylactic and therapeutic drug for various diseases associated with prolactin secretion, such as hypoovarianism, spermatic underdevelopment, osteoporosis, menopausal symptoms, agalactosis, hypothyroidism, renal insufficiency, etc.

Moreover, the polypeptide of the present invention as the prolactin secretion inhibitor is useful as a prophylactic and therapeutic drug for various diseases associated with prolactin secretion, such as hyperprolactinemia, pituitary tumor, diencephalon tumor, menstrual disorder, stress, autoimmune diseases, prolactinoma, sterility, impotence, amenorrhea, lactorrhea, acromegaly, Chiari-Frommel syndrome, Argonz-del Castilo syndrome, Forbes-Albright syndrome, breast cancer lymphoma or Sheehan's syndrome, or spermatogenesis disorder, etc.

In particular, the polypeptide of the present invention is useful as a prophylactic and therapeutic drug for hyperprolactinemia, agalactosis, autoimmune diseases and breast cancer.

In addition, the polypeptide of the present invention is useful not only as a testing agent to examine the function of prolactin secretion but also as a veterinary drug such as a lactation stimulant for livestock mammals including bovine, goat, swine, etc. Furthermore, an application of the polypeptide to the production of useful substances is also expected, which involves producing a useful substance in the body of livestock mammals and secreting the substance into milk, or the like.

## Claims

1. A prolactin secretion regulatory agent comprising a polypeptide containing the same or substantially the same amino acid sequence as the amino acid sequence represented by SEQ ID NO:1, its amide or ester, or a salt thereof.

2. The agent according to claim 1, wherein substantially the same amino acid sequence is represented by SEQ ID NO:8, SEQ ID NO:14, SEQ ID NO:18, SEQ ID NO:33 or SEQ ID NO:50.

3. A prolactin secretion regulatory agent comprising a partial peptide of a polypeptide containing the same or substantially the same amino acid sequence as the amino acid sequence represented by SEQ ID NO:1, its amide or ester, or a salt thereof.

4. The prolactin secretion regulatory agent according to claim 3, comprising a partial peptide composed of acid residues 81 (Met) to 92 (Phe) of SEQ ID NO:1, its amide or ester, or a salt thereof.

5. The prolactin secretion regulatory agent according to claim 3, comprising a partial peptide composed of amino acid residues 101 (Ser) to 112 (Ser) of SEQ ID NO:1, its amide or ester, or a salt thereof.

6. The prolactin secretion regulatory agent according to claim 3, comprising a partial peptide composed of amino acid residues 124 (Val) to 131 (Phe) of SEQ ID NO: 1, its amide or ester, or a salt thereof.

**7.** The prolactin secretion regulatory agent according to claim 3, comprising a partial peptide composed of amino acid residues 56 (Ser) to 92 (Phe) of SEQ ID NO: 1, its amide or ester, or a salt thereof.

**8.** The prolactin secretion regulatory agent according to claim 3, comprising an amide of a partial peptide of a polypeptide containing the same or substantially the same amino acid sequence as the amino acid sequence represented by SEQ ID NO: 1, or a salt thereof.

**9.** The prolactin secretion regulatory agent according to claim 8, comprising a partial peptide of a polypeptide containing the same or substantially the same amino acid sequence as the amino acid sequence represented by SEQ ID NO:1, the C-terminal carboxyl of which is amidated, or a salt thereof.

**10.** The prolactin secretion regulatory agent according to claim 1 or 3, which is a prolactin secretion stimulant.

**11.** The prolactin secretion regulatory agent according to claim 1 or 3, which is a prolactin secretion inhibitor.

**12.** The prolactin secretion stimulant according to claim 10, which is a medicament for the prevention or treatment of hypoovarianism, spermatic underdevelopment, osteoporosis, menopausal symptoms, agalactosis, hypothyroidism or renal insufficiency.

**13.** The a prolactin secretion inhibitor according to claim 11, which is a medicament for the prevention or treatment of hyperprolactinemia, pituitary tumor, diencephalon tumor, menstrual disorder, stress, autoimmune diseases, prolactinoma, sterility, impotence, amenorrhea, lactorrhea, acromegaly, Chiari-Frommel syndrome, Argonz-del Castilo syndrome, Forbes-Albright syndrome, breast cancer lymphoma or Sheehan's syndrome, or spermatogenesis disorder.

**14.** The prolactin secretion regulatory agent according to claim 1 or 3, which is a galactosis stimulant for livestock mammal.

**15.** The prolactin secretion regulatory agent according to claim 1 or 3, which is a test agent for prolactin secretion function.

**16.** A prolactin secretion regulatory agent comprising a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, which is obtainable using:

a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof; or,

a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof.

**17.** A prolactin secretion regulatory agent comprising a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, which is obtainable using:

(I) a method of screening a compound or its salt that promotes or inhibits the activity of (i) a polypeptide containing the same or substantially the same amino acid sequence as the amino acid sequence represented by SEQ ID NO: 1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof; or,
(II) a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, and (iii) a protein containing the same or substantially the same amino acid sequence represented by SEQ ID NO:37 or a salt thereof, or its partial peptide, its amide or ester, or a salt thereof.

**18.** (1) A peptide containing an amino acid sequence composed of 81(Met) to 92 (Phe) of SEQ ID NO:1, its amide or ester, or a salt thereof, (2) a peptide containing an amino acid sequence composed of 101(Ser) to 112 (Ser) of SEQ ID NO:1, its amide or ester, or a salt thereof, (3) a peptide containing an amino acid sequence composed of 124(Val) to 131 (Phe) of SEQ ID NO:1, its amide or ester, or a salt thereof, (4) a peptide containing an amino acid sequence composed of 56 (Ser) to 92 (Phe) of SEQ ID NO:1, its amide or ester, or a salt thereof, (5) a peptide containing an amino acid sequence composed of 81 (Met) to 92 (Phe) of SEQ ID NO:14, its amide or ester, or a salt thereof, (6) a peptide containing an amino acid sequence composed of 101 (Ser) to 112 (Leu) of SEQ ID NO: 14, its amide or ester, or a salt thereof, (7) a peptide containing an amino acid sequence composed of 58 (Ser) to 92 (Phe) of SEQ ID NO:14, its amide or ester, or a salt thereof, (8) a peptide containing an amino acid sequence composed of 83 (Val) to 94 (Phe) of SEQ ID NO:33, its amide or ester, or a salt thereof, (9) a peptide containing an amino acid sequence composed of 118 (Phe) to 125 (Phe) of SEQ ID NO:33, its amide or ester, or a salt thereof, (10) a peptide containing an amino acid sequence composed of 58 (Ser) to 94 (Phe) of SEQ ID NO:33, its amide or ester, or a salt thereof, or (11) a peptide containing an amino acid sequence composed of 58 (Ser) to 94 (Phe) of SEQ ID NO:50, its amide or ester, or a salt thereof.

**19.** An amide of the peptide according to claim 18, or a salt thereof.

**20.** The peptide according to claim 18 wherein the C-terminal carboxyl is amidated, its amide or ester, or a salt thereof.

**21.** A DNA encoding the peptide according to claim 18.

**22.** The DNA containing (1) a 241-276 base sequence of SEQ ID NO:2, (2) a 301-336 base sequence of SEQ ID NO: 2, (3) a 370-393 base sequence of SEQ ID NO:2, (4) a 166-276 base sequence of SEQ ID NO:2, (5) a 241-276 base sequence of SEQ ID NO:15, (6) a 301-336 base sequence of SEQ ID NO:15, (7) a 172-276 base sequence of SEQ ID NO:15, (8) a 247-282 base sequence of SEQ ID NO:34, (9) a 352-375 base sequence of SEQ ID NO: 34, (10) a 172-282 base sequence of SEQ ID NO:34, or (11) a 172-282 base sequence of SEQ ID NO:51.

**23.** An antibody to the peptide according to claim 18, or its amide or ester, or a salt thereof.

**24.** A diagnostic comprising the DNA according to claim 21 or the antibody according to claim 23.

**25.** An antisense DNA having a base sequence complementary or substantially complementary to the DNA according to claim 21 and capable of inhibiting expression of the DNA.

**26.** An agent comprising the peptide according to claim 18, or its amide or ester, or a salt thereof.

**27.** A pharmaceutical composition comprising the peptide according to claim 18, or its amide or ester, or a salt thereof.

**28.** The pharmaceutical composition according to claim 27, which is a prolactin secretion regulatory agent.

**29.** A method of screening a compound or its salt that promotes or inhibits the activity of the peptide according to claim 18, or its amide or ester, or a salt thereof, which comprises using the peptide according to claim 18, or its amide or ester, or a salt thereof.

**30.** The method of screening according to claim 29, wherein a protein containing the same or substantially the same amino acid sequence represented by SEQ ID NO:37 or a salt thereof, or its partial peptide, its amide or ester, or a salt thereof is further used.

**31.** A kit for screening a compound or its salt that promotes or inhibits the activity of the peptide according to claim 18, or its amide or ester, or a salt thereof, which comprises using the peptide according to claim 18, or its amide or ester, or a salt thereof.

**32.** A compound or its salt that promotes or inhibits the activity of the peptide according to claim 18, or its amide or ester, or a salt thereof, which is obtainable using the screening method according to claim 29 or the screening kit according to claim 31.

**33.** Use of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof, for the manufacture of a medicament having a prolactin secretion regulating activity.

**34.** A method of regulating the secretion of prolactin, which comprises administering to a mammal (i) a polypeptide containing the same or substantially the same amino acid sequence as the amino acid sequence represented by SEQ ID NO: I, its amide or ester, or a salt thereof, or (ii) a partial peptide of a polypeptide containing the same or substantially the same amino acid sequence as the amino acid sequence represented by SEQ ID NO:1, its amide or ester, or a salt thereof.

**35.** Use of a compound or its salt that promotes or inhibits the activity of (i) a polypeptide containing the same or substantially the same amino acid sequence as the amino acid sequence represented by SEQ ID NO: 1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; or, a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof.

**36.** Use of a compound or its salt that that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

(I) a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof; or,
(II) a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO: 1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof.

**37.** A method of regulating the secretion of prolactin, which comprises administering to a mammal a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof. or,
a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof.

**38.** A method of regulating the secretion of prolactin, which comprises administering to a mammal a compound or its salt that that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, for the manufacture of a medicament having a prolactin secretion regulating activity, which is obtainable using:

(I) a method of screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, which comprises using (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof; or,

(II) a kit for screening a compound or its salt that promotes or inhibits the activity of (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof; the kit comprising (i) a polypeptide containing the same or substantially the same amino acid sequence as the amino acid sequence represented by SEQ ID NO:1, its amide or ester, or a salt thereof, or (ii) a partial peptide of a polypeptide containing the same or substantially the same amino acid sequence as the amino acid sequence represented by SEQ ID NO:1, its amide or ester, or a salt thereof, and (iii) a protein containing the same or substantially the same amino acid sequence represented by SEQ ID NO:37 or a salt thereof, or its partial peptide, its amide or ester, or a salt thereof.

# Fig.1

```
                 9           18          27          36          45          54
5'  ATG GAA ATT ATT TCA TCA AAA CTA TTC ATT TTA TTG ACT TTA GCC ACT TCA AGC
    --- --- --- --- --- --- --- --- --- --- --- --- --- --- --- --- --- ---
    Met Glu Ile Ile Ser Ser Lys Leu Phe Ile Leu Leu Thr Leu Ala Thr Ser Ser

                63          72          81          90          99         108
    TTG TTA ACA TCA AAC ATT TTT TGT GCA GAT GAA TTA GTG ATG TCC AAT CTT CAC
    --- --- --- --- --- --- --- --- --- --- --- --- --- --- --- --- --- ---
    Leu Leu Thr Ser Asn Ile Phe Cys Ala Asp Glu Leu Val Met Ser Asn Leu His

               117         126         135         144         153         162
    AGC AAA GAA AAT TAT GAC AAA TAT TCT GAG CCT AGA GGA TAC CCA AAA GGG GAA
    --- --- --- --- --- --- --- --- --- --- --- --- --- --- --- --- --- ---
    Ser Lys Glu Asn Tyr Asp Lys Tyr Ser Glu Pro Arg Gly Tyr Pro Lys Gly Glu

               171         180         189         198         207         216
    AGA AGC CTC AAT TTT GAG GAA TTA AAA GAT TGG GGA CCA AAA AAT GTT ATT AAG
    --- --- --- --- --- --- --- --- --- --- --- --- --- --- --- --- --- ---
    Arg Ser Leu Asn Phe Glu Glu Leu Lys Asp Trp Gly Pro Lys Asn Val Ile Lys

               225         234         243         252         261         270
    ATG AGT ACA CCT GCA GTC AAT AAA ATG CCA CAC TCC TTC GCC AAC TTG CCA TTG
    --- --- --- --- --- --- --- --- --- --- --- --- --- --- --- --- --- ---
    Met Ser Thr Pro Ala Val Asn Lys Met Pro His Ser Phe Ala Asn Leu Pro Leu

               279         288         297         306         315         324
    AGA TTT GGG AGG AAC GTT CAA GAA GAA AGA AGT GCT GGA GCA ACA GCC AAC CTG
    --- --- --- --- --- --- --- --- --- --- --- --- --- --- --- --- --- ---
    Arg Phe Gly Arg Asn Val Gln Glu Glu Arg Ser Ala Gly Ala Thr Ala Asn Leu

               333         342         351         360         369         378
    CCT CTG AGA TCT GGA AGA AAT ATG GAG GTG AGC CTC GTG AGA CGT GTT CCT AAC
    --- --- --- --- --- --- --- --- --- --- --- --- --- --- --- --- --- ---
    Pro Leu Arg Ser Gly Arg Asn Met Glu Val Ser Leu Val Arg Arg Val Pro Asn

               387         396         405         414         423         432
    CTG CCC CAA AGG TTT GGG AGA ACA ACA ACA GCC AAA AGT GTC TGC AGG ATG CTG
    --- --- --- --- --- --- --- --- --- --- --- --- --- --- --- --- --- ---
    Leu Pro Gln Arg Phe Gly Arg Thr Thr Thr Ala Lys Ser Val Cys Arg Met Leu

               441         450         459         468         477         486
    AGT GAT TTG TGT CAA GGA TCC ATG CAT TCA CCA TGT GCC AAT GAC TTA TTT TAC
    --- --- --- --- --- --- --- --- --- --- --- --- --- --- --- --- --- ---
    Ser Asp Leu Cys Gln Gly Ser Met His Ser Pro Cys Ala Asn Asp Leu Phe Tyr

               495         504         513         522         531         540
    TCC ATG ACC TGC CAG CAC CAA GAA ATC CAG AAT CCC GAT CAA AAA CAG TCA AGG
    --- --- --- --- --- --- --- --- --- --- --- --- --- --- --- --- --- ---
    Ser Met Thr Cys Gln His Gln Glu Ile Gln Asn Pro Asp Gln Lys Gln Ser Arg

    TAA 3'
    ---
    ***
```

Fig.2

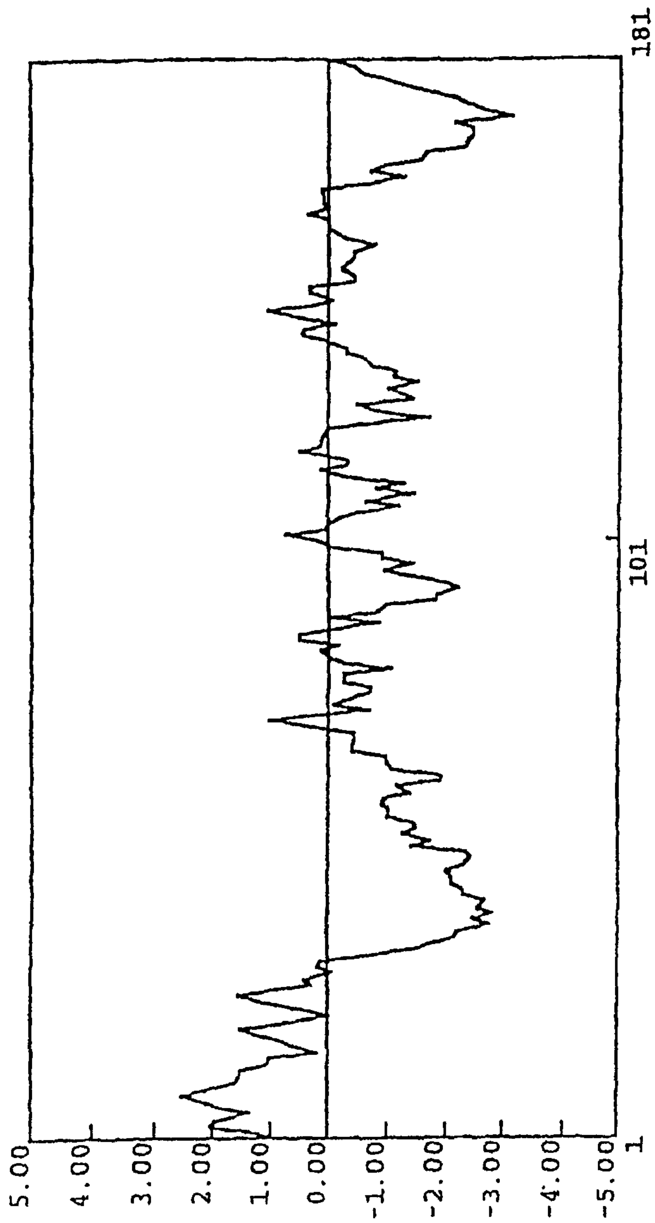

# Fig.3

```
                9           18          27          36          45          54
5'  ATG GAA ATT ATT TCA TCA AAA CTA TTC ATT TTA TTG ACT TTA GCC ACT TCA AGC
    --- --- --- --- --- --- --- --- --- --- --- --- --- --- --- --- --- ---
    Met Glu Ile Ile Ser Ser Lys Leu Phe Ile Leu Leu Thr Leu Ala Thr Ser Ser

               63          72          81          90          99         108
    TTG TTA ACA TCA AAC ATT TTT TGT GCA GAT GAA TTA GTG ATG TCC AAT CTT CAC
    --- --- --- --- --- --- --- --- --- --- --- --- --- --- --- --- --- ---
    Leu Leu Thr Ser Asn Ile Phe Cys Ala Asp Glu Leu Val Met Ser Asn Leu His

              117         126         135         144         153         162
    AGC AAA GAA AAT TAT GAC AAA TAT TCT GAG CCT AGA GGA TAC CCA AAA GGG GAA
    --- --- --- --- --- --- --- --- --- --- --- --- --- --- --- --- --- ---
    Ser Lys Glu Asn Tyr Asp Lys Tyr Ser Glu Pro Arg Gly Tyr Pro Lys Gly Glu

              171         180         189         198         207         216
    AGA AGC CTC AAT TTT GAG GAA TTA AAA GAT TGG GGA CCA AAA AAT GTT ATT AAG
    --- --- --- --- --- --- --- --- --- --- --- --- --- --- --- --- --- ---
    Arg Ser Leu Asn Phe Glu Glu Leu Lys Asp Trp Gly Pro Lys Asn Val Ile Lys

              225         234         243         252         261         270
    ATG AGT ACA CCT GCA GTC AAT AAA ATG CCA CAC TCC TTC GCC AAC TTG CCA TTG
    --- --- --- --- --- --- --- --- --- --- --- --- --- --- --- --- --- ---
    Met Ser Thr Pro Ala Val Asn Lys Met Pro His Ser Phe Ala Asn Leu Pro Leu

              279         288         297         306         315         324
    AGA TTT GGG AGG AAC GTT CAA GAA GAA AGA AGT GCT GGA GCA ACA GCC AAC CTG
    --- --- --- --- --- --- --- --- --- --- --- --- --- --- --- --- --- ---
    Arg Phe Gly Arg Asn Val Gln Glu Glu Arg Ser Ala Gly Ala Thr Ala Asn Leu

              333         342         351         360         369         378
    CCT CTG AGA TCT GGA AGA AAT ATG GAG GTG AGC CTC GTG AGA CGT GTT CCT AAC
    --- --- --- --- --- --- --- --- --- --- --- --- --- --- --- --- --- ---
    Pro Leu Arg Ser Gly Arg Asn Met Glu Val Ser Leu Val Arg Arg Val Pro Asn

              387         396         405         414         423         432
    CTG CCC CAA AGG TTT GGG AGA ACA ACA ACA GCC AAA AGT GTC TGC AGG ATG CTG
    --- --- --- --- --- --- --- --- --- --- --- --- --- --- --- --- --- ---
    Leu Pro Gln Arg Phe Gly Arg Thr Thr Thr Ala Lys Ser Val Cys Arg Met Leu

              441         450         459         468         477         486
    AGT GAT TTG TGT CAA GGA TCC ATG CAT TCA CCA TGT GCC AAT GAC TTA TTT TAC
    --- --- --- --- --- --- --- --- --- --- --- --- --- --- --- --- --- ---
    Ser Asp Leu Cys Gln Gly Ser Met His Ser Pro Cys Ala Asn Asp Leu Phe Tyr

              495         504         513         522         531         540
    TCC ATG ACC TGC CAG CAC CAA GAA ATC CAG AAT CCC GAT CAA AAA CAG TCA AGG
    --- --- --- --- --- --- --- --- --- --- --- --- --- --- --- --- --- ---
    Ser Met Thr Cys Gln His Gln Glu Ile Gln Asn Pro Asp Gln Lys Gln Ser Arg

              549         558         567         576         585
    AGA CTG CTA TTC AAG AAA ATA GAT GAT GCA GAA TTG AAA CAA GAA AAA TAA 3'
    --- --- --- --- --- --- --- --- --- --- --- --- --- --- --- --- ---
    Arg Leu Leu Phe Lys Lys Ile Asp Asp Ala Glu Leu Lys Gln Glu Lys ***
```

# Fig.4

```
             9           18          27          36          45          54
5' ATG GAA ATT ATT TCA TTA AAA CGA TTC ATT TTA TTG ATG TTA GCC ACT TCA AGC
   --- --- --- --- --- --- --- --- --- --- --- --- --- --- --- --- --- ---
   Met Glu Ile Ile Ser Leu Lys Arg Phe Ile Leu Leu Met Leu Ala Thr Ser Ser

            63           72          81          90          99         108
   TTG TTA ACA TCA AAC ATC TTC TGC ACA GAC GAA TCA AGG ATG CCC AAT CTT TAC
   --- --- --- --- --- --- --- --- --- --- --- --- --- --- --- --- --- ---
   Leu Leu Thr Ser Asn Ile Phe Cys Thr Asp Glu Ser Arg Met Pro Asn Leu Tyr

           117          126         135         144         153         162
   AGC AAA AAG AAT TAT GAC AAA TAT TCC GAG CCT AGA GGA GAT CTA GGC TGG GAG
   --- --- --- --- --- --- --- --- --- --- --- --- --- --- --- --- --- ---
   Ser Lys Lys Asn Tyr Asp Lys Tyr Ser Glu Pro Arg Gly Asp Leu Gly Trp Glu

           171          180         189         198         207         216
   AAA GAA AGA AGT CTT ACT TTT GAA GAA GTA AAA GAT TGG GCT CCA AAA ATT AAG
   --- --- --- --- --- --- --- --- --- --- --- --- --- --- --- --- --- ---
   Lys Glu Arg Ser Leu Thr Phe Glu Glu Val Lys Asp Trp Ala Pro Lys Ile Lys

           225          234         243         252         261         270
   ATG AAT AAA CCT GTA GTC AAC AAA ATG CCA CCT TCT GCA GCC AAC CTG CCA CTG
   --- --- --- --- --- --- --- --- --- --- --- --- --- --- --- --- --- ---
   Met Asn Lys Pro Val Val Asn Lys Met Pro Pro Ser Ala Ala Asn Leu Pro Leu

           279          288         297         306         315         324
   AGA TTT GGG AGG AAC ATG GAA GAA GAA AGG AGC ACT AGG GCG ATG GCC CAC CTG
   --- --- --- --- --- --- --- --- --- --- --- --- --- --- --- --- --- ---
   Arg Phe Gly Arg Asn Met Glu Glu Glu Arg Ser Thr Arg Ala Met Ala His Leu

           333          342         351         360         369         378
   CCT CTG AGA CTC GGA AAA AAT AGA GAG GAC AGC CTC TCC AGA TGG GTC CCA AAT
   --- --- --- --- --- --- --- --- --- --- --- --- --- --- --- --- --- ---
   Pro Leu Arg Leu Gly Lys Asn Arg Glu Asp Ser Leu Ser Arg Trp Val Pro Asn

           387          396         405         414         423         432
   CTG CCC CAG AGG TTT GGA AGA ACA ACA ACA GCC AAA AGC ATT ACC AAG ACC CTG
   --- --- --- --- --- --- --- --- --- --- --- --- --- --- --- --- --- ---
   Leu Pro Gln Arg Phe Gly Arg Thr Thr Thr Ala Lys Ser Ile Thr Lys Thr Leu

           441          450         459         468         477         486
   AGT AAT TTG CTC CAG CAG TCC ATG CAT TCA CCA TCT ACC AAT GGG CTA CTC TAC
   --- --- --- --- --- --- --- --- --- --- --- --- --- --- --- --- --- ---
   Ser Asn Leu Leu Gln Gln Ser Met His Ser Pro Ser Thr Asn Gly Leu Leu Tyr

           495          504         513         522         531         540
   TCC ATG GCC TGC CAG CCC CAA GAA ATC CAG AAT CCT GGT CAA AAG AAC CTA AGG
   --- --- --- --- --- --- --- --- --- --- --- --- --- --- --- --- --- ---
   Ser Met Ala Cys Gln Pro Gln Glu Ile Gln Asn Pro Gly Gln Lys Asn Leu Arg

           549          558         567         576         585
   AGA CGG GGA TTC CAG AAA ATA GAT GAT GCA GAA TTG AAA CAA GAA AAA TAA 3'
   --- --- --- --- --- --- --- --- --- --- --- --- --- --- --- --- ---
   Arg Arg Gly Phe Gln Lys Ile Asp Asp Ala Glu Leu Lys Gln Glu Lys ***
```

# Fig.5

```
          9           18          27          36          45          54
5' ATG GAA ATT ATT TCA TCA AAG CGA TTC ATT TTA TTG ACT TTA GCA ACT TCA AGC
   Met Glu Ile Ile Ser Ser Lys Arg Phe Ile Leu Leu Thr Leu Ala Thr Ser Ser
          63          72          81          90          99         108
   TTC TTA ACT TCA AAC ACC CTT TGT TCA GAT GAA TTA ATG ATG CCC CAT TTT CAC
   Phe Leu Thr Ser Asn Thr Leu Cys Ser Asp Glu Leu Met Met Pro His Phe His
         117         126         135         144         153         162
   AGC AAA GAA GGT TAT GGA AAA TAT TAC CAG GTG AGA GGA ATC CCA AAA GGG GTA
   Ser Lys Glu Gly Tyr Gly Lys Tyr Tyr Gln Leu Arg Gly Ile Pro Lys Gly Val
         171         180         189         198         207         216
   AAG GAA AGA AGT GTG ACT TTT CAA GAA CTC AAA GAT TGG GGG GCA AAG AAA GAT
   Lys Glu Arg Ser Val Thr Phe Gln Glu Leu Lys Asp Trp Gly Ala Lys Lys Asp
         225         234         243         252         261         270
   ATT AAG ATG AGT CCA GCC CCT GCC AAC AAA GTG CCC CAC TCA GCA GCC AAC CTT
   Ile Lys Met Ser Pro Ala Pro Ala Asn Lys Val Pro His Ser Ala Ala Asn Leu
         279         288         297         306         315         324
   CCC CTG AGG TTT GGG AGG AAC ATA GAA GAC AGA AGA AGC CCC AGG GCA CGG GCC
   Pro Leu Arg Phe Gly Arg Asn Ile Glu Asp Arg Arg Ser Pro Arg Ala Arg Ala
         333         342         351         360         369         378
   AAC ATG GAG GCA GGG ACC ATG AGC CAT TTT CCC AGC CTG CCC CAA AGG TTT GGG
   Asn Met Glu Ala Gly Thr Met Ser His Phe Pro Ser Leu Pro Gln Arg Phe Gly
         387         396         405         414         423         432
   AGA ACA ACA GCC AGA CGC ATC ACC AAG ACA CTG GCT GGT TTG CCC CAG AAA TCC
   Arg Thr Thr Ala Arg Arg Ile Thr Lys Thr Leu Ala Gly Leu Pro Gln Lys Ser
         441         450         459         468         477         486
   CTG CAC TCC CTG GCC TCC AGT GAA TCG CTC TAT GCC ATG ACC CGC CAG CAT CAA
   Leu His Ser Leu Ala Ser Ser Glu Ser Leu Tyr Ala Met Thr Arg Gln His Gln
         495         504         513         522         531         540
   GAA ATT CAG AGT CCT GGT CAA GAC CAA CCT AGG AAA CGC GTG TTC ACG GAA ACA
   Glu Ile Gln Ser Pro Gly Gln Glu Gln Pro Arg Lys Arg Val Phe Thr Glu Thr
         549         558         567         576         585         594
   GAT GAT GCA GAA AGG AAA CAA GAA AAA ATA GGA AAC CTC CAG CCA GTC CTT CAA
   Asp Asp Ala Glu Arg Lys Gln Glu Lys Ile Gly Asn Leu Gln Pro Val Leu Gln
         603         612
   GGG GCT ATG AAG CTG TGA  3'
   Gly Ala Met Lys Leu ***
```

# Fig.6

```
                         10         20         30         40         50
hLPLRF.aa     1 MEIISSKLFI LLTLATSSLL TSNIFCADEL VMSNLHSKEN YDKYSEPRG-    50
bLPLRF.aa     1 MEIISLKRFI LLMLATSSLL TSNIFCIDES RMPNLYSKKN YDKYSEPRGD    50
rLPLRF.aa     1 MEIISSKRFI LLTLATSSFL TSNTLCSDEL MMPHFHSKEG YGKYYQLRGI    50

                         60         70         80         90        100
hLPLRF.aa    51 --YPKG--ER SLNFEELKDW GPKNVIKMST PAVNKMPHSF ANLPLRFGRN   100
bLPLRF.aa    51 LGWEK---ER SLTFEEVKDW APK--IKMNK PVVNKMPPSA ANLPLRFGRN   100
rLPLRF.aa    51 ---PKGVKER SVTFQELKDW GAKKDIKMSP APANKVPHSA ANLPLRFGRN   100

                        110        120        130        140        150
hLPLRF.aa   101 VQEERSAGAT ANLPLRSGRN MEVSLVRRVP NLPQRFGRTT TAKSVCRMLS   150
bLPLRF.aa   101 MEEERSIRAM AHLPLRLGKN REDSLSRWVP NLPQRFGRTT TAKSITKTLS   150
rLPLRF.aa   101 IEDRRSPRAR ANM------- -EAGTMSHFP SLPQRFGRTT -ARRITKTLA   150

                        160        170        180        190        200
hLPLRF.aa   151 DLCQGSMHSP CANDLFYSMT CQHQEIQNPD QKQSRRLLFK KIDDAELKQE   200
bLPLRF.aa   151 NLLQQSMHSP STNGLLYSMA CQPQEIQNPG QKNLRRRGFQ KIDDAELKQE   200
rLPLRF.aa   151 GLPQKSIHSL ASSESLYAMT RQHQEIQSPG QEQPRKRVET ETDDAEPKQE   200

                        210        220        230        240        250
hLPLRF.aa   201 K*-------- ------.... .......... .......... ..........   250
bLPLRF.aa   201 K*-------- ------.... .......... .......... ..........   250
rLPLRF.aa   201 KIGNLQPVLQ GAMKL*.... .......... .......... ..........   250
```

# Fig.7

1 TTTAGACTTAGACGAAATGGAAATTATTTCATTAAAACGATTCATTTTATTGACTGTG 58

1 MetGluIleIleSerLeuLysArgPheIleLeuLeuThrVal 14

59 GCAACTTCAAGCTTCTTAACATCAAACACCTTCTGTACAGATGAGTTCATGATGCCTCAT 118

15 AlaThrSerSerPheLeuThrSerAsnThrPheCysThrAspGluPheMetMetProHis 34

119 TTTCACAGCAAAGAAGGTGACGGAAAATACTCCCAGCTGAGAGGAATCCCAAAAGGGGAA 178

35 PheHisSerLysGluGlyAspGlyLysTyrSerGlnLeuArgGlyIleProLysGlyGlu 54

179 AAGGAAAGAAGTGTCAGTTTTCAAGAACTAAAAGATTGGGGGGCAAAGAATGTTATTAAG 238

55 LysGluArgSerValSerPheGlnGluLeuLysAspTrpGlyAlaLysAsnValIleLys 74

239 ATGAGTCCAGCCCCTGCCAACAAAGTGCCCCACTCAGCAGCCAACCTGCCCCTGAGATTT 298

75 MetSerProAlaProAlaAsnLysValProHisSerAlaAlaAsnLeuProLeuArgPhe 94

299 GGAAGGACCATAGATGAGAAAAGAAGCCCCGCAGCACGGGTCAACATGGAGGCAGGGACC 358

95 GlyArgThrIleAspGluLysArgSerProAlaAlaArgValAsnMetGluAlaGlyThr 114

359 AGGAGCCATTTCCCCAGCCTGCCCCAAAGGTTTGGGAGAACAACAGCCAGAAGCCCCAAG 418

115 ArgSerHisPheProSerLeuProGlnArgPheGlyArgThrThrAlaArgSerProLys 154

419 ACACCCGCTGATTTGCCACAGAAACCCCTGCACTCACTGGGCTCCAGCGAGTTGCTCTAC 538

135 ThrProAlaAspLeuProGlnLysProLeuHisSerLeuGlySerSerGluLeuLeuTyr 154

479 GTCATGATCTGCCAGCACCAAGAAATTCAGAGTCCTGGTGGAAAGCGAACGAGGAGAGGA 538

155 ValMetIleCysGlnHisGlnGluIleGlnSerProGlyGlyLysArgThrArgArgGly 174

539 GCGTTTGTGGAAACAGATGATGCAGAAAGGGAAACCAGAAAAATAGGAAACTCGAGCCCG 598

175 AlaPheValGluThrAspAspAlaGluArgLysProGluLys*** 188

599 ACTTCAAGAGGCTACGGAGC 618

188 188

# Fig.8

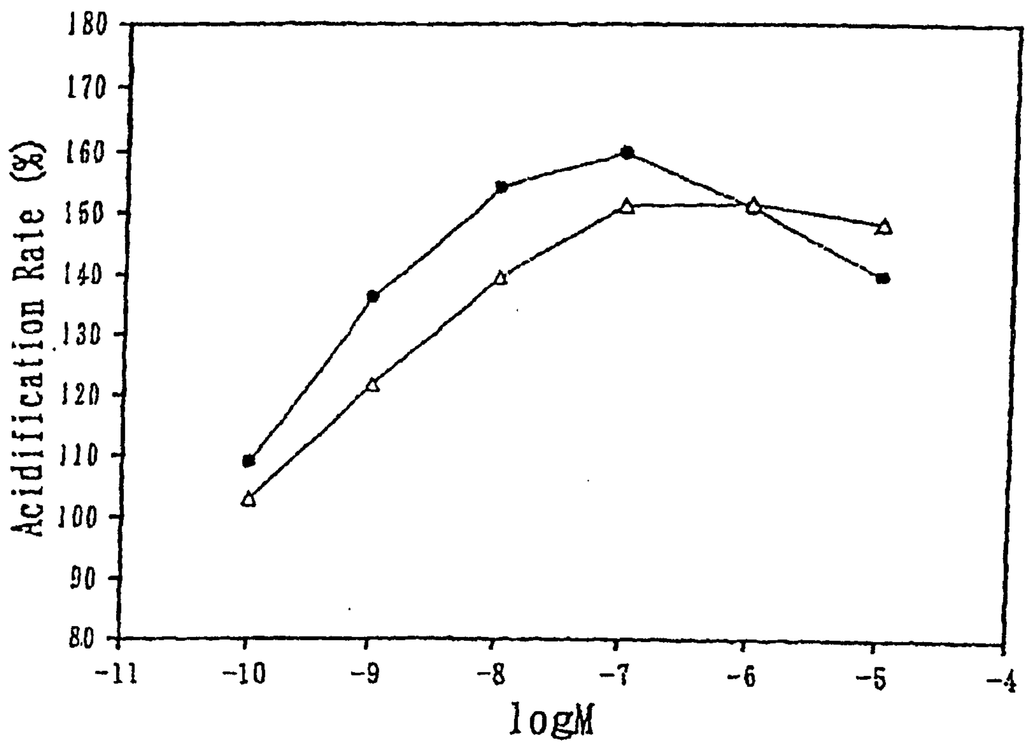
180
170
160
150
140
130
120
110
100
90
80
Acidification Rate (%)
-11
-10
-9
-8
-7
-6
-5
-4
logM

Fig.9

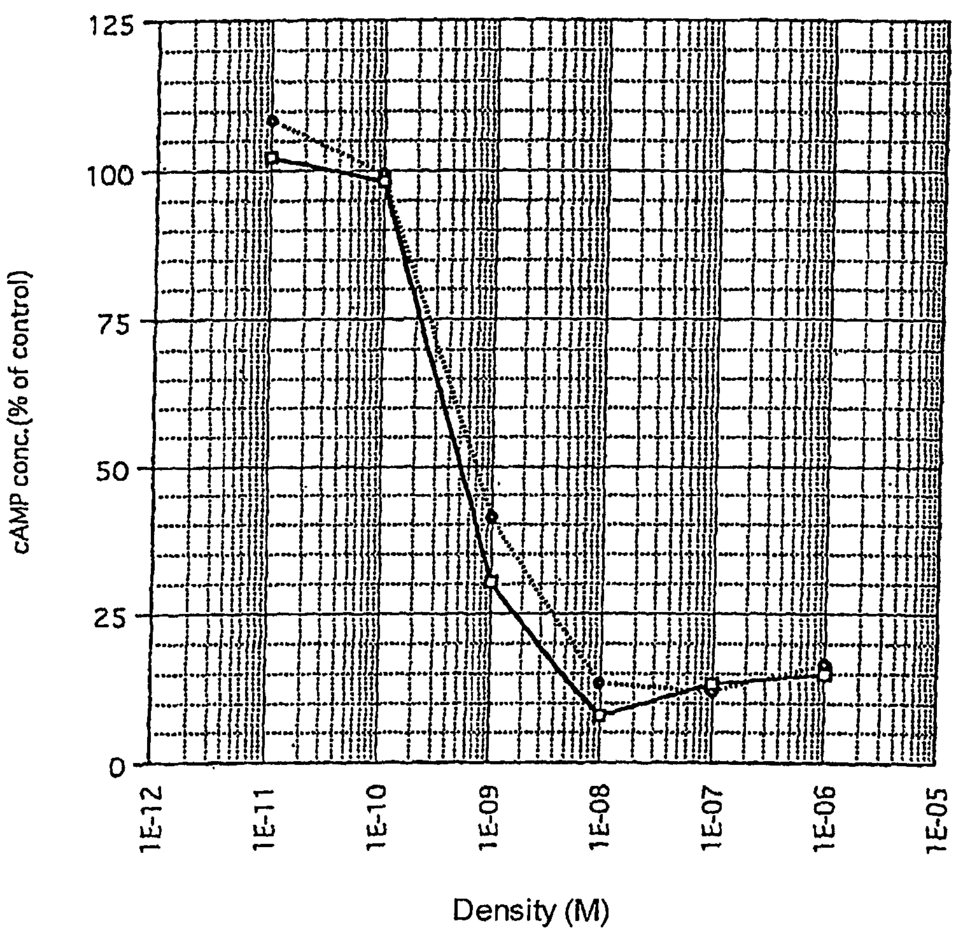
125
100
75
50
25
0
cAMP conc.(% of control)
1E-12
1E-11
1E-10
1E-09
1E-08
1E-07
1E-06
1E-05
Density (M)

Fig.10

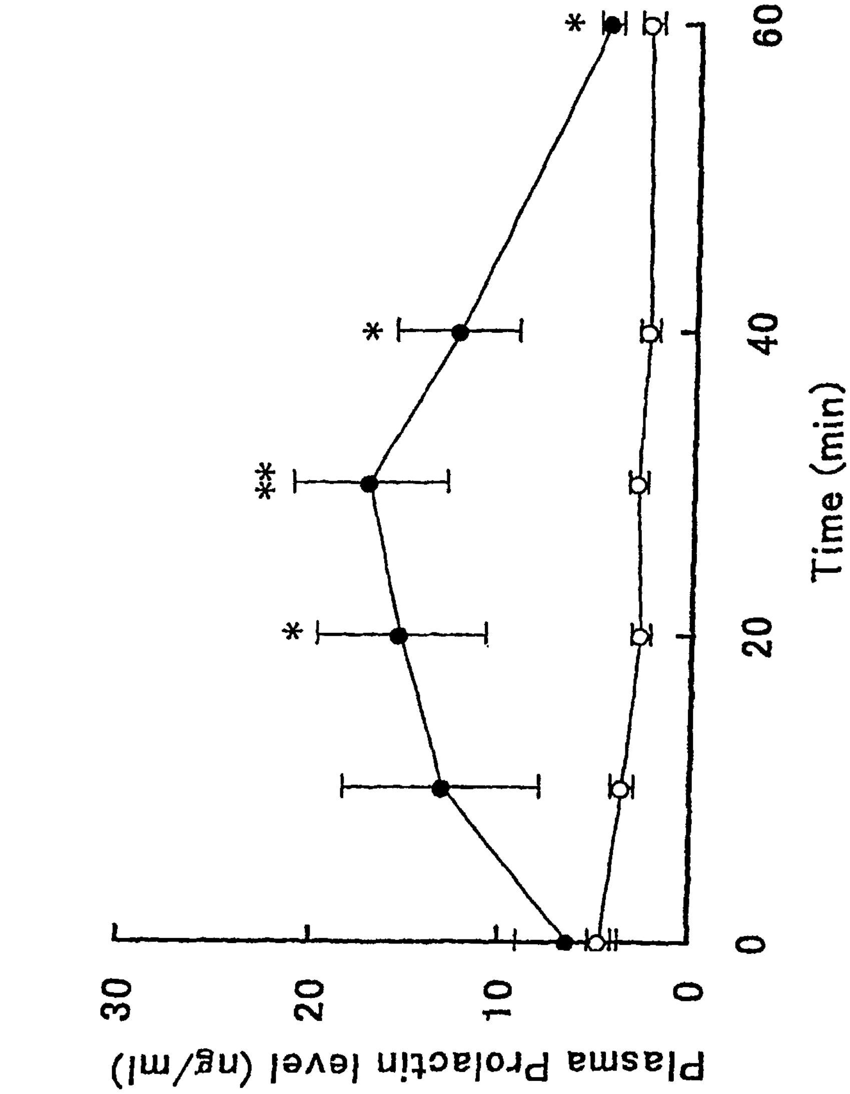

Fig.11

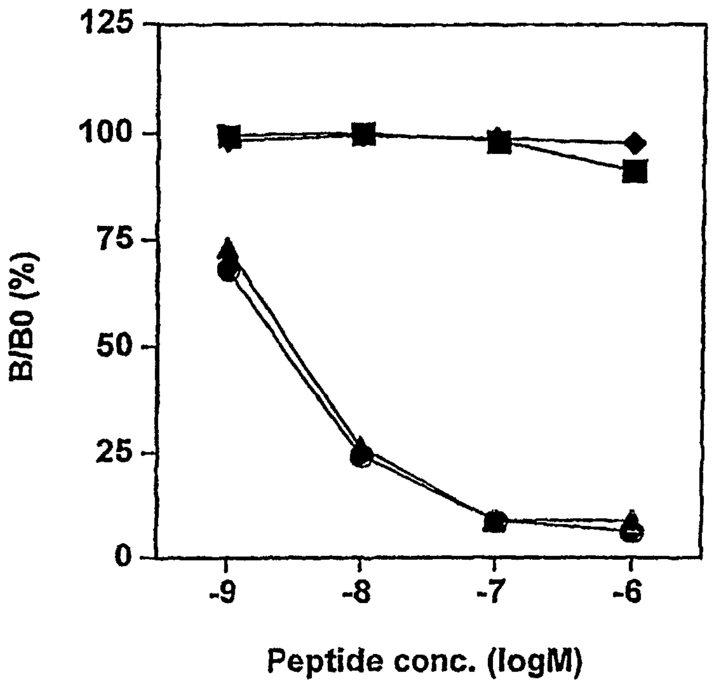

# Fig.12

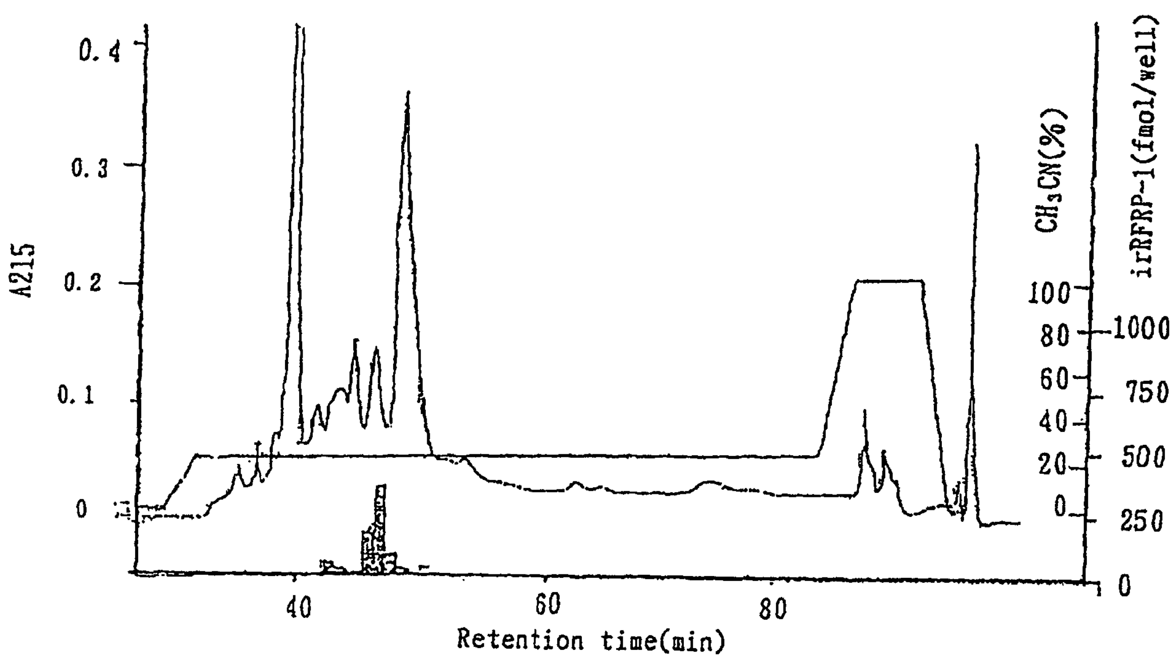
A215
0.4
0.3
0.2
0.1
0
40
60
80
Retention time(min)
CH$_3$CN(%)
irRFRP-1(fmol/well)
100
80
60
40
20
0
1000
750
500
250
0

# Fig.13

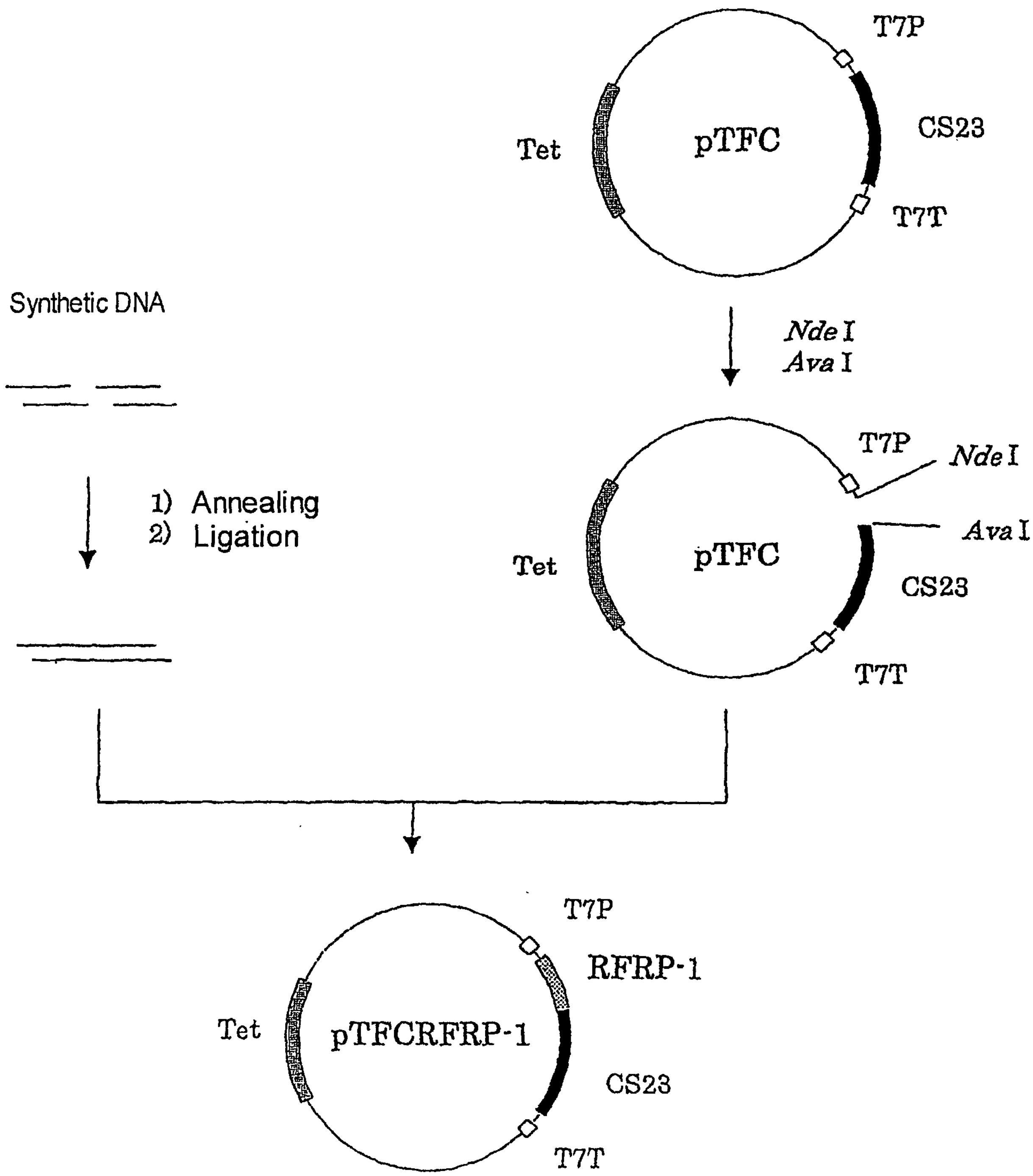
Synthetic DNA
1) Annealing
2) Ligation
T7P
CS23
Tet
pTFC
T7T
Nde I
Ava I
T7P
Nde I
Ava I
pTFC
Tet
CS23
T7T
T7P
RFRP-1
Tet
pTFCRFRP-1
CS23
T7T

Fig.14

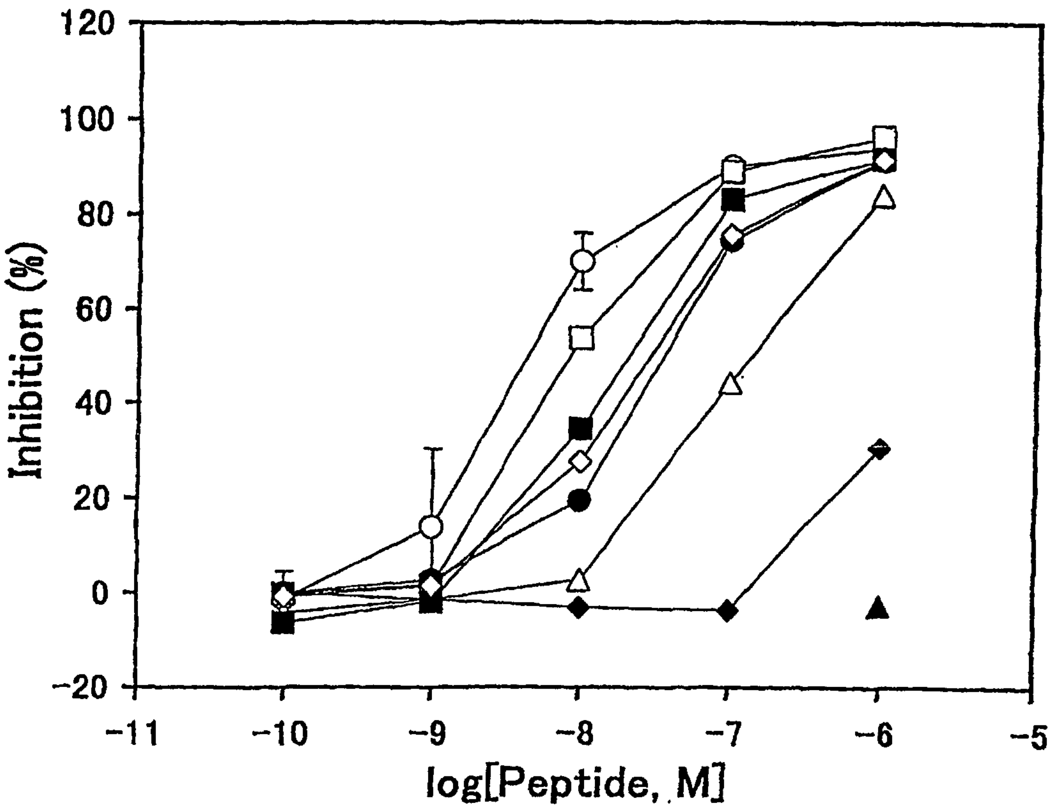

Fig.15

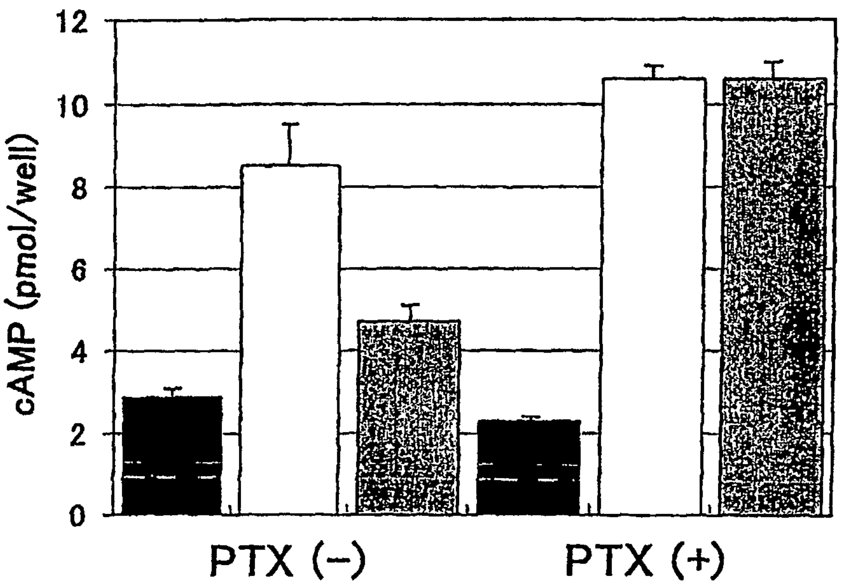

## INTERNATIONAL SEARCH REPORT

International application No.
PCT/JP01/01716

A. CLASSIFICATION OF SUBJECT MATTER
Int.Cl[7] A61K38/22, 45/00, A61P5/10, 15/00, 15/08, 15/10, C07K14/72, 16/28, C12N15/12, C12Q1/02, G01N33/12, 33/50, 33/566, 33/53

According to International Patent Classification (IPC) or to both national classification and IPC

B. FIELDS SEARCHED

Minimum documentation searched (classification system followed by classification symbols)
Int.Cl[7] A61K38/22, 45/00, A61P5/10, 15/00, 15/08, 15/10, C07K14/72, 16/28, C12N15/12, C12Q1/02, G01N33/12, 33/50, 33/566, 33/53

Documentation searched other than minimum documentation to the extent that such documents are included in the fields searched

Electronic data base consulted during the international search (name of data base and, where practicable, search terms used)
CAPLUS(STN),REGISTRY(STN),MEDLINE(STN),EMBASE(STN),BIOSIS(STN)
SwissProt/PIR/GeneSeq
Genbank/EMBL/DDBJ/GeneSeq

C. DOCUMENTS CONSIDERED TO BE RELEVANT

| Category* | Citation of document, with indication, where appropriate, of the relevant passages | Relevant to claim No. |
|---|---|---|
| P,X | WO, 2000/029441, A1 (Takeda Chemical Industries, Ltd.), 25 May, 2000 (25.05.00) (Family: none) & Database CAPLUS on STN, AMERICAN CHEMICAL SOCIETY (ACS),(Columbus, OH, USA), DN.133:16312 | 1-15,18-31 |
| P,X | HINUMA Shuji, et al., 'New neuropeptides containing carboxy-terminal RFamide and their receptor in mammals.' Nat. Cell Biol., October, 2000, Vol.2, No.10, pages 703 to 708 | 1-15,18-31 |
| A | SATAKE Honoo, et al.,'Characterization of a cDNA encoding a precursor of Carassius RFamide, structurally related to a mammalian prolactin-releasing peptide.' FEBS Lett., 1999, Vol.446, Nos.2,3, pages 247 to 250 | 1-15,18-31 |
| A | TENSEN Cornelis P., et al.,'The Lymnaea cardioexcitatory peptide (LyCEP) receptor: a G-protein-coupled receptor for a novel member of the RFamide neuropeptide family.' J. Neurosci., 1998, Vol.18, No.23, pages 9812 to 9821 | 1-15,18-31 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 25 May, 2001 (25.05.01) | 12 June, 2001 (12.06.01) |
| Name and mailing address of the ISA/ Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP01/01716

C (Continuation). DOCUMENTS CONSIDERED TO BE RELEVANT

| Category* | Citation of document, with indication, where appropriate, of the relevant passages | Relevant to claim No. |
|---|---|---|
| A | WO, 98/589621, A1 (Takeda Chemical Industries, Ltd.), 30 December, 1998 (30.12.98) & EP, 1001989, A1 & JP, 11-71300, A | 1-15,18-31 |
| A | WO, 97/24436, A2 (Takeda Chemical Industries, Ltd.), 10 July, 1997 (10.07.97) & EP, 870020, A2 & JP, 10-146192, A | 1-15,18-31 |
| X | Database REGISTRY on STN, AMERICAN CHEMICAL SOCIETY (ACS), (Columbus, OH, USA), Registry Number of 225879-38-9, 203609-95-4, 179723-46-7, 167743-76-2 | 22 |

Form PCT/ISA/210 (continuation of second sheet) (July 1992)

**INTERNATIONAL SEARCH REPORT**

International application No.
PCT/JP01/01716

**Box I Observations where certain claims were found unsearchable (Continuation of item 1 of first sheet)**

This international search report has not been established in respect of certain claims under Article 17(2)(a) for the following reasons:

1. ☒ Claims Nos.: 34,37,38
because they relate to subject matter not required to be searched by this Authority, namely:

Claims 34, 37 and 38 pertain to methods for treatment of the human body by therapy and thus relate to a subject matter which this International Searching Authority is not required, under the provisions of Article 17(2)(a)(i) of the PCT and Rule 39.1(iv) of the Regulations under the PCT, to search.

2. ☒ Claims Nos.: 16,17,32,33,35,36
because they relate to parts of the international application that do not comply with the prescribed requirements to such an extent that no meaningful international search can be carried out, specifically:

It can be neither specified nor anticipated by a person skilled in the art what compounds are involved in the scope of the compounds obtained by using a screening method or a screening kit.

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

[Sequence Listing]

<110> Takeda Chemical Industries, Ltd.

<120> Prolactin secretion modulator containing RFRP

<130> 2697WOOP

<150> JP 12-065752

<151> 2000-03-06

<150> JP 12-378001

<151> 2000-12-07

<160> 62

<210> 1

<211> 180

<212> PRT

<213> Human

<400> 1

Met Glu Ile Ile Ser Ser Lys Leu Phe Ile Leu Leu Thr Leu Ala Thr

1 5 10 15

Ser Ser Leu Leu Thr Ser Asn Ile Phe Cys Ala Asp Glu Leu Val Met

20 25 30

Ser Asn Leu His Ser Lys Glu Asn Tyr Asp Lys Tyr Ser Glu Pro Arg

35 40 45

Gly Tyr Pro Lys Gly Glu Arg Ser Leu Asn Phe Glu Glu Leu Lys Asp

50 55 60

Trp Gly Pro Lys Asn Val Ile Lys Met Ser Thr Pro Ala Val Asn Lys

65 70 75 80

Met Pro His Ser Phe Ala Asn Leu Pro Leu Arg Phe Gly Arg Asn Val

85 90 95

Gln Glu Glu Arg Ser Ala Gly Ala Thr Ala Asn Leu Pro Leu Arg Ser
100 105 110
Gly Arg Asn Met Glu Val Ser Leu Val Arg Arg Val Pro Asn Leu Pro
115 120 125
Gln Arg Phe Gly Arg Thr Thr Thr Ala Lys Ser Val Cys Arg Met Leu
130 135 140
Ser Asp Leu Cys Gln Gly Ser Met His Ser Pro Cys Ala Asn Asp Leu
145 150 155 160
Phe Tyr Ser Met Thr Cys Gln His Gln Glu Ile Gln Asn Pro Asp Gln
165 170 175
Lys Gln Ser Arg
180

<210> 2
<211> 540
<212> DNA
<213> Human
<400> 2

atggaaatta tttcatcaaa actattcatt ttattgactt tagccacttc aagcttgtta 60
acatcaaaca ttttttgtgc agatgaatta gtgatgtcca atcttcacag caaagaaaat 120
tatgacaaat attctgagcc tagaggatac ccaaaagggg aaagaagcct caattttgag 180
gaattaaaag attggggacc aaaaaatgtt attaagatga gtacacctgc agtcaataaa 240
atgccacact ccttcgccaa cttgccattg agatttggga ggaacgttca agaagaaaga 300
agtgctggag caacagccaa cctgcctctg agatctggaa agaaatatgga ggtgagcctc 360
gtgagacgtg ttcctaacct gccccaaagg tttgggagaa caacaacagc caaaagtgtc 420
tgcaggatgc tgagtgattt gtgtcaagga tccatgcatt caccatgtgc caatgactta 480
ttttactcca tgacctgcca gcaccaagaa atccagaatc ccgatcaaaa acagtcaagg 540

<210> 3

<211> 27

<212> DNA

<213> Artificial Sequence

<220>

<223>

<400> 3

gggctgcaca tagagactta attttag 27

<210> 4

<211> 27

<212> DNA

<213> Artificial Sequence

<220>

<223>

<400> 4

ctagaccacc tctatataac tgcccat 27

<210> 5

<211> 30

<212> DNA

<213> Artificial Sequence

<220>

<223>

<400> 5

gcacatagag acttaatttt agatttagac 30

<210> 6

<211> 27

<212> DNA

<213> Artificial Sequence

<220>

<223>

<400> 6

catgcacttt gactggtttc caggtat 27

<210> 7

<211> 27

<212> DNA

<213> Artificial Sequence

<220>

<223>

<400> 7

cagctttagg gacaggctcc aggtttc 27

<210> 8

<211> 196

<212> PRT

<213> Human

<400> 8

Met Glu Ile Ile Ser Ser Lys Leu Phe Ile Leu Leu Thr Leu Ala Thr
1 5 10 15

Ser Ser Leu Leu Thr Ser Asn Ile Phe Cys Ala Asp Glu Leu Val Met
20 25 30

Ser Asn Leu His Ser Lys Glu Asn Tyr Asp Lys Tyr Ser Glu Pro Arg
35 40 45

Gly Tyr Pro Lys Gly Glu Arg Ser Leu Asn Phe Glu Glu Leu Lys Asp

50 55 60

Trp Gly Pro Lys Asn Val Ile Lys Met Ser Thr Pro Ala Val Asn Lys

65 70 75 80

Met Pro His Ser Phe Ala Asn Leu Pro Leu Arg Phe Gly Arg Asn Val

85 90 95

Gln Glu Glu Arg Ser Ala Gly Ala Thr Ala Asn Leu Pro Leu Arg Ser

100 105 110

Gly Arg Asn Met Glu Val Ser Leu Val Arg Arg Val Pro Asn Leu Pro

115 120 125

Gln Arg Phe Gly Arg Thr Thr Thr Ala Lys Ser Val Cys Arg Met Leu

130 135 140

Ser Asp Leu Cys Gln Gly Ser Met His Ser Pro Cys Ala Asn Asp Leu

145 150 155 160

Phe Tyr Ser Met Thr Cys Gln His Gln Glu Ile Gln Asn Pro Asp Gln

165 170 175

Lys Gln Ser Arg Arg Leu Leu Phe Lys Lys Ile Asp Asp Ala Glu Leu

180 185 190

Lys Gln Glu Lys

195

<210> 9

<211> 588

<212> DNA

<213> Human

<400> 9

atggaaatta tttcatcaaa actattcatt ttattgactt tagccacttc aagcttgtta 60

acatcaaaca tttttgtgc agatgaatta gtgatgtcca atcttcacag caaagaaaat 120

tatgacaaat attctgagcc tagaggatac ccaaaagggg aaagaagcct caattttgag 180

gaattaaaag attggggacc aaaaaatgtt attaagatga gtacacctgc agtcaataaa 240

atgccacact ccttcgccaa cttgccattg agatttggga ggaacgttca agaagaaaga 300

agtgctggag caacagccaa cctgcctctg agatctggaa gaaatatgga ggtgagcctc 360

gtgagacgtg ttcctaacct gccccaaagg tttgggagaa caacaacagc caaaagtgtc 420

tgcaggatgc tgagtgattt gtgtcaagga tccatgcatt caccatgtgc caatgactta 480

ttttactcca tgacctgcca gcaccaagaa atccagaatc ccgatcaaaa acagtcaagg 540

agactgctat tcaagaaaat agatgatgca gaattgaaac aagaaaaa 588

<210> 10

<211> 27

<212> DNA

<213> Artificial Sequence

<220>

<223>

<400> 10

gcctagagga gatctaggct gggagga 27

<210> 11

<211> 27

<212> DNA

<213> Artificial Sequence

<220>

<223>

<400> 11

gggaggaaca tggaagaaga aaggagc 27

<210> 12

<211> 27

<212> DNA

<213> Artificial Sequence

<220>

<223>

<400> 12

gatggtgaat gcatggactg ctggagc 27

<210> 13

<211> 27

<212> DNA

<213> Artificial Sequence

<220>

<223>

<400> 13

ttcctcccaa atctcagtgg caggttg 27

<210> 14

<211> 196

<212> PRT

<213> Bovine

<400> 14

Met Glu Ile Ile Ser Leu Lys Arg Phe Ile Leu Leu Met Leu Ala Thr
1 5 10 15

Ser Ser Leu Leu Thr Ser Asn Ile Phe Cys Thr Asp Glu Ser Arg Met
20 25 30

Pro Asn Leu Tyr Ser Lys Lys Asn Tyr Asp Lys Tyr Ser Glu Pro Arg
35 40 45

Gly Asp Leu Gly Trp Glu Lys Glu Arg Ser Leu Thr Phe Glu Glu Val

50 55 60

Lys Asp Trp Ala Pro Lys Ile Lys Met Asn Lys Pro Val Val Asn Lys

65 70 75 80

Met Pro Pro Ser Ala Ala Asn Leu Pro Leu Arg Phe Gly Arg Asn Met

85 90 95

Glu Glu Glu Arg Ser Thr Arg Ala Met Ala His Leu Pro Leu Arg Leu

100 105 110

Gly Lys Asn Arg Glu Asp Ser Leu Ser Arg Trp Val Pro Asn Leu Pro

115 120 125

Gln Arg Phe Gly Arg Thr Thr Thr Ala Lys Ser Ile Thr Lys Thr Leu

130 135 140

Ser Asn Leu Leu Gln Gln Ser Met His Ser Pro Ser Thr Asn Gly Leu

145 150 155 160

Leu Tyr Ser Met Ala Cys Gln Pro Gln Glu Ile Gln Asn Pro Gly Gln

165 170 175

Lys Asn Leu Arg Arg Arg Gly Phe Gln Lys Ile Asp Asp Ala Glu Leu

180 185 190

Lys Gln Glu Lys

195

<210> 15

<211> 588

<212> DNA

<213> Bovine

<400> 15

atggaaatta tttcattaaa acgattcatt ttattgatgt tagccacttc aagcttgtta 60

acatcaaaca tcttctgcac agacgaatca aggatgccca atctttacag caaaaagaat 120

tatgacaaat attccgagcc tagaggagat ctaggctggg agaaagaaag aagtcttact 180
tttgaagaag taaaagattg ggctccaaaa attaagatga ataaacctgt agtcaacaaa 240
atgccacctt ctgcagccaa cctgccactg agatttggga ggaacatgga agaagaaagg 300
agcactaggg cgatggccca cctgcctctg agactcggaa aaaatagaga ggacagcctc 360
tccagatggg tcccaaatct gccccagagg tttggaagaa caacaacagc caaaagcatt 420
accaagaccc tgagtaattt gctccagcag tccatgcatt caccatctac caatgggcta 480
ctctactcca tggcctgcca gccccaagaa atccagaatc ctggtcaaaa gaacctaagg 540
agacggggat tccagaaaat agatgatgca gaattgaaac aagaaaaa 588

<210> 16
<211> 27
<212> DNA
<213> Artificial Sequence
<220>
<223>
<400> 16
ccctggggct tcttctgtct tctatgt 27

<210> 17
<211> 26
<212> DNA
<213> Artificial Sequence
<220>
<223>
<400> 17
agcgattcat tttattgact ttagca 26

<210> 18
<211> 203

<212> PRT

<213> Rat

<400> 18

Met Glu Ile Ile Ser Ser Lys Arg Phe Ile Leu Leu Thr Leu Ala Thr
1 5 10 15

Ser Ser Phe Leu Thr Ser Asn Thr Leu Cys Ser Asp Glu Leu Met Met
20 25 30

Pro His Phe His Ser Lys Glu Gly Tyr Gly Lys Tyr Tyr Gln Leu Arg
35 40 45

Gly Ile Pro Lys Gly Val Lys Glu Arg Ser Val Thr Phe Gln Glu Leu
50 55 60

Lys Asp Trp Gly Ala Lys Lys Asp Ile Lys Met Ser Pro Ala Pro Ala
65 70 75 80

Asn Lys Val Pro His Ser Ala Ala Asn Leu Pro Leu Arg Phe Gly Arg
85 90 95

Asn Ile Glu Asp Arg Arg Ser Pro Arg Ala Arg Ala Asn Met Glu Ala
100 105 110

Gly Thr Met Ser His Phe Pro Ser Leu Pro Gln Arg Phe Gly Arg Thr
115 120 125

Thr Ala Arg Arg Ile Thr Lys Thr Leu Ala Gly Leu Pro Gln Lys Ser
130 135 140

Leu His Ser Leu Ala Ser Ser Glu Ser Leu Tyr Ala Met Thr Arg Gln
145 150 155 160

His Gln Glu Ile Gln Ser Pro Gly Gln Glu Gln Pro Arg Lys Arg Val
165 170 175

Phe Thr Glu Thr Asp Asp Ala Glu Arg Lys Gln Glu Lys Ile Gly Asn

180 185 190

Leu Gln Pro Val Leu Gln Gly Ala Met Lys Leu

195 200

<210> 19

<211> 609

<212> DNA

<213> Rat

<400> 19

atggaaatta tttcatcaaa gcgattcatt ttattgactt tagcaacttc aagcttctta 60

acttcaaaca ccctttgttc agatgaatta atgatgcccc attttcacag caaagaaggt 120

tatggaaaat attaccagct gagaggaatc ccaaaagggg taaaggaaag aagtgtcact 180

tttcaagaac tcaaagattg gggggcaaag aaagatatta agatgagtcc agcccctgcc 240

aacaaagtgc cccactcagc agccaacctt cccctgaggt ttgggaggaa catagaagac 300

agaagaagcc ccagggcacg ggccaacatg gaggcaggga ccatgagcca ttttcccagc 360

ctgccccaaa ggtttgggag aacaacagcc agacgcatca ccaagacact ggctggtttg 420

ccccagaaat ccctgcactc cctggcctcc agtgaatcgc tctatgccat gacccgccag 480

catcaagaaa ttcagagtcc tggtcaagag caacctagga aacgggtgtt cacggaaaca 540

gatgatgcag aaaggaaaca agaaaaaata ggaaacctcc agccagtcct tcaaggggct 600

atgaagctg 609

<210> 20

<211> 12

<212> DNA

<213> Artificial Sequence

<220>

<223>

<400> 20

mgnityggna ar 12

<210> 21

<211> 12

<212> DNA

<213> Artificial Sequence

<220>

<223>

<400> 21

mgnttyggnm gn 12

<210> 22

<211> 12

<212> DNA

<213> Artificial Sequence

<220>

<223>

<400> 22

mgnwsnggna ar 12

<210> 23

<211> 12

<212> DNA

<213> Artificial Sequence

<220>

<223>

<400> 23

mgnwsnggnm gn 12

<210> 24

<211> 12

<212> DNA

<213> Artificial Sequence

<220>

<223>

<400> 24

mgnytnggna ar 12

<210> 25

<211> 12

<212> DNA

<213> Artificial Sequence

<220>

<223>

<400> 25

mgnytnggnm gn 12

<210> 26

<211> 30

<212> DNA

<213> Artificial Sequence

<220>

<223>

<400> 26

gacttaattt tagatttaga caaaatggaa 30

<210> 27

<211> 25

<212> DNA

<213> Artificial Sequence

<220>

<223>

<400> 27

ttctcccaaa cctttggggc aggtt 25

<210> 28

<211> 28

<212> DNA

<213> Artificial Sequence

<220>

<223>

<400> 28

acagcaaaga aggtgacgga aaatactc 28

<210> 29

<211> 28

<212> DNA

<213> Artificial Sequence

<220>

<223>

<400> 29

atagatgaga aaagaagccc cgcagcac 28

<210> 30

<211> 28

<212> DNA

<213> Artificial Sequence

<220>

<223>

<400> 30

gtgctgcggg gcttcttttc tcatctat 28

<210> 31

<211> 21

<212> DNA

<213> Artificial Sequence

<220>

<223>

<400> 31

tttagactta gacgaaatgg a 21

<210> 32

<211> 21

<212> DNA

<213> Artificial Sequence

<220>

<223>

<400> 32

gctccgtagc ctcttgaagt c 21

<210> 33

<211> 188

<212> PRT

<213> Mouse

<400> 33

Met Glu Ile Ile Ser Leu Lys Arg Phe Ile Leu Leu Thr Val Ala Thr

1 5 10 15

Ser Ser Phe Leu Thr Ser Asn Thr Phe Cys Thr Asp Glu Phe Met Met
20 25 30
Pro His Phe His Ser Lys Glu Gly Asp Gly Lys Tyr Ser Gln Leu Arg
35 40 45
Gly Ile Pro Lys Gly Glu Lys Glu Arg Ser Val Ser Phe Gln Glu Leu
50 55 60
Lys Asp Trp Gly Ala Lys Asn Val Ile Lys Met Ser Pro Ala Pro Ala
65 70 75 80
Asn Lys Val Pro His Ser Ala Ala Asn Leu Pro Leu Arg Phe Gly Arg
85 90 95
Thr Ile Asp Glu Lys Arg Ser Pro Ala Ala Arg Val Asn Met Glu Ala
100 105 110
Gly Thr Arg Ser His Phe Pro Ser Leu Pro Gln Arg Phe Gly Arg Thr
115 120 125
Thr Ala Arg Ser Pro Lys Thr Pro Ala Asp Leu Pro Gln Lys Pro Leu
130 135 140
His Ser Leu Gly Ser Ser Glu Leu Leu Tyr Val Met Ile Cys Gln His
145 150 155 160
Gln Glu Ile Gln Ser Pro Gly Gly Lys Arg Thr Arg Arg Gly Ala Phe
165 170 175
Val Glu Thr Asp Asp Ala Glu Arg Lys Pro Glu Lys
180 185

<210> 34
<211> 564
<212> DNA
<213> Mouse

<400> 34

atggaaatta tttcattaaa acgattcatt ttattgactg tggcaacttc aagcttctta 60

acatcaaaca ccttctgtac agatgagttc atgatgcctc attttcacag caaagaaggt 120

gacggaaaat actcccagct gagaggaatc ccaaaagggg aaaaggaaag aagtgtcagt 180

tttcaagaac taaaagattg gggggcaaag aatgttatta agatgagtcc agcccctgcc 240

aacaaagtgc cccactcagc agccaacctg cccctgagat ttggaaggac catagatgag 300

aaaagaagcc ccgcagcacg ggtcaacatg gaggcaggga ccaggagcca tttccccagc 360

ctgccccaaa ggtttgggag aacaacagcc agaagcccca agacacccgc tgatttgcca 420

cagaaacccc tgcactcact gggctccagc gagttgctct acgtcatgat ctgccagcac 480

caagaaattc agagtcctgg tggaaagcga acgaggagag gagcgtttgt ggaaacagat 540

gatgcagaaa ggaaaccaga aaaa 564

<210> 35

<211> 27

<212> DNA

<213> Artificial Sequence

<220>

<223>

<400> 35

agtcgacagt atggaggcgg agccctc 27

<210> 36

<211> 29

<212> DNA

<213> Artificial Sequence

<220>

<223>

<400> 36

gactagttca aatgttccag gccgggatg 29

<210> 37

<211> 432

<212> PRT

<213> Rat

<400> 37

Met Glu Ala Glu Pro Ser Gln Pro Pro Asn Gly Ser Trp Pro Leu Gly
5 10 15

Gln Asn Gly Ser Asp Val Glu Thr Ser Met Ala Thr Ser Leu Thr Phe
20 25 30

Ser Ser Tyr Tyr Gln His Ser Ser Pro Val Ala Ala Met Phe Ile Ala
35 40 45

Ala Tyr Val Leu Ile Phe Leu Leu Cys Met Val Gly Asn Thr Leu Val
50 55 60

Cys Phe Ile Val Leu Lys Asn Arg His Met Arg Thr Val Thr Asn Met
65 70 75 80

Phe Ile Leu Asn Leu Ala Val Ser Asp Leu Leu Val Gly Ile Phe Cys
85 90 95

Met Pro Thr Thr Leu Val Asp Asn Leu Ile Thr Gly Trp Pro Phe Asp
100 105 110

Asn Ala Thr Cys Lys Met Ser Gly Leu Val Gln Gly Met Ser Val Ser
115 120 125

Ala Ser Val Phe Thr Leu Val Ala Ile Ala Val Glu Arg Phe Arg Cys
130 135 140

Ile Val His Pro Phe Arg Glu Lys Leu Thr Leu Arg Lys Ala Leu Phe
145 150 155 160

Thr Ile Ala Val Ile Trp Ala Leu Ala Leu Leu Ile Met Cys Pro Ser
165 170 175

Ala Val Thr Leu Thr Val Thr Arg Glu Glu His His Phe Met Leu Asp
180 185 190

Ala Arg Asn Arg Ser Tyr Pro Leu Tyr Ser Cys Trp Glu Ala Trp Pro
195 200 205

Glu Lys Gly Met Arg Lys Val Tyr Thr Ala Val Leu Phe Ala His Ile
210 215 220

Tyr Leu Val Pro Leu Ala Leu Ile Val Val Met Tyr Val Arg Ile Ala
225 230 235 240

Arg Lys Leu Cys Gln Ala Pro Gly Pro Ala Arg Asp Thr Glu Glu Ala
245 250 255

Val Ala Glu Gly Gly Arg Thr Ser Arg Arg Arg Ala Arg Val Val His
260 265 270

Met Leu Val Met Val Ala Leu Phe Phe Thr Leu Ser Trp Leu Pro Leu
275 280 285

Trp Val Leu Leu Leu Leu Ile Asp Tyr Gly Glu Leu Ser Glu Leu Gln
290 295 300

Leu His Leu Leu Ser Val Tyr Ala Phe Pro Leu Ala His Trp Leu Ala
305 310 315 320

Phe Phe His Ser Ser Ala Asn Pro Ile Ile Tyr Gly Tyr Phe Asn Glu
325 330 335

Asn Phe Arg Arg Gly Phe Gln Ala Ala Phe Arg Ala Gln Leu Cys Trp
340 345 350

Pro Pro Trp Ala Ala His Lys Gln Ala Tyr Ser Glu Arg Pro Asn Arg
355 360 365

Leu Leu Arg Arg Arg Val Val Val Asp Val Gln Pro Ser Asp Ser Gly
370 375 380

Leu Pro Ser Glu Ser Gly Pro Ser Ser Gly Val Pro Gly Pro Gly Arg
385 390 395 400

Leu Pro Leu Arg Asn Gly Arg Val Ala His Gln Asp Gly Pro Gly Glu
405 410 415

Gly Pro Gly Cys Asn His Met Pro Leu Thr Ile Pro Ala Trp Asn Ile
420 425 430

<210> 38

<211> 1299

<212> DNA

<213> Rat

<400> 38

atggaggcgg agccctccca gcctcccaac ggcagctggc ccctgggtca gaacgggagt 60

gatgtggaga ccagcatggc aaccagcctc accttctcct cctactacca acactcctct 120

ccggtggcag ccatgttcat cgcggcctac gtgctcatct tcctcctctg catggtgggc 180

aacaccctgg tctgcttcat tgtgctcaag aaccggcaca tgcgcactgt caccaacatg 240

tttatcctca acctggccgt cagcgacctg ctggtgggca tcttctgcat gcccacaacc 300

cttgtggaca accttatcac tggttggcct tttgacaacg ccacatgcaa gatgagcggc 360

ttggtgcagg gcatgtccgt gtctgcatcg gttttcacac tggtggccat cgctgtggaa 420

aggttccgct gcatcgtgca ccctttccgc gagaagctga cccttcggaa ggcgctgttc 480

accatcgcgg tgatctgggc tctggcgctg ctcatcatgt gtccctcggc ggtcactctg 540

acagtcaccc gagaggagca tcacttcatg ctggatgctc gtaaccgctc ctacccgctc 600

tactcgtgct gggaggcctg gcccgagaag ggcatgcgca aggtctacac cgcggtgctc 660

ttcgcgcaca tctacctggt gccgctggcg ctcatcgtag tgatgtacgt gcgcatcgcg 720

cgcaagctat gccaggcccc cggtcctgcg cgcgacacgg aggaggcggt ggccgagggt 780

ggccgcactt cgcgccgtag ggccccgcgtg gtgcacatgc tggtcatggt ggcgctcttc 840
ttcacgttgt cctggctgcc actctgggtg ctgctgctgc tcatcgacta tggggagctg 900
agcgagctgc aactgcacct gctgtcggtc tacgccttcc ccttggcaca ctggctggcc 960
ttcttccaca gcagcgccaa ccccatcatc tacggctact tcaacgagaa cttccgccgc 1020
ggcttccagg ctgccttccg tgcacagctc tgctggcctc cctgggccgc ccacaagcaa 1080
gcctactcgg agcggcccaa ccgcctcctg cgcaggcggg tggtggtgga cgtgcaaccc 1140
agcgactccg gcctgccatc agagtctggc cccagcagcg gggtcccagg gcctggccgg 1200
ctgccactgc gcaatgggcg tgtggcccat caggatggcc cgggggaagg gccaggctgc 1260
aaccacatgc ccctcaccat cccggcctgg aacatttga 1299

<210> 39

<211> 12

<212> PRT

<213> Artificial Sequence

<220>

<223> the C-terminus of the polypeptide is amide ($-CONH_2$) form

<400> 39

Met Pro His Ser Phe Ala Asn Leu Pro Leu Arg Phe
1 5 10

<210> 40

<211> 8

<212> PRT

<213> Artificial Sequence

<220>

<223> the C-terminus of the polypeptide is amide ($-CONH_2$) form

<400> 40

Val Pro Asn Leu Pro Gln Arg Phe

1 5

<210> 41

<211> 11

<212> PRT

<213> Artificial Sequence

<220>

<223> the C-terminus of the polypeptide is amide (-$CONH_2$) form

<400> 41

Ser Ala Gly Ala Thr Ala Asn Leu Pro Arg Ser

1 5 10

<210> 42

<211> 36

<212> DNA

<213> Human

<400> 42

atgccacact ccttcgccaa cttgccattg agattt 36

<210> 43

<211> 36

<212> DNA

<213> Human

<400> 43

agtgctggag caacagccaa cctgcctctg agatct 36

<210> 44

<211> 24

<212> DNA

<213> Human

<400> 44

gttcctaacc tgccccaaag gttt 24

<210> 45

<211> 276

<212> DNA

<213> Human

<400> 45

atggaaatta tttcatcaaa actattcatt ttattgactt tagccacttc aagcttgtta 60

acatcaaaca tttttgtgc agatgaatta gtgatgtcca atcttcacag caaagaaaat 120

tatgacaaat attctgagcc tagaggatac ccaaaagggg aaagaagcct caattttgag 180

gaattaaaag attggggacc aaaaaatgtt attaagatga gtacacctgc agtcaataaa 240

atgccacact ccttcgccaa cttgccattg agattt 276

<210> 46

<211> 336

<212> DNA

<213> Human

<400> 46

atggaaatta tttcatcaaa actattcatt ttattgactt tagccacttc aagcttgtta 60

acatcaaaca tttttgtgc agatgaatta gtgatgtcca atcttcacag caaagaaaat 120

tatgacaaat attctgagcc tagaggatac ccaaaagggg aaagaagcct caattttgag 180

gaattaaaag attggggacc aaaaaatgtt attaagatga gtacacctgc agtcaataaa 240

atgccacact ccttcgccaa cttgccattg agatttggga ggaacgttca agaagaaaga 300

agtgctggag caacagccaa cctgcctctg agatct 336

<210> 47

<211> 393

<212> DNA

<213> Human

<400> 47

atggaaatta tttcatcaaa actattcatt ttattgactt tagccacttc aagcttgtta 60

acatcaaaca tttttgtgc agatgaatta gtgatgtcca atcttcacag caaagaaaat 120

tatgacaaat attctgagcc tagaggatac ccaaaagggg aaagaagcct caattttgag 180

gaattaaaag attggggacc aaaaaatgtt attaagatga gtacacctgc agtcaataaa 240

atgccacact ccttcgccaa cttgccattg agatttggga ggaacgttca agaagaaaga 300

agtgctggag caacagccaa cctgcctctg agatctgga agaaatatgga ggtgagcctc 360

gtgagacgtg ttcctaacct gccccaaagg ttt 393

<210> 48

<211> 27

<212> DNA

<213> Artificial Sequence

<220>

<223>

<400> 48

ccctggggct tcttctgtct tctatgt 27

<210> 49

<211> 26

<212> DNA

<213> Artificial Sequence

<220>

<223>

<400> 49

agcgattcat tttattgact ttagca 26

<210> 50

<211> 203

<212> PRT

<213> Rat

<400> 50

Met Glu Ile Ile Ser Ser Lys Arg Phe Ile Leu Leu Thr Leu Ala Thr
1 5 10 15

Ser Ser Phe Leu Thr Ser Asn Thr Leu Cys Ser Asp Glu Leu Met Met
20 25 30

Pro His Phe His Ser Lys Glu Gly Tyr Gly Lys Tyr Tyr Gln Leu Arg
35 40 45

Gly Ile Pro Lys Gly Val Lys Glu Arg Ser Val Thr Phe Gln Glu Leu
50 55 60

Lys Asp Trp Gly Ala Lys Lys Asp Ile Lys Met Ser Pro Ala Pro Ala
65 70 75 80

Asn Lys Val Pro His Ser Ala Ala Asn Leu Pro Leu Arg Phe Gly Arg
85 90 95

Asn Ile Glu Asp Arg Arg Ser Pro Arg Ala Arg Ala Asn Met Glu Ala
100 105 110

Gly Thr Met Ser His Phe Pro Ser Leu Pro Gln Arg Phe Gly Arg Thr
115 120 125

Thr Ala Arg Arg Ile Thr Lys Thr Leu Ala Gly Leu Pro Gln Lys Ser
130 135 140

Leu His Ser Leu Ala Ser Ser Glu Leu Leu Tyr Ala Met Thr Arg Gln
145 150 155 160

His Gln Glu Ile Gln Ser Pro Gly Gln Glu Gln Pro Arg Lys Arg Val
165 170 175

Phe Thr Glu Thr Asp Asp Ala Glu Arg Lys Gln Glu Lys Ile Gly Asn

180 185 190

Leu Gln Pro Val Leu Gln Gly Ala Met Lys Leu

195 200

<210> 51

<211> 609

<212> DNA

<213> Rat

<400> 51

atggaaatta tttcatcaaa gcgattcatt ttattgactt tagcaacttc aagcttctta 60

acttcaaaca ccctttgttc agatgaatta atgatgcccc attttcacag caaagaaggt 120

tatggaaaat attaccagct gagaggaatc ccaaaagggg taaaggaaag aagtgtcact 180

tttcaagaac tcaaagattg gggggcaaag aaagatatta agatgagtcc agcccctgcc 240

aacaaagtgc cccactcagc agccaacctt cccctgaggt ttgggaggaa catagaagac 300

agaagaagcc ccagggcacg ggccaacatg gaggcaggga ccatgagcca ttttcccagc 360

ctgccccaaa ggtttgggag aacaacagcc agacgcatca ccaagacact ggctggtttg 420

ccccagaaat ccctgcactc cctggcctcc agtgaattgc tctatgccat gacccgccag 480

catcaagaaa ttcagagtcc tggtcaagag caacctagga aacgggtgtt cacggaaaca 540

gatgatgcag aaaggaaaca agaaaaaata ggaaacctcc agccagtcct tcaaggggct 600

atgaagctg 609

<210> 52

<211> 27

<212> DNA

<213> Artificial Sequence

<220>

<223>

<400> 52

ttctagattt tggacaaaat ggaaatt 27

<210> 53

<211> 27

<212> DNA

<213> Artificial Sequence

<220>

<223>

<400> 53

cgtctttagg gacaggctcc agatttc 27

<210> 54

<211> 430

<212> PRT

<213> Human

<400> 54

Met Glu Gly Glu Pro Ser Gln Pro Pro Asn Ser Ser Trp Pro Leu Ser
1 5 10 15

Gln Asn Gly Thr Asn Thr Glu Ala Thr Pro Ala Thr Asn Leu Thr Phe
20 25 30

Ser Ser Tyr Tyr Gln His Thr Ser Pro Val Ala Ala Met Phe Ile Val
35 40 45

Ala Tyr Ala Leu Ile Phe Leu Leu Cys Met Val Gly Asn Thr Leu Val
50 55 60

Cys Phe Ile Val Leu Lys Asn Arg His Met His Thr Val Thr Asn Met
65 70 75 80

Phe Ile Leu Asn Leu Ala Val Ser Asp Leu Leu Val Gly Ile Phe Cys

85 90 95

Met Pro Thr Thr Leu Val Asp Asn Leu Ile Thr Gly Trp Pro Phe Asp

100 105 110

Asn Ala Thr Cys Lys Met Ser Gly Leu Val Gln Gly Met Ser Val Ser

115 120 125

Ala Ser Val Phe Thr Leu Val Ala Ile Ala Val Glu Arg Phe Arg Cys

130 135 140

Ile Val His Pro Phe Arg Glu Lys Leu Thr Leu Arg Lys Ala Leu Val

145 150 155 160

Thr Ile Ala Val Ile Trp Ala Leu Ala Leu Leu Ile Met Cys Pro Ser

165 170 175

Ala Val Thr Leu Thr Val Thr Arg Glu Glu His His Phe Met Val Asp

180 185 190

Ala Arg Asn Arg Ser Tyr Pro Leu Tyr Ser Cys Trp Glu Ala Trp Pro

195 200 205

Glu Lys Gly Met Arg Arg Val Tyr Thr Thr Val Leu Phe Ser His Ile

210 215 220

Tyr Leu Ala Pro Leu Ala Leu Ile Val Val Met Tyr Ala Arg Ile Ala

225 230 235 240

Arg Lys Leu Cys Gln Ala Pro Gly Pro Ala Pro Gly Gly Glu Glu Ala

245 250 255

Ala Asp Pro Arg Ala Ser Arg Arg Arg Ala Arg Val Val His Met Leu

260 265 270

Val Met Val Ala Leu Phe Phe Thr Leu Ser Trp Leu Pro Leu Trp Ala

275 280 285

Leu Leu Leu Leu Ile Asp Tyr Gly Gln Leu Ser Ala Pro Gln Leu His

290 295 300

Leu Val Thr Val Tyr Ala Phe Pro Phe Ala His Trp Leu Ala Phe Phe

305 310 315 320

Asn Ser Ser Ala Asn Pro Ile Ile Tyr Gly Tyr Phe Asn Glu Asn Phe

325 330 335

Arg Arg Gly Phe Gln Ala Ala Phe Arg Ala Arg Leu Cys Pro Arg Pro

340 345 350

Ser Gly Ser His Lys Glu Ala Tyr Ser Glu Arg Pro Gly Gly Leu Leu

355 360 365

His Arg Arg Val Phe Val Val Val Arg Pro Ser Asp Ser Gly Leu Pro

370 375 380

Ser Glu Ser Gly Pro Ser Ser Gly Ala Pro Arg Pro Gly Arg Leu Pro

385 390 395 400

Leu Arg Asn Gly Arg Val Ala His His Gly Leu Pro Arg Glu Gly Pro

405 410 415

Gly Cys Ser His Leu Pro Leu Thr Ile Pro Ala Trp Asp Ile

420 425 430

<210> 55

<211> 1290

<212> DNA

<213> Human

<400> 55

atggaggggg agccctccca gcctcccaac agcagttggc cctaagtca gaatgggact 60

aacactgagg ccacccggc tacaaacctc accttctcct cctactatca gcacacctcc 120

cctgtggcgg ccatgttcat tgtggcctat gcgctcatct tcctgctctg catggtgggc 180

aacaccctgg tctgtttcat cgtgctcaag aaccggcaca tgcatactgt caccaacatg 240

ttcatcctca acctggctgt cagtgacctg ctggtgggca tcttctgcat gcccaccacc 300

cttgtggaca acctcatcac tgggtggccc ttcgacaatg ccacatgcaa gatgagcggc 360

ttggtgcagg gcatgtctgt gtcggcttcc gttttcacac tggtggccat tgctgtggaa 420

aggttccgct gcatcgtgca ccctttccgc gagaagctga ccctgcggaa ggcgctcgtc 480

accatcgccg tcatctgggc cctggcgctg ctcatcatgt gtccctcggc cgtcacgctg 540

accgtcaccc gtgaggagca ccacttcatg gtggacgccc gcaaccgctc ctaccctctc 600

tactcctgct gggaggcctg gcccgagaag ggcatgcgca gggtctacac cactgtgctc 660

ttctcgcaca tctacctggc gccgctggcg ctcatcgtgg tcatgtacgc ccgcatcgcg 720

cgcaagctct gccaggcccc gggcccggcc cccgggggcg aggaggctgc ggacccgcga 780

gcatcgcggc gcagagcgcg cgtggtgcac atgctggtca tggtggcgct gttcttcacg 840

ctgtcctggc tgccgctctg ggcgctgctg ctgctcatcg actacgggca gctcagcgcg 900

ccgcagctgc acctggtcac cgtctacgcc ttccccttcg cgcactggct ggccttcttc 960

aacagcagcg ccaaccccat catctacggc tacttcaacg agaacttccg ccgcggcttc 1020

caggccgcct tccgcgcccg cctctgcccg cgcccgtcgg ggagccacaa ggaggcctac 1080

tccgagcggc ccggcgggct tctgcacagg cgggtcttcg tggtggtgcg gcccagcgac 1140

tccgggctgc cctctgagtc gggccctagc agtggggccc ccaggcccgg ccgcctcccg 1200

ctgcggaatg ggcgggtggc tcaccacggc ttgcccaggg aagggcctgg ctgctcccac 1260

ctgcccctca ccattccagc ctgggatatc 1290

<210> 56

<211> 1290

<212> DNA

<213> Human

<400> 56

atggaggggg agccctccca gcctcccaac agcagttggc ccctaagtca gaatgggact 60

aacactgagg ccaccccggc tacaaacctc accttctcct cctactatca gcacacctcc 120

cctgtggcgg ccatgttcat tgtggcctat gcgctcatct tcctgctctg catggtgggc 180

aacaccctgg tctgtttcat cgtgctcaag aaccggcaca tgcatactgt caccaacatg 240
ttcatcctca acctggctgt cagtgacctg ctggtgggca tcttctgcat gcccaccacc 300
cttgtggaca acctcatcac tgggtggccc ttcgacaatg ccacatgcaa gatgagcggc 360
ttggtgcagg gcatgtctgt gtcggcttcc gttttcacac tggtggccat tgctgtggaa 420
aggttccgct gcatcgtgca ccctttccgc gagaagctga ccctgcggaa ggcgctcgtc 480
accatcgccg tcatctgggc cctggcgctg ctcatcatgt gtccctcggc cgtcacgctg 540
accgtcaccc gtgaggagca ccacttcatg gtggacgccc gcaaccgctc ctacccgctc 600
tactcctgct gggaggcctg gcccgagaag ggcatgcgca gggtctacac cactgtgctc 660
ttctcgcaca tctacctggc gccgctggcg ctcatcgtgg tcatgtacgc ccgcatcgcg 720
cgcaagctct gccaggcccc gggcccggcc cccgggggcg aggaggctgc ggacccgcga 780
gcatcgcggc gcagagcgcg cgtggtgcac atgctggtca tggtggcgct gttcttcacg 840
ctgtcctggc tgccgctctg ggcgctgctg ctgctcatcg actacgggca gctcagcgcg 900
ccgcagctgc acctggtcac cgtctacgcc ttccccttcg cgcactggct ggccttcttc 960
aacagcagcg ccaaccccat catctacggc tacttcaacg agaacttccg ccgcggcttc 1020
caggccgcct tccgcgcccg cctctgcccg cgcccgtcgg ggagccacaa ggaggcctac 1080
tccgagcggc ccggcgggct tctgcacagg cgggtcttcg tggtggtgcg gcccagcgac 1140
tccgggctgc cctctgagtc gggccctagc agtggggccc ccaggcccgg ccgcctcccg 1200
ctgcggaatg ggcgggtggc tcaccacggc ttgcccaggg aagggcctgg ctgctcccac 1260
ctgcccctca ccattccagc ctgggatatc 1290

<210> 57

<211> 31

<212> DNA

<213> Artificial Sequence

<220>

<223>

<400> 57

gtcgacatgg agggggagcc ctcccagcct c 31

<210> 58

<211> 29

<212> DNA

<213> Artificial Sequence

<220>

<223>

<400> 58

actagttcag atatcccagg ctggaatgg 29

<210> 59

<211> 57

<212> DNA

<213> Artificial Sequence

<220>

<223> Primer

<400> 59

tatgagcctg aactttgaag aactgaaagat tggggtccga aaaatgtgat taaaatg 57

<210> 60

<211> 61

<212> DNA

<213> Artificial Sequence

<220>

<223> Primer

<400> 60

agcaccccgg cggtgaataa aatgccgcat agctttgcga atctgccgct gcgtttttgc 60

c 61

<210> 61

<211> 62

<212> DNA

<213> Artificial Sequence

<220>

<223> Primer

<400> 61

ggtgctcatt ttaatcacat ttttcggacc ccaatctttc agttcttcaa agttcaggct 60

ca 62

<210> 62

<211> 58

<212> DNA

<213> Artificial Sequence

<220>

<223> Primer

<400> 62

tcggggcaaa aacgcagcgg cagattcgca aagctatgcgg catttatttc accgccgg 58